# EUROPEAN PATENT SPECIFICATION

(11) **EP 4 028 097 B1**
(45) Date of publication and mention of the grant of the patent: **04.03.2026**
(21) Application number: 20863021.0
(22) Date of filing: 09.09.2020
(51) Int. Cl.: A61M 16/10, A61M 16/20

(54) **SUPPLEMENTARY GAS SOURCE DETECTION AND RELATED APPARATUSES**
ZUSÄTZLICHE GASQUELLENDETEKTION UND ZUGEHÖRIGE VORRICHTUNGEN
DÉTECTION DE SOURCE DE GAZ SUPPLÉMENTAIRE ET APPAREILS

(30) Priority: 09.09.2019 US 201962897899 P; 14.05.2020 US 202063025151 P
(43) Date of publication of application: 20.07.2022
(73) Proprietor: Fisher & Paykel Healthcare Limited, East Tamaki Auckland 2013 (NZ)
(72) Inventor: SANSON, Samuel Carey Matthew, Auckland, 2013 (NZ); CHAN, Leon Yip, Auckland, 2013 (NZ); GULLEY, Anton Kim, Auckland, 2013 (NZ); CHAN, Hamish, Auckland, 2013 (NZ); HSU, Jack Che-Wei, Auckland, 2013 (NZ); ROBERTSON, Michael Jesse, Auckland, 2013 (NZ)
(74) Representative: Gill Jennings & Every LLP
(86) International application number: PCT/IB2020/058351
(87) International publication number: WO 2021/048744

(56) References cited:
- EP-A2- 0 916 358
- WO-A1-2015/172160
- WO-A1-2017/059530
- WO-A1-2018/154060
- WO-A2-2013/151448
- CA-A1- 2 969 315
- US-A1- 2010 078 026
- US-A1- 2016 082 220

## Description

### FIELD OF THE INVENTION

The present invention relates to apparatuses for use in the delivery of a flow of gas, and particularly but not solely to apparatuses for detection of a supplemental gases source, such as in a respiratory support system, such as a respiratory humidification system or a high flow support system.

### BACKGROUND

Respiratory apparatuses are used in various environments such as in hospitals or other medical facilities, residential care facilities, or home environments. These respiratory apparatuses may be used to deliver a gas flow to a person, such as for use in breathing assistance.

Such a respiratory apparatus may provide control over characteristics of the gases flow, including characteristics such as flow rate, temperature, gas concentration, such as oxygen concentration, humidity, or pressure.

Some respiratory therapy apparatuses may provide for forms of closed loop control, where the device operates to provide a desired output gases characteristic or characteristics. For example, the operation of such a closed loop control regime may be the result of setting a target output gas oxygen concentration or a target blood oxygen concentration of a patient.

Such closed loop control regimes may allow less physician interaction in order to provide the desired therapy. However, such devices may be reliant on the connection and supply of a high pressure gases source.

WO2013151448A2 (published 10 October 2013) discloses a breathing assistance apparatus configured with features that improve serviceability of the apparatus.

### SUMMARY

Disclosed are apparatuses and methods (not claimed) for gas source detection and related apparatuses and methods.

According to a first aspect, the present disclosure provides a **device for delivery of** a **flow of gases,** the device comprising
a first inlet for receiving a gases flow from a first gases source,
a second inlet for connection of a supplemental gases source,
a valve in fluid communication with the second inlet and operable to control a flow of gases to the device by the second inlet,
a gases composition sensor, and
a controller to detect the connection of a supplemental gases source to the second inlet, wherein the controller is configured to detect connection by:
   pulsing the valve from a closed condition to an open condition for a first predetermined time period;
   receiving a gases composition reading from the gases composition sensor, and
   comparing the gases composition reading to a predetermined threshold, and determine that a supplemental gases source is connected at the second inlet if the reading exceeds the predetermined threshold.

The predetermined threshold is a threshold of gas composition.

The predetermined threshold is a threshold of a concentration of one or more target gas species in the gases flow.

The target gas species is a gas species of the supplemental gases.

The target gas species is a gas species of both the supplemental gases and the gases of the first gases source.

The target gas species is oxygen.

The gases composition sensor measures an oxygen concentration in a gases flow past the gases composition sensor.

The predetermined threshold is a threshold of oxygen composition of the gases flow.

The predetermined threshold is a threshold of oxygen composition of the gases flow in excess of an ambient air oxygen concentration.

The threshold of oxygen composition is a composition of about 21%.

The threshold of oxygen composition is a composition of about 25%.

The threshold of oxygen composition is a composition of about 35%.

The controller is configured to monitor an oxygen concentration from the gases composition sensor outside of the detection of the connection of a supplemental gases source to the second inlet, and the controller triggers an alarm if the sensed oxygen concentration exceeds 25%.

The controller is configured to prevent the commencement of the detection of the connection of a supplemental gases source to the second inlet if the sensed oxygen concentration exceeds 25%.

The gases composition sensor is located downstream of the second inlet.

The gases composition sensor is located downstream of both the first inlet and the second inlet.

If the reading does not exceed the predetermined threshold, the controller determines that there is no supplemental gas flow.

The fault condition is associated with either or both of a) a fault of the valve, and b) a lack of connection of a supplemental gases source at the second inlet.

If the controller determines the existence of the fault condition the controller triggers an alarm.

If the controller determines the existence of the fault condition the controller continues to attempt to detect the connection of a supplemental gases source.

The detection is repeated until either a) the controller determines that a supplemental gases source is connected at the second inlet, or b) controller receives an input to stop.

The detection is repeated at a predetermined rate until either condition (a) or condition (b) is satisfied.

The detection is completed when either a) the controller determines that a supplemental gases source is connected at the second inlet, or b) a user intervenes.

The comparing of the reading to the predetermined threshold occurs within a threshold time limit.

The comparison of the reading to the predetermined threshold occurs after a threshold time limit.

The threshold time limit is a time subsequent to the pulsing of the valve.

The threshold time limit is a time beginning at the pulsing of the valve.

The threshold time limit is a time such as to allow for a mixing of a gases flow from the first inlet and the second inlet.

The threshold time limit is a time such as to allow supplemental gases introduced into the device by the pulsing of the valve to reach the gases composition sensor.

The threshold time limit is dependent on a gases flow rate between the first inlet and second inlet and the gases composition sensor.

The threshold time limit is selected from a time of about 1 s to about 30 s.

The threshold time limit is approximately 5 s.

The device comprises a gases mixer to mix inlet gases, and the gases mixer is positioned upstream of the gases composition sensor and downstream of the first inlet and second inlet.

The device is or comprises a flow generator for generating a flow of gases from the inlets to an outlet.

The flow generator comprises a blower.

A or the gases mixer is comprised by the blower.

The controller operates the flow generator at or above a minimum flow condition as a precursor for the initiation of the detection of the connection of a supplemental gases source to the second inlet.

The controller operates the blower at or above the minimum flow condition until the detection is completed.

The controller operates the blower at or above the minimum flow condition subsequent to the completion of the detection.

The controller operates the blower at or above the minimum flow condition subsequent to the completion of the detection for a time suitable to substantially completely purge gases contained within the device at the completion of the detection.

The time is dependent on the predetermined flow condition.

The time is from about 5 s to about 60 s.

The time is approximately 30 s.

The valve is a proportional valve.

The valve is biased to a closed condition.

The pulsing of the valve comprises an opening of the valve from a closed condition to an open condition and back to a closed condition.

The pulsing of the valve by the controller comprises an opening of the valve for the first predetermined time period.

The pulsing of the valve by the controller comprises an opening of the valve and closing of the valve within the first predetermined time period.

The pulsing of the valve comprises a substantially complete opening of the valve.

The pulsing of the valve comprises a substantially complete closing of the valve.

The pulsing of the valve maximises the duration at which the valve is in its open condition.

The first predetermined time period is from about 10 ms to about 1 s.

The first predetermined time period is approximately 350 ms.

The controller monitors the current to the valve during the pulsing of the valve as an indicator of valve operation.

The controller operates to detect the connection of a supplemental gases source at the second inlet upon receipt of an instruction from a user to use the supplemental gases source.

The controller operates to detect the connection of a supplemental gases source at the second inlet upon receipt of an instructed operation of the device from a user, the instructed operation being dependent on the use of the supplemental gases source.

The controller operates to detect the connection of a supplemental gases source at the second inlet upon receipt an input from a user of a target oxygen concentration in excess of an ambient oxygen concentration.

The controller operates the detection upon the setting of the target oxygen concentration in excess of 21%.

The device comprises a patient blood oxygen sensor for sensing a blood oxygen concentration of the patient.

The controller receives an input from a patient blood oxygen sensor.

The controller operates the detection upon an operation of the device to a closed loop gas control mode.

The closed loop gas control mode comprises closed loop oxygen control, the controller receives a target blood oxygen concentration and dependent on this target operates at least the second inlet valve to control the oxygen concentration of gases delivered by the device.

The controller is configured to disable a or the closed loop gas control mode for a third predetermined time after the completion of the detection.

The third predetermined time is a time such as to substantially completely purge gases contained within the device at the completion of the detection.

The third predetermined time is from about 5 s to about 120 s.

The third predetermined time is approximately 60 s.

The device comprises a pressure sensor associated with the second inlet, and a signal from the pressure sensor is indicative of the connection of a supplemental gases source at the second inlet, such that the controller can alternatively detect the connection of a supplemental gases source to the second inlet a) by a sensed pressure exceeding a threshold pressure, and b) independently of a pulsing of the valve.

The threshold pressure is ambient pressure.

The threshold pressure is a reference value which is greater than an ambient pressure.

The pressure sensor is located upstream of the valve of the second inlet.

The pressure sensor is located upstream of the valve of the second inlet and a or the blower.

The controller operates the alternative detection substantially continuously independent of an operational mode of the device.

The controller operates the alternative detection independently of a delivery of therapy by the device.

The pressure sensor is located downstream of the valve of the second inlet.

The supplemental gases source is a pressurised gas source.

The device comprises a gases flow sensor, and the controller is configured to detect the connection of a supplemental gases source at the second inlet:
a) by an increase in sensed flow above a threshold subsequent to and within a fourth predetermined time of the pulsing of the valve by the controller, and
b) independent of the reading or comparison of a signal from the gases composition sensor.

The first inlet is an ambient air inlet.

The first gases source is an ambient air source.

The second inlet is an oxygen inlet.

The second inlet is a high pressure oxygen inlet.

The supplemental gases source is a pressurised gases source.

The supplemental gases source is an oxygen source.

The device is a respiratory support device for providing a flow of gases to a patient.

The respiratory support device comprises a housing that receives a or the flow generator and a respiratory humidifier.

The respiratory support device comprises a or the flow generator, and the flow generator is for use with a respiratory humidifier.

The gases composition sensor comprises an ultrasonic transducer sensor.

According to another aspect, the present disclosure provides **a method of detecting the connection of a supplemental gases source** to a respiratory therapy device for the delivery of a flow of gases to a patient, the respiratory therapy device comprising a first inlet for receiving a gases flow from a first gases source, a second inlet for connection of a supplemental gases source, a valve in fluid communication with the second inlet and operable to control the introduction of gases to the device by second inlet, a gases composition sensor, the method comprising
automatically pulsing the valve at least once from a closed condition to an open condition and back to a closed condition, pulsing occurring within a first predetermined time period,
sensing a gases composition with the gases composition sensor and generating a signal reading based on the sensed gas composition,
automatically receiving the signal reading generated by the gas composition sensor and comparing the gases composition reading to a predetermined threshold of gases composition,
autonomously determining that a supplemental gases source is connected at the second inlet if the gases composition reading exceeds the predetermined threshold, and
automatically generating a command signal in response to the determination.

The command signal is indicative that a supplemental gases source is connected at the second inlet.

The predetermined threshold is a threshold of a concentration of one or more gas species in the gases flow.

The target gas species is a gas species of the supplemental gases.

The target gas species is a gas species of both the supplemental gases and the gases of the first gases source.

The target gas species is oxygen.

The gases composition sensor measures an oxygen concentration in a gases flow past the gases composition sensor.

The predetermined threshold is a threshold of oxygen composition of the gases flow.

The predetermined threshold is a threshold of oxygen composition of the gases flow in excess of an ambient air oxygen concentration.

The threshold of oxygen composition is a composition of about 21%.

The threshold of oxygen composition is a composition of about 25%.

The threshold of oxygen composition is a composition of about 35%.

The gases composition sensor is located downstream of the second inlet.

The gases composition sensor is located downstream of both the first inlet and the second inlet.

The respiratory therapy device comprises a gases mixer to mix inlet gases, and the gases mixer is positioned upstream of the gases composition sensor and downstream of the first inlet and second inlet.

The respiratory therapy device is or comprises a flow generator for generating a flow of gases from the inlets to an outlet.

The flow generator comprises a blower.

A or the gases mixer is comprised by the blower.

The blower is operated at or above a minimum flow condition as a precursor for the initiation of the detection of the connection of a supplemental gases source to the second inlet.

The blower is operated at or above the minimum flow condition until the detection is completed.

The blower is operated at or above the minimum flow condition subsequent to the completion of the detection.

The blower is operated at or above the minimum flow condition subsequent to the completion of the detection for a time suitable to substantially completely purge gases contained within the device at the completion of the detection.

The time is dependent on the predetermined flow condition.

The time is from about 5 s to about 60 s.

The time is approximately 30 s.

The first inlet is an ambient air inlet.

The first gases source is an ambient air source.

The second inlet is an oxygen inlet.

The second inlet is a high pressure oxygen inlet.

The supplemental gases source is a pressurised gases source.

The supplemental gases source is an oxygen source.

The respiratory support device comprises a housing that receives a or the flow generator and a respiratory humidifier.

The respiratory support device comprises a or the flow generator, and the flow generator is for use with a respiratory humidifier.

The gases composition sensor comprises an ultrasonic transducer sensor.

The comparing of the gases composition reading to the predetermined threshold occurs within a threshold time limit.

The comparison of the gases composition reading to the predetermined threshold occurs after a threshold time limit.

The threshold time limit is a time subsequent to the pulsing of the valve.

The threshold time limit is a time beginning at the pulsing of the valve.

The threshold time limit is a time such as to allow for a mixing of a gases flow from the first inlet and the second inlet.

The threshold time limit is a time such as to allow supplemental gases introduced into the device by the pulsing of the valve to reach the gases composition sensor.

The threshold time limit is dependent on a gases flow rate between the first inlet and second inlet and the gases composition sensor.

The threshold time limit is selected from a time of about 1 s to about 30 s.

The threshold time limit is approximately 5 s.

If the reading does not exceed the predetermined threshold, a determination is made of a fault condition associated with the receipt of no supplemental gases from the supplemental gases source by the device.

The fault condition is associated with either or both of a) a fault of the valve, and b) a lack of connection of a supplemental gases source at the second inlet.

If the fault condition is determined to exist, an alarm is triggered.

If the fault condition is determined to exist, the detection for the connection of a supplemental gases source is continued to be operated.

The detection is repeated until either a) it is determined that a supplemental gases source is connected at the second inlet, or b) an intervention occurs.

The detection is repeated at a predetermined rate until either condition (a) or condition (b) is satisfied.

The detection is completed when either a) it is determined that a supplemental gases source is connected at the second inlet, or b) an intervention occurs.

The valve is a proportional valve.

The valve is biased to a closed condition.

The first predetermined time period is from about 10 ms to about 1 s.

The first predetermined time period is approximately 350 ms.

Monitoring the current to the valve during the pulsing of the valve as an indicator of valve operation.

Detecting the connection of a supplemental gases source at the second inlet upon receipt of an instruction from a user to use the supplemental gases source.

Detecting the connection of a supplemental gases source at the second inlet upon receipt of an instructed operation of the device, the instructed operation being dependent on the use of the supplemental gases source.

Detection of the connection of a supplemental gases source at the second inlet is conducted upon receipt an input of a target oxygen concentration in excess of an ambient oxygen concentration.

Detection is conducted upon the setting of the target oxygen concentration in excess of 21%.

The device comprises a patient blood oxygen sensor for sensing a blood oxygen concentration of the patient.

The device receives an input from a patient blood oxygen sensor.

The detection is operated upon an operation of the device to a closed loop gas control mode.

The closed loop gas control mode comprises closed loop oxygen control, the method comprising the step of receiving a target blood oxygen concentration and dependent on this target operating at least the second inlet valve to control the oxygen concentration of gases delivered by the device.

A or the closed loop gas control mode is disabled for a third predetermined time after the completion of the detection.

The third predetermined time is a time such as to substantially completely purge gases contained within the device at the completion of the detection.

The third predetermined time is from about 5 s to about 120 s.

The third predetermined time is approximately 60 s.

The device comprises a pressure sensor associated with the second inlet, and the method further comprises the step of monitoring a signal from the pressure sensor, wherein a signal from the pressure sensor is indicative of the connection of a supplemental gases source at the second inlet, such that the connection of a supplemental gases source to the second inlet can be determined upon sensing a of a pressure exceeding a threshold pressure, and
wherein the alternative detection can be operated independent of a pulsing of the valve and monitoring of a gases composition reading by the gases composition sensor.

The threshold pressure is ambient pressure.

The threshold pressure is a reference value which is greater than an ambient pressure.

The pressure sensor is located upstream of the valve of the second inlet.

The pressure sensor is located upstream of the valve of the second inlet and a or the blower.

The alternative detection is operated substantially continuously independent of an operational mode of the device.

The alternative detection is operated independently of a delivery of therapy by the device.

The pressure sensor is located downstream of the valve of the second inlet.

The supplemental gases source is a pressurised gas source.

The device comprises a gases flow sensor, the detection of the connection of a supplemental gases source at the second inlet is determined by:
a) an increase in sensed flow above a threshold subsequent to and within a fourth predetermined time of the pulsing of the valve, and
b) independent of the reading or comparison of a signal from the gases composition sensor.

According to another aspect, the present disclosure provides a device **for** delivery **of a flow of** gases, the device operational in one or more therapy modes in which therapy is delivered and one or more standby modes in which therapy is not delivered, the device comprising
a first inlet for receiving a gases flow from a first gases source,
a second inlet for connection of a supplemental gases source,
a blower for generating a flow of gases from the inlets to an outlet of the device,
a gases composition sensor located downstream of both the first inlet and the second inlet, and
a controller configured in the one or more therapy modes to control the blower to deliver therapy and in the one or more standby modes to monitor a gas composition reading from the gas composition sensor, wherein in the one or more standby modes the controller is configured to:
   monitor the gases composition sensor, and if a gases composition reading in excess of a predetermined threshold is detected,
   trigger an alarm indicative of the detection of supplemental gases in the standby mode.

The supplemental gas is oxygen.

The first inlet is an ambient air inlet.

The first gases source is an ambient air source.

The second inlet is an oxygen inlet.

The second inlet is a high pressure oxygen inlet.

The supplemental gases source is a pressurised gases source.

The supplemental gases source is an oxygen source.

The predetermined threshold is a threshold of gas composition.

The predetermined threshold is a threshold of a concentration of one or more target gas species in the gases flow.

The target gas species is a gas species of the supplemental gases.

The target gas species is a gas species of both the supplemental gases and the gases of the first gases source.

The target gas species is oxygen.

The gases composition sensor measures an oxygen concentration in a gases flow past the gases composition sensor.

The predetermined threshold is a threshold of oxygen composition of the gases flow.

The predetermined threshold is a threshold of oxygen composition of the gases flow in excess of an ambient air oxygen concentration.

The threshold of oxygen composition is a composition of about 21%.

The threshold of oxygen composition is a composition of about 25%.

The gases composition sensor measures an oxygen concentration in a gases flow past the gases composition sensor.

The predetermined threshold comprises a predetermined oxygen concentration level.

The predetermined threshold is a concentration of about 21%.

The predetermined threshold is a concentration of about a sum of 21% and a measurement error of the sensor expressed as a percentage.

The predetermined threshold is a concentration of about 22% to about 35%.

The controller deactivates the blower in the one or more standby modes.

Each of the one or more standby modes comprise a mode in which the device is on but in which therapy is not delivered.

The alarm is indicative of the detection of a concentration of supplemental gases in the standby mode in excess of a concentration of the supplemental gases in gases from the first gases source.

The controller, upon detection of a gases composition reading in excess of the predetermined threshold, operates the blower to an increased flow condition relative to the minimum flow condition.

The increased flow condition comprises a flow rate of about 1 litre per minute to about 4 litres per minute.

The increased flow condition comprises a flow rate of about 2 litres per minute.

The controller, upon detection of a gas composition reading in excess of the predetermined threshold, operates the blower to an increased speed relative to the minimum speed.

The controller is configured such that, upon the triggering of an alarm indicative of the detection of supplemental gases in the standby mode, the controller continues to monitor the sensor for a gases composition reading.

If a gases composition reading below the predetermined threshold is detected, the controller clears the alarm and continues monitoring of the sensor.

The gases composition reading below the predetermined threshold is a concentration of about 1% to about 10% below the predetermined threshold.

The clearing of the alarm is associated with a reduction of the flow condition and/or speed of the blower to the minimum flow condition or minimum speed respectively.

The device comprises a third inlet for receiving a supplemental gases flow from a supplemental gases source.

The supplemental gases source of the third inlet is a different source to the supplemental gases source of the second inlet.

The supplemental gas of the third inlet is the same supplemental gas as supplied by the supplemental gas source of the second inlet.

The second inlet is for receiving a supplemental gases flow of higher pressure than the supplemental gases flow of the third inlet.

The device receives a supplemental gases flow via the second inlet when the device is operated in a high pressure mode.

The device receives a supplemental gases flow from the third inlet when the device is operated in a low pressure mode.

The second inlet and third inlet are of different configurations, such as to allow the connection of a gases source connector to only one of the second inlet and the third inlet.

The second inlet and third inlet are sized differently to each other.

The third inlet is an oxygen inlet.

The device is a respiratory support device for providing a flow of gases to a patient.

The device comprises a gases mixer for mixing a gases flow from the first inlet and at least one other inlet, and gases mixer is disposed upstream of the gases composition sensor.

The device is or comprises a flow generator for generating a flow of gases from the inlets to an outlet.

The flow generator comprises the blower.

A or the gases mixer is comprised by the blower.

The gases composition sensor comprises an ultrasonic transducer sensor.

The gases composition sensor is located downstream of the second inlet.

The gases composition sensor is located downstream of both the first inlet and the second inlet.

According to another aspect, the present disclosure provides **a method of detection and alerting** to the presence of supplemental gases in a standby mode of a respiratory therapy device, the respiratory therapy device comprising a first inlet for receiving a gases flow from a first gases source, a second inlet for connection of a supplemental gases source, a blower for generating a flow of gases from the inlets to an outlet of the device, a gases composition sensor located downstream of both the first inlet and the second inlet, the respiratory therapy device operable in one or more therapy modes in which therapy is delivered and one or more standby modes in which therapy is not delivered, in the one or more standby modes the method comprising the steps of
obtaining a gases composition reading by a gas composition sensor,
automatically reading the gases composition reading,
automatically comparing the gases composition reading to a predetermined threshold of gases composition, and
autonomously triggering an alarm if the gases composition reading exceeds the predetermined threshold of gases composition,
wherein the alarm is indicative of the detection of supplemental gases in the standby mode.

The alarm is indicative of the detection of a concentration of supplemental gases in the standby mode exceeding a concentration of the supplemental gases in gases from the first gases source.

Upon detection of a gases composition reading exceeding of the predetermined threshold, the blower is operated to an increased flow condition relative to the minimum flow condition.

The increased flow condition comprises a flow rate of about 1 litre per minute to about 4 litres per minute.

The increased flow condition comprises a flow rate of about 2 litres per minute.

Upon detection of a gas composition reading exceeding the predetermined threshold, the blower is operated to an increased speed relative to the minimum speed.

Upon the triggering of an alarm indicative of the detection of supplemental gases in the standby mode, the monitoring of the sensor for a gases composition reading is continued.

If a gases composition reading below the predetermined threshold is detected, the alarm is cleared and the monitoring of the sensor is continued.

The gases composition reading below the predetermined threshold is a concentration of about 1% to about 10% below the predetermined threshold.

The clearing of the alarm is associated with a reduction of the flow condition and/or speed of the blower to the minimum flow condition or minimum speed respectively.

The method is operated by a controller.

The supplemental gas is oxygen.

The first inlet is an ambient air inlet.

The first gases source is an ambient air source.

The second inlet is an oxygen inlet.

The second inlet is a high pressure oxygen inlet.

The supplemental gases source is a pressurised gases source.

The supplemental gases source is an oxygen source.

The predetermined threshold is a threshold of gas composition.

The predetermined threshold is a threshold of a concentration of one or more target gas species in the gases flow.

The target gas species is a gas species of the supplemental gases.

The target gas species is a gas species of both the supplemental gases and the gases of the first gases source.

The target gas species is oxygen.

The gases composition sensor measures an oxygen concentration in a gases flow past the gases composition sensor.

The predetermined threshold is a threshold of oxygen composition of the gases flow.

The predetermined threshold is a threshold of oxygen composition of the gases flow in excess of an ambient air oxygen concentration.

The threshold of oxygen composition is a composition of about 21%.

The threshold of oxygen composition is a composition of about 25%.

The gases composition sensor measures an oxygen concentration in a gases flow past the gases composition sensor.

The predetermined threshold comprises a predetermined oxygen concentration level.

The predetermined threshold is a concentration of about 21%.

The predetermined threshold is a concentration of about a sum of 21% and a measurement error of the sensor expressed as a percentage.

The predetermined threshold is a concentration of about 22% to about 35%.

According to another aspect, the present disclosure provides a **device for delivery of a flow of gases,** the device operational in one or more therapy modes in which therapy is delivered and one or more standby modes in which therapy is not delivered, the device comprising
a first inlet for receiving a gases flow from a first gases source,
a second inlet for connection of a supplemental gases source,
a blower for generating a flow of gases from the inlets to an outlet of the device,
a gases composition sensor located downstream of both the first inlet and the second inlet, and
a controller to operate the blower at a minimum flow condition in the one or more standby modes and upon receiving a command to transition from a therapy mode to a standby mode, the controller configured to:
   receive a command to transition from a therapy mode to a standby mode,
   delay an entry into the standby mode until up to a predetermined time period, and
   operate the blower to a predetermined flow condition for up to the predetermined time period so as to flush from the device gases contained within the device at the time of the command to transition.

The one or more therapy modes comprise at least one high pressure mode and at least one low pressure mode, wherein the pressure of the supplemental gases received in each high pressure mode is greater than the pressure of the supplemental gases received in each low pressure mode.

The predetermined flow condition is a flow condition suitable to flush from the device gases contained within the device at the time of the user command to transition.

The flushing comprises a flushing to a concentration of supplemental gases below a safety limit.

The predetermined flow condition comprises a flow of about 2 litres per minute.

The controller is further configured when the device is in a low pressure mode and responsive to the receipt of a command to transition from a therapy mode to a standby mode to instruct the user to shut off the supplemental gases source associated with the second inlet.

The device comprises a third inlet for receiving a supplemental gases flow from a supplemental gases source distinct from the second inlet supplemental gases source, wherein the second inlet is for receiving a supplemental gases flow of higher pressure than the supplemental gases flow of the third inlet.

The instruction to shut off the supplemental gases source comprises an instruction to shut off the supplemental gases source associated with the second inlet.

The instruction to shut off the supplemental gases source comprises an instruction to shut off the supplemental gases source associated with the third inlet.

The device receives a supplemental gases flow via the second inlet when the device is operated in a high pressure mode.

The device receives a supplemental gases flow from the third inlet when the device is operated in a low pressure mode.

The controller is further configured when the device is in a low pressure mode and responsive to the receipt of a command to transition from the low pressure mode to a standby mode to instruct the user to shut off the supplemental gases source associated with the third inlet.

The device receives a supplemental gases flow via the second inlet when the device is operated in a high pressure mode.

The device comprises a valve operable to control a flow of gases introduced to the device by the second inlet, and the valve is operable by the controller.

The valve is a proportional valve.

The valve is biased to a closed condition.

The controller is further configured responsive to the receipt of a command to transition from a therapy mode to a standby mode to operate the valve to a closed condition.

The therapy mode is a high pressure mode.

The controller operates the valve to a closed condition prior to the beginning of the predetermined time period.

The controller operates the valve to a closed condition during the predetermined time period.

The operation of the device between the receipt of a command to transition from a therapy mode to a standby mode and the standby mode comprises a transitional period.

The controller is further configured, responsive to the receipt of a command to transition from a therapy mode to a standby mode, to operate the blower to a reduced flow condition relative to a flow condition of the blower in the preceding therapy mode.

The reduced flow condition is about 1 litre per minute to about 3 litres per minute.

The reduced flow condition is about 2 litres per minute.

The controller, when the predetermined time period is met or exceeded, operates the device to a standby mode.

The controller, when the device is in the transitional period, operates the blower to a reduced flow condition relative to a flow condition associated with the preceding therapy mode.

The minimum flow condition, when the device is in a standby mode, is a reduced flow condition relative to a flow condition associated with the transitional period.

The minimum flow condition, when the device is in a standby mode, comprises a deactivation of the blower.

The command to transition from a therapy mode to a standby mode comprises a user command.

A user interface comprising one or more of a display and a user input device such as a touch screen, a voice input device, a keyboard, a trigger, a mouse, or a button.

The device comprises a gases composition sensor.

The gases composition sensor comprises an ultrasonic transducer sensor.

The controller is further configured, responsive to the receipt of a command to transition from a therapy mode to a standby mode, to monitor a gas composition reading from the gases composition sensor for the duration of the predetermined time period.

The controller is further configured, if at any time during the predetermined time period the reading is below a predetermined threshold, to terminate the delay and operate the device to a standby mode.

The controller is further configured, responsive to the receipt of a command to transition from a therapy mode to a standby mode, to operate the device to a standby mode only upon both a) the expiry of the predetermined time period, and b) the reading of the gases composition sensor being below a predetermined threshold.

The predetermined threshold is a threshold of a concentration of one or more target gas species in the gases flow.

The target gas species is a gas species of the supplemental gases.

The target gas species is a gas species of both the supplemental gases and the gases of the first gases source.

The target gas species is oxygen.

The predetermined threshold is a threshold of oxygen composition of the gases flow.

The predetermined threshold is a threshold of oxygen composition of the gases flow in excess of an ambient air oxygen concentration.

The threshold of oxygen composition is a composition of about 21%.

The threshold of oxygen composition is a composition of about 25%.

The predetermined time period is about 5 s to about 2 min.

The predetermined time period is about 30 seconds.

The gases composition sensor is located downstream of the second inlet.

The gases composition sensor is located downstream of both the first inlet and the second inlet.

The flushing of the gases comprises a purging from the device of the gases contained within the device at the time of the user command to transition.

The flushing comprises a flushing to a concentration of supplemental gases below a safety limit.

The gases to be flushed from the device are supplemental gases source gases.

The controller is configured in the one or more therapy modes to operate the blower to deliver a therapy.

The first inlet is an ambient air inlet.

The first gases source is an ambient air source.

The second inlet is an oxygen inlet.

The second inlet is a high pressure oxygen inlet.

The supplemental gases source is a pressurised gases source.

The supplemental gases source is an oxygen source.

The valve is a proportional valve.

The valve is biased to a closed condition.

The device is a respiratory support device for providing a flow of gases to a patient.

The device comprises a gases mixer for mixing a gases flow from the first inlet and at least one other inlet, and gases mixer is disposed upstream of the gases composition sensor.

The device is or comprises a flow generator for generating a flow of gases from the inlets to an outlet.

The flow generator comprises the blower.

A or the gases mixer is comprised by the blower.

According to another aspect, the present disclosure provides a **method of transitioning of a respiratory therapy device** for delivery of a flow of gases to a patient from a therapy mode in which a therapy is delivered to a standby mode in which a therapy is not delivered, the respiratory therapy device comprising a first inlet for receiving a gases flow from a first gases source, a second inlet for connection of a supplemental gases source, a blower for generating a flow of gases from the inlets to an outlet of the device, a gases composition sensor located downstream of both the first inlet and the second inlet,
wherein the blower is operated at a minimum flow condition in the one or more standby modes, the method comprising the steps of
autonomously monitoring for a direction to transition from a therapy mode to a standby mode,
receiving a direction to transition and upon receipt of the direction:
   automatically delaying an entry into the standby mode until after the expiry of a predetermined time period, and
   dynamically operating the blower to a predetermined flow condition for up to the predetermined time period so as to flush from the device gases contained within the device at the time of the direction to transition.

The predetermined flow condition is a flow condition suitable to flush from the device gases contained within the device at the time of the direction to transition.

The flushing comprises a flushing to a concentration of supplemental gases below a safety limit.

The predetermined flow condition comprises a flow of about 2 litres per minute.

The flushing of the gases comprises a flushing substantially complete purging from the device of the gases contained within the device at the time of the direction to transition.

The flushing comprises a flushing to a concentration of supplemental gases below a safety limit.

The gases to be flushed from the device are supplemental gases source gases.

The one or more therapy modes comprise at least one high pressure mode and at least one low pressure mode, wherein the pressure of the supplemental gases received in each high pressure mode is greater than the pressure of the supplemental gases received in each low pressure mode.

The method further comprising the step, when the device is in a low pressure mode and responsive to the receipt of a direction to transition from a therapy mode to a standby mode, of instructing the user to shut off the supplemental gases source associated with the second inlet.

The device comprises a third inlet for receiving a supplemental gases flow from a supplemental gases source distinct from the second inlet supplemental gases source, wherein the second inlet is for receiving a supplemental gases flow of higher pressure than the supplemental gases flow of the third inlet.

The instruction to shut off the supplemental gases source comprises an instruction to shut off the supplemental gases source associated with the second inlet.

The instruction to shut off the supplemental gases source comprises an instruction to shut off the supplemental gases source associated with the third inlet.

The device receives a supplemental gases flow from the second inlet when the device is operated in a high pressure mode.

The device receives a supplemental gases flow from the third inlet when the device is operated in a low pressure mode.

The method further comprising the step, when the device is in a low pressure mode and responsive to the receipt of a direction to transition from the low pressure mode to a standby mode, of instructing the user to shut off the supplemental gases source associated with the third inlet.

The device receives a supplemental gases flow from the second inlet when the device is operated in a high pressure mode.

The device comprises a valve operable to control a flow of gases introduced to the device by the second inlet, and the valve is operable as part of the method.

The valve is a proportional valve.

The valve is biased to a closed condition.

The method further comprising the step, responsive to the receipt of a direction to transition from a therapy mode to a standby mode, of operating the valve to a closed condition.

The therapy mode is a high pressure mode.

The valve is operated to a closed condition prior to the beginning of the predetermined time period.

The valve is operated to a closed condition during the predetermined time period.

The operation of the device between the receipt of a direction to transition from a therapy mode to a standby mode and the standby mode comprises a transitional period.

The method further comprising the step of, responsive to the receipt of a direction to transition from a therapy mode to a standby mode, operating the blower to a reduced flow condition relative to a flow condition of the blower in the preceding therapy mode.

The reduced flow condition is about 1 litre per minute to about 3 litres per minute.

The reduced flow condition is about 2 litres per minute.

When the predetermined time period is met or exceeded the device is operated to a standby mode.

When the device is in the transitional period the blower is operated to a reduced flow condition relative to a flow condition associated with the preceding therapy mode.

The minimum flow condition, when the device is in a standby mode, is a reduced flow condition relative to a flow condition associated with the transitional period.

The minimum flow condition, when the device is in a standby mode, comprises a deactivation of the blower.

The device comprises a gases composition sensor.

The gases composition sensor comprises an ultrasonic transducer sensor.

The method further comprising the step, responsive to the receipt of a direction to transition from a therapy mode to a standby mode, of monitoring a gas composition reading from the gases composition sensor for the duration of the predetermined time period.

The method further comprising the step, if at any time during the predetermined time period the reading is below a predetermined threshold, of terminating the delay and operating the device to a standby mode.

The method further comprising the step, responsive to the receipt of a direction to transition from a therapy mode to a standby mode, of operating the device to a standby mode only upon both a) the expiry of the predetermined time period, and b) the reading of the gases composition sensor being below a predetermined threshold.

The predetermined threshold is a threshold of a concentration of one or more target gas species in the gases flow.

The target gas species is a gas species of the supplemental gases.

The target gas species is a gas species of both the supplemental gases and the gases of the first gases source.

The target gas species is oxygen.

The predetermined threshold is a threshold of oxygen composition of the gases flow.

The predetermined threshold is a threshold of oxygen composition of the gases flow in excess of an ambient air oxygen concentration.

The threshold of oxygen composition is a composition of about 21%.

The threshold of oxygen composition is a composition of about 25%.

The predetermined time period is about 5 s to about 2 min.

The predetermined time period is about 30 seconds.

The gases composition sensor is located downstream of the second inlet.

The gases composition sensor is located downstream of both the first inlet and the second inlet.

The direction to transition is a user direction.

The method is operated by a controller.

The first inlet is an ambient air inlet.

The first gases source is an ambient air source.

The second inlet is an oxygen inlet.

The second inlet is a high pressure oxygen inlet.

The supplemental gases source is a pressurised gases source.

The supplemental gases source is an oxygen source.

The valve is a proportional valve.

The valve is biased to a closed condition.

The device comprises a gases mixer for mixing a gases flow from the first inlet and at least one other inlet, and gases mixer is disposed upstream of the gases composition sensor.

The device is or comprises a flow generator for generating a flow of gases from the inlets to an outlet.

The flow generator comprises the blower.

A or the gases mixer is comprised by the blower.

According to another aspect, the present disclosure provides a **device for delivery of a flow of gases,** the device comprising
a first inlet for receiving a gases flow from a first gases source,
a second inlet for connection of a supplemental gases source,
a valve operable to control a flow of supplemental gases introduced to the device by the second inlet,
a gases composition sensor located downstream of both the first inlet and the second inlet to sense a gas species concentration in flow of gases, and
a controller to control the valve based at least in part on a) a target threshold of gas composition and b) a gases composition reading from the gases composition sensor, the controller configured to:
   monitor a gases composition reading of the gases composition sensor,
   trigger a first alarm if the sensed gas species concentration is in excess of a concentration of the gas species in the gases flow from the first gases source but the sensed gas species concentration is outside the target threshold gas composition, and
   trigger a second alarm if a) the sensed gas species concentration is below a threshold concentration, the threshold concentration being the sum of a concentration of the gas species in the gases flow from the first gases source and a predetermined measurement error of the gases composition sensor, and b) the sensed gas species concentration is outside of the target threshold gas composition,
   wherein the first alarm and second alarm are different from each other.

The target threshold of gas composition is a target threshold of concentration of the gas species.

The target threshold of gas composition comprises a dead zone either side of the threshold.

The target threshold of gas composition is greater than an initial gases composition reading.

If the sensed gases composition is less than the target threshold of gas composition, as a precursor to the triggering of the first alarm the controller operates the valve to a more open condition.

The operation of the valve to a more open condition comprises a progressive operation of the valve.

Upon the operation of the valve to a more open condition, the controller again checks if the sensed gas species concentration is in excess of the gas species concentration in the gases flow from the first gases source, but the sensed gas species concentration is outside the target threshold gas composition.

The operation of the valve ultimately comprises the operation of the valve to a fully open condition.

The controller triggers the first alarm dependent on the valve being at its fully open condition.

The controller triggers the first alarm dependent on the valve having been in its open condition for a first predetermined time period.

The first predetermined time period is about 10 ms to about 1 min.

The first predetermined time period is from about 1 s to about 20 s.

The concentration of the gas species in the gases flow from the first gases source is a predetermined concentration.

The first gases source is an ambient air source, and the concentration of the gas species in the gases flow from the first gases source comprises a concentration of an ambient air gas species.

The supplemental gas comprises a supply of the gas species.

The gas species is oxygen.

The oxygen concentration in the ambient air is approximately 21%.

The threshold concentration is a the sum of 21% and a measurement error of the sensor expressed as a percentage.

The threshold concentration about 22% to about 35%.

The threshold concentration is about 24%.

The supplementary gases source is a source of the gas species.

The first gases source comprises a substantially zero concentration of the gas species.

The first alarm indicates to a user that the target threshold of gas composition cannot be achieved.

The first alarm indicates to a user that the supply of supplemental gases is inadequate to achieve the target threshold of gas composition.

The controller, after the triggering of the first alarm, is configured to clear the first alarm if a sensed gases composition reading reaches or exceeds the target threshold of gas composition.

The second alarm indicates to a user that supplemental gases from the supplemental gases source are not being received by the device.

The first alarm and second alarm each provide a recommendation to a user to check a supplemental gases supply from the supplemental gases source.

The controller in association with the triggering of the second alarm a) deactivates valve control dependent on the target gases composition and the gases composition reading, and b) operates the valve to a closed condition.

The controller is configured to suspend the control of the device based on the target threshold of gas composition and the gases composition reading upon the triggering of the second alarm.

The controller, upon the triggering of the second alarm, is configured to detect the connection of a supplemental gases source by
pulsing the valve from a closed condition to an open condition,
comparing a gases composition reading from the gases composition sensor to a predetermined threshold of gases composition, and
dependent on the reading exceeding the predetermined threshold, clearing the second alarm.

The comparing of the reading to the predetermined threshold occurs within a second predetermined time period.

The comparison of the reading to the predetermined threshold occurs at a second predetermined time period.

The comparison of the reading to the predetermined threshold occurs after a second predetermined time period.

The second predetermined time period is a time subsequent to the pulsing of the valve.

The second predetermined time is a time such as to allow for a mixing of a gases flow from the first inlet and the second inlet at the gases composition sensor.

The second predetermined time period is dependent on a gases flow rate between the first inlet and second inlet and the gases composition sensor.

The second predetermined time period is selected from a time of about 1 s to about 30 s.

The second predetermined time period is approximately 5 s.

The controller upon the clearing of the second alarm reactivates the control of the device based on the target gases composition and the gases composition reading.

The reactivation of the control of the device based on the target gases composition and the gases composition reading is further dependent on the expiry of a time delay.

The time delay is from about 5 s to about 5 min.

The time delay is approximately 1 min.

The reading exceeding the predetermined threshold indicates that a supplemental gases source is connected at the second inlet.

The clearing of the alarm is further dependent on the reading exceeding the predetermined threshold within a second predetermined time period.

The second predetermined time period is about 1 s to about 30 s.

The second predetermined time period is about 5 s.

If the reading does not exceed the predetermined threshold, the controller maintains the second alarm, being an alarm associated with the receipt of no supplemental gases from the supplemental gases source by the device.

If the second alarm is maintained, the controller continues to attempt to detect the connection of a supplemental gases source.

The predetermined threshold is a threshold of gas composition.

The predetermined threshold is a threshold of a concentration of one or more target gas species in the gases flow.

The target gas species is a gas species of the supplemental gases.

The target gas species is a gas species of both the supplemental gases and the gases of the first gases source.

The target gas species is oxygen.

The predetermined threshold is a threshold of oxygen composition of the gases flow.

The predetermined threshold is a threshold of oxygen composition of the gases flow in excess of an ambient air oxygen concentration.

The threshold of oxygen composition is a composition of about 21%.

The threshold of oxygen composition is a composition of about 25%.

The gases composition sensor measures an oxygen concentration in a gases flow past the gases composition sensor.

The device comprises a third inlet for receiving a supplemental gases flow.

The second inlet is for receiving a supplemental gases flow of higher pressure than the supplemental gases flow of the third inlet.

The device receives a supplemental gases flow from the second inlet when the device is operated in a high pressure mode.

The device receives a supplemental gases flow from the third inlet when the device is operated in a low pressure mode.

The second inlet and third inlet are of different configurations, such as to allow the connection of a gases source connector to only one of the second inlet and the third inlet.

The second inlet and third inlet are sized differently to each other.

The device comprises a pressure sensor associated with the second inlet, and a signal from the pressure sensor is indicative of the connection of a supplemental gases source at the second inlet, such that the controller can alternatively detect the connection of a supplemental gases source at the second inlet independent of a pulsing of the valve.

The pressure sensor is located upstream of the valve of the second inlet.

The controller operates the alternative detection substantially continuously independent of an operational mode of the device.

The controller operates the alternative detection independently of a delivery of therapy by the device.

The pressure sensor is located downstream of the valve of the second inlet.

The device comprises a gases flow sensor, and the controller is configured to detect the connection of a supplemental gases source at the second inlet:
a) by an increase in sensed flow above a threshold subsequent to and within a fourth predetermined time of the pulsing of the valve by the controller, and
b) independent of the reading or comparison of a signal from the gases composition sensor.

The valve is a proportional valve.

The valve is biased to a closed condition.

The pulsing of the valve by the controller comprises an opening of the valve for a third predetermined time period.

The third predetermined time period is from about 10 ms to about 1 s.

The third predetermined time period is approximately 350 ms.

The pulsing of the valve by the controller comprises an opening of the valve and closing of the valve within the third predetermined time period.

The pulsing of the valve comprises a substantially complete opening of the valve.

The pulsing of the valve comprises a substantially complete closing of the valve.

The pulsing of the valve maximises the duration at which the valve is in its open condition.

The controller monitors the current to the valve during the pulsing of the valve as an indicator of valve operation.

The target threshold of gas composition is settable by a user.

The first inlet is an ambient air inlet.

The first gases source is an ambient air source.

The second inlet is an oxygen inlet.

The second inlet is a high pressure oxygen inlet.

The supplemental gases source is a pressurised gases source.

The supplemental gases source is an oxygen source.

The triggering of the first alarm is further dependent on the connection of the supplemental gases source at the second inlet.

The controller operates the valve to attain the target threshold of gas composition based on the gases composition reading from the gases composition sensor.

The gases composition sensor comprises an ultrasonic transducer sensor.

The gases composition sensor is located downstream of the second inlet.

The gases composition sensor is located downstream of both the first inlet and the second inlet.

The device is a respiratory support device for providing a flow of gases to a patient.

The device comprises a gases mixer for mixing a gases flow from the first inlet and at least one other inlet, and gases mixer is disposed upstream of the gases composition sensor.

The device is or comprises a flow generator for generating a flow of gases from the inlets to an outlet.

The flow generator comprises a blower.

A or the gases mixer is comprised by the blower.

According to another aspect, the present disclosure provides a **method of controlling** a **valve** of a second inlet of a respiratory therapy device for delivering a gases flow to a patient, the respiratory therapy device comprising a first inlet for receiving a gases flow from a first gases source, a second inlet for connection of a supplemental gases source, a valve operable to control a flow of supplemental gases introduced to the device at the second inlet, a gases composition sensor located downstream of both the first inlet and the second inlet to sense a gas species concentration in flow of gases, wherein the control of the valve is based at least in part on a) a target threshold of gas composition and b) a gases composition reading from the gases composition sensor, the method comprising the steps of
autonomously monitoring a gases composition reading of the gases composition sensor,
automatically generating a first alarm if the sensed concentration of the gas species is in excess of a concentration of the gas species in the gases flow from the first gases source but the sensed gas species concentration is outside the target threshold gas composition, and
automatically generating a second alarm if a) the concentration of the gas species is below a threshold concentration, the threshold concentration being the sum of a concentration of the gas species in the gases flow from the first gases source and a predetermined measurement error of the gases composition sensor, and b) the sensed gas species concentration is outside of the target threshold gas composition,
wherein the first alarm and second alarm are different from each other.

The target threshold of gas composition is a target threshold of concentration of the gas species.

The target threshold of gas composition comprises a dead zone either side of the threshold.

The target threshold of gas composition is greater than an initial gases composition reading.

If the sensed gases composition is less than the target threshold of gas composition, as a precursor to the triggering of the first alarm the valve is operated to a more open condition.

The operation of the valve to a more open condition comprises a progressive operation of the valve.

Upon the operation of the valve to a more open condition, it is checked whether the sensed gas species concentration is in excess of the concentration of the gas species in the gases flow from the first gases source, but the sensed gas species concentration is outside the target threshold gas composition.

The operation of the valve ultimately comprises the operation of the valve to a fully open condition.

The first alarm is triggered dependent on the valve being at its fully open condition.

The triggering of the first alarm is dependent on the valve having been in its open condition for a first predetermined time period.

The first predetermined time period is about 10 ms to about 1 min.

The first predetermined time period is from about 1 s to about 20 s.

The concentration of the gas species in the gases flow from the first gases source is a predetermined concentration.

The first gases source is an ambient air source, and the concentration of the gas species in the gases flow from the first gases source comprises a concentration of an ambient air gas species.

The supplemental gas comprises a supply of the gas species.

The gas species is oxygen.

The oxygen concentration in the ambient air is approximately 21%.

The threshold concentration is a the sum of 21% and a measurement error of the sensor expressed as a percentage.

The threshold concentration about 22% to about 35%.

The threshold concentration is about 24%.

The supplementary gases source is a source of the gas species.

The first gases source comprises a substantially zero concentration of the gas species.

The first alarm indicates to a user that the target threshold of gas composition cannot be achieved.

The first alarm indicates to a user that the supply of supplemental gases is inadequate to achieve the target threshold of gas composition.

The method further comprising the step, after the triggering of the first alarm, of clearing the first alarm if a sensed gases composition reading reaches or exceeds the target threshold of gas composition.

The second alarm indicates to a user that supplemental gases from the supplemental gases source are not being received by the device.

The first alarm and second alarm each provide a recommendation to a user to check a supplemental gases supply from the supplemental gases source.

The method further comprising the steps, in association with the triggering of the second alarm a) deactivating valve control dependent on the target gases composition and the gases composition reading, and b) operating the valve to a closed condition.

The method further comprising the step of suspending the control of the device based on the target threshold of gas composition and the gases composition reading upon the triggering of the second alarm.

The method further comprising the step, upon the triggering of the second alarm, of detecting the connection of a supplemental gases source by
pulsing the valve from a closed condition to an open condition,
comparing a gases composition reading from the gases composition sensor to a predetermined threshold of gases composition, and
dependent on the reading exceeding the predetermined threshold, clearing the second alarm.

The comparing of the reading to the predetermined threshold occurs within a second predetermined time period.

The comparison of the reading to the predetermined threshold occurs at a second predetermined time period.

The comparison of the reading to the predetermined threshold occurs after a second predetermined time period.

The second predetermined time period is a time subsequent to the pulsing of the valve.

The second predetermined time is a time such as to allow for a mixing of a gases flow from the first inlet and the second inlet at the gases composition sensor.

The second predetermined time period is dependent on a gases flow rate between the first inlet and second inlet and the gases composition sensor.

The second predetermined time period is selected from a time of about 1 s to about 30 s.

The second predetermined time period is approximately 5 s.

The method further comprising the step, upon the clearing of the second alarm, of reactivating the control of the device based on the target gases composition and the gases composition reading.

The reactivation of the control of the device based on the target gases composition and the gases composition reading is further dependent on the expiry of a time delay.

The time delay is from about 5 s to about 5 min.

The time delay is approximately 1 min.

The reading exceeding the predetermined threshold indicates that a supplemental gases source is connected at the second inlet.

The clearing of the alarm is further dependent on the reading exceeding the predetermined threshold within a second predetermined time period.

The second predetermined time period is about 1 s to about 30 s.

The second predetermined time period is about 5 s.

If the reading does not exceed the predetermined threshold, the second alarm is maintained, the second alarm being an alarm associated with the receipt of no supplemental gases from the supplemental gases source by the device.

If the second alarm is maintained, detection test for the connection of a supplemental gases source continues to be operated.

The predetermined threshold is a threshold of gas composition.

The predetermined threshold is a threshold of a concentration of one or more target gas species in the gases flow.

The target gas species is a gas species of the supplemental gases.

The target gas species is a gas species of both the supplemental gases and the gases of the first gases source.

The target gas species is oxygen.

The predetermined threshold is a threshold of oxygen composition of the gases flow.

The predetermined threshold is a threshold of oxygen composition of the gases flow in excess of an ambient air oxygen concentration.

The threshold of oxygen composition is a composition of about 21%.

The threshold of oxygen composition is a composition of about 25%.

The gases composition sensor measures an oxygen concentration in a gases flow past the gases composition sensor.

The device comprises a third inlet for receiving a supplemental gases flow.

The second inlet is for receiving a supplemental gases flow of higher pressure than the supplemental gases flow of the third inlet.

The device receives a supplemental gases flow from the second inlet when the device is operated in a high pressure mode.

The device receives a supplemental gases flow from the third inlet when the device is operated in a low pressure mode.

The second inlet and third inlet are of different configurations, such as to allow the connection of a gases source connector to only one of the second inlet and the third inlet.

The second inlet and third inlet are sized differently to each other.

The device comprises a pressure sensor associated with the second inlet, and a signal from the pressure sensor is indicative of the connection of a supplemental gases source at the second inlet, such that the connection of a supplemental gases source at the second inlet may be determined independent of a pulsing of the valve.

The pressure sensor is located upstream of the valve of the second inlet.

The method further comprising the step of operating the alternative detection substantially continuously independent of an operational mode of the device.

The alternative detection is operated independently of a delivery of therapy by the device.

The pressure sensor is located downstream of the valve of the second inlet.

The device comprises a gases flow sensor, and the detection of the connection of a supplemental gases source at the second inlet may alternatively be determined:
a) by an increase in sensed flow above a threshold subsequent to and within a fourth predetermined time of the pulsing of the valve, and
b) independent of the reading or comparison of a signal from the gases composition sensor.

The method is operated by a controller.

The target threshold of gas composition is settable by a user.

The first inlet is an ambient air inlet.

The first gases source is an ambient air source.

The second inlet is an oxygen inlet.

The second inlet is a high pressure oxygen inlet.

The supplemental gases source is a pressurised gases source.

The supplemental gases source is an oxygen source.

The triggering of the first alarm is further dependent on the connection of the supplemental gases source at the second inlet.

The valve is operated to attain the target threshold of gas composition based on the gases composition reading from the gases composition sensor.

The gases composition sensor comprises an ultrasonic transducer sensor.

The gases composition sensor is located downstream of the second inlet.

The gases composition sensor is located downstream of both the first inlet and the second inlet.

The device comprises a gases mixer for mixing a gases flow from the first inlet and at least one other inlet, and gases mixer is disposed upstream of the gases composition sensor.

The device is or comprises a flow generator for generating a flow of gases from the inlets to an outlet.

The flow generator comprises a blower.

A or the gases mixer is comprised by the blower.

According to another aspect, the present disclosure provides a **device for delivery of a flow of gases,** the device comprising
a first inlet for receiving a gases flow from a first gases source,
a second inlet for connection of a pressurised supplemental gases source,
a valve operable to control a flow of gases introduced to the device by the second inlet,
a pressure sensor associated with the second inlet, the pressure sensor located upstream of the valve, and
a controller configured to detect the connection of a supplemental gases source to the second inlet, wherein the controller is configured to:
   receive a pressure reading from the pressure sensor, and
   compare the pressure reading to a threshold pressure, and determine that a supplemental gases source is connected at the second inlet if the reading exceeds the threshold pressure.

The threshold pressure is ambient pressure.

The threshold pressure is a reference value which is greater than an ambient pressure.

The device further comprises a gases composition sensor, and the controller is configured to control the valve based at least in part on a) a target threshold of gas composition and b) a gases composition reading from the gases composition sensor, the controller configured to:
monitor a gases composition reading of the gases composition sensor,
trigger a first alarm if a determination has been made that a supplemental gases source is connected at the second inlet, but the sensed gas species concentration is outside the target threshold gas composition, and
trigger a second alarm if a) the sensed concentration of the gas species is below a threshold concentration, the threshold concentration being the sum of a concentration of the gas species in the gases flow from the first gases source and a predetermined measurement error of the gases composition sensor, and b) the sensed gas species concentration is outside of the target threshold gas composition,
wherein the first alarm and second alarm are different from each other.

The target threshold of gas composition is a target threshold of concentration of the gas species.

The target threshold of gas composition is greater than an initial gases composition reading.

If the sensed gases composition is less than the target threshold of gas composition, as a precursor to the triggering of the first alarm the controller operates the valve to a more open condition.

The operation of the valve to a more open condition comprises a progressive operation of the valve.

Upon the operation of the valve to a more open condition, the controller again checks if the sensed gas species concentration is in excess of the concentration of the gas species in the gases flow from the first gases source, but the sensed gas species concentration is outside the target threshold gas composition.

The operation of the valve ultimately comprises the operation of the valve to a fully open condition.

The controller triggers the first alarm dependent on the valve being at its fully open condition.

The controller triggers the first alarm dependent on the valve having been in its open condition for a first predetermined time period.

The first predetermined time period is about 10 ms to about 1 min.

The first predetermined time period is from about 1 s to about 20 s.

The first gases source is an ambient air source, and the concentration of the gas species in the gases flow from the first gases source comprises a concentration of an ambient air gas species.

The supplemental gas comprises a supply of the gas species, and the gas species is oxygen.

The oxygen concentration in the ambient air is approximately 21%.

The threshold concentration is the sum of 21% and a measurement error of the sensor expressed as a percentage.

The threshold concentration about 22% to about 35%.

The threshold concentration is about 24%.

The first alarm indicates to a user that the target threshold of gas composition cannot be achieved.

The first alarm indicates to a user that the supply of supplemental gases is inadequate to achieve the target threshold of gas composition.

The controller, after the triggering of the first alarm, is configured to clear the first alarm if a sensed gases composition reading reaches or exceeds the target threshold of gas composition.

The second alarm indicates to a user that supplemental gases from the supplemental gases source are not being received by the device.

The first alarm and second alarm each provide a recommendation to a user to check a supplemental gases supply from the supplemental gases source.

The controller in association with the triggering of the second alarm a) deactivates valve control dependent on the target gases composition and the gases composition reading, and b) operates the valve to a closed condition.

The controller is configured to suspend the control of the device based on the target threshold of gas composition and the gases composition reading upon the triggering of the second alarm.

The controller, upon the triggering of the second alarm, is configured to detect the connection of a supplemental gases source by
pulsing the valve from a closed condition to an open condition,
comparing a gases composition reading from the gases composition sensor to a predetermined threshold of gases composition, and
dependent on the reading exceeding the predetermined threshold, clearing the second alarm.

The comparing of the reading to the predetermined threshold occurs within a second predetermined time period.

The comparison of the reading to the predetermined threshold occurs at a second predetermined time period.

The comparison of the reading to the predetermined threshold occurs after a second predetermined time period.

The second predetermined time period is a time subsequent to the pulsing of the valve.

The second predetermined time is a time such as to allow for a mixing of a gases flow from the first inlet and the second inlet at the gases composition sensor.

The second predetermined time period is dependent on a gases flow rate between the first inlet and second inlet and the gases composition sensor.

The second predetermined time period is selected from a time of about 1 s to about 30 s.

The second predetermined time period is approximately 5 s.

The controller upon the clearing of the second alarm reactivates the control of the device based on the target gases composition and the gases composition reading.

The reactivation of the control of the device based on the target gases composition and the gases composition reading is further dependent on the expiry of a time delay.

The time delay is from about 5 s to about 5 min.

The time delay is approximately 1 min.

The reading exceeding the predetermined threshold indicates that a supplemental gases source is connected at the second inlet.

The clearing of the alarm is further dependent on the reading exceeding the predetermined threshold within a second predetermined time period.

The second predetermined time period is about 1 s to about 30 s.

The second predetermined time period is about 5 s.

If the reading does not exceed the predetermined threshold, the controller maintains the second alarm, being an alarm associated with the receipt of no supplemental gases from the supplemental gases source by the device.

If the second alarm is maintained, the controller continues to attempt to detect the connection of a supplemental gases source.

The predetermined threshold is a threshold of gas composition.

The predetermined threshold is a threshold of a concentration of one or more target gas species in the gases flow.

The target gas species is a gas species of the supplemental gases.

The target gas species is a gas species of both the supplemental gases and the gases of the first gases source.

The target gas species is oxygen.

The predetermined threshold is a threshold of oxygen composition of the gases flow.

The predetermined threshold is a threshold of oxygen composition of the gases flow in excess of an ambient air oxygen concentration.

The threshold of oxygen composition is a composition of about 21%.

The threshold of oxygen composition is a composition of about 25%.

The gases composition sensor measures an oxygen concentration in a gases flow past the gases composition sensor.

The device comprises a third inlet for receiving a supplemental gases flow.

The second inlet is for receiving a supplemental gases flow of higher pressure than the supplemental gases flow of the third inlet.

The device receives a supplemental gases flow from the second inlet when the device is operated in a high pressure mode.

The device receives a supplemental gases flow from the third inlet when the device is operated in a low pressure mode.

The second inlet and third inlet are of different configurations, such as to allow the connection of a gases source connector to only one of the second inlet and the third inlet.

The second inlet and third inlet are sized differently to each other.

The gases composition sensor is located downstream of the second inlet.

The gases composition sensor is located downstream of both the first inlet and the second inlet.

The controller operates to detect the connection of a supplemental gases source to the second inlet substantially continuously, independent of the operation of the device into either a therapy mode or a non-therapy mode.

The target threshold of gas composition is settable by a user.

The first inlet is an ambient air inlet.

The first gases source is an ambient air source.

The second inlet is an oxygen inlet.

The second inlet is a high pressure oxygen inlet.

The supplemental gases source is a pressurised gases source.

The supplemental gases source is an oxygen source.

The triggering of the first alarm is further dependent on the connection of the supplemental gases source at the second inlet.

The controller operates the valve to attain the target threshold of gas composition based on the gases composition reading from the gases composition sensor.

The gases composition sensor comprises an ultrasonic transducer sensor.

The gases composition sensor is located downstream of the second inlet.

The gases composition sensor is located downstream of both the first inlet and the second inlet.

The device is a respiratory support device for providing a flow of gases to a patient.

The device comprises a gases mixer for mixing a gases flow from the first inlet and at least one other inlet, and gases mixer is disposed upstream of the gases composition sensor.

The device is or comprises a flow generator for generating a flow of gases from the inlets to an outlet.

The flow generator comprises a blower.

A or the gases mixer is comprised by the blower.

According to another aspect, the present disclosure provides a **method of detecting the connection of a supplemental gases source** to the second inlet of a respiratory therapy device, the respiratory therapy device comprising a first inlet for receiving a gases flow from a first gases source, a second inlet for connection of a pressurised supplemental gases source, a valve operable to control a flow of gases introduced to the device at the second inlet, a pressure sensor associated with the second inlet, the pressure sensor located upstream of the valve, the method comprising the steps of
receiving a pressure reading from the pressure sensor, and
autonomously comparing the pressure reading to a threshold pressure, and
automatically determining that a supplemental gases source is connected at the second inlet if the reading exceeds the threshold pressure and
generating a command signal.

The command signal is indicative that a supplemental gases source is connected at the second inlet.

The threshold pressure is ambient pressure.

The threshold pressure is a reference value which is greater than an ambient pressure.

The device further comprises a gases composition sensor, and the valve is operated based at least in part on a) a target threshold of gas composition and b) a gases composition reading from the gases composition sensor, the method further comprising the steps of:
monitoring a gases composition reading of the gases composition sensor,
generating a first alarm if a determination has been made that a supplemental gases source is connected at the second inlet, but the sensed gas species concentration is outside the target threshold gas composition, and
generating a second alarm if a) the sensed concentration of the gas species is below a threshold concentration, the threshold concentration being the sum of a concentration of the gas species in the gases flow from the first gases source and a predetermined measurement error of the gases composition sensor, and b) the sensed gas species concentration is outside of the target threshold gas composition,
wherein the first alarm and second alarm are different from each other.

The target threshold of gas composition is a target threshold of concentration of the gas species.

The target threshold of gas composition is greater than an initial gases composition reading.

If the sensed gases composition is less than the target threshold of gas composition, as a precursor to the triggering of the first alarm the valve is operated to a more open condition.

The operation of the valve to a more open condition comprises a progressive operation of the valve.

Upon the operation of the valve to a more open condition, checking if the sensed gas species concentration is in excess of the concentration of the gas species in the gases flow from the first gases source, but the sensed gas species concentration is outside the target threshold gas composition.

The operation of the valve ultimately comprises the operation of the valve to a fully open condition.

The triggering of the first alarm is dependent on the valve being at its fully open condition.

The triggering of the first alarm is dependent on the valve having been in its open condition for a first predetermined time period.

The first predetermined time period is about 10 ms to about 1 min.

The first predetermined time period is from about 1 s to about 20 s.

The first gases source is an ambient air source, and the concentration of the gas species in the gases flow from the first gases source comprises a concentration of an ambient air gas species.

The supplemental gas comprises a supply of the gas species, and the gas species is oxygen.

The oxygen concentration in the ambient air is approximately 21%.

The threshold concentration is the sum of 21% and a measurement error of the sensor expressed as a percentage.

The threshold concentration about 22% to about 35%.

The threshold concentration is about 24%.

The first alarm indicates to a user that the target threshold of gas composition cannot be achieved.

The first alarm indicates to a user that the supply of supplemental gases is inadequate to achieve the target threshold of gas composition.

After triggering of the first alarm, the first alarm is cleared if a sensed gases composition reading reaches or exceeds the target threshold of gas composition.

The second alarm indicates to a user that supplemental gases from the supplemental gases source are not being received by the device.

The first alarm and second alarm each provide a recommendation to a user to check a supplemental gases supply from the supplemental gases source.

In association with the triggering of the second alarm a) valve control dependent on the target gases composition and the gases composition reading is deactivated, and b) the valve is operated to a closed condition.

The method further comprising the step of suspending the control of the device based on the target threshold of gas composition and the gases composition reading upon the triggering of the second alarm.

The method further comprising the step of, upon the triggering of the second alarm, detecting the connection of a supplemental gases source by
pulsing the valve from a closed condition to an open condition,
comparing a gases composition reading from the gases composition sensor to a predetermined threshold of gases composition, and
dependent on the reading exceeding the predetermined threshold, clearing the second alarm.

The comparing of the reading to the predetermined threshold occurs within a second predetermined time period.

The comparison of the reading to the predetermined threshold occurs at a second predetermined time period.

The comparison of the reading to the predetermined threshold occurs after a second predetermined time period.

The second predetermined time period is a time subsequent to the pulsing of the valve.

The second predetermined time is a time such as to allow for a mixing of a gases flow from the first inlet and the second inlet at the gases composition sensor.

The second predetermined time period is dependent on a gases flow rate between the first inlet and second inlet and the gases composition sensor.

The second predetermined time period is selected from a time of about 1 s to about 30 s.

The second predetermined time period is approximately 5 s.

The method further comprising the step, upon the clearing of the second alarm, of reactivating the control of the device based on the target gases composition and the gases composition reading.

The reactivation of the control of the device based on the target gases composition and the gases composition reading is further dependent on the expiry of a time delay.

The time delay is from about 5 s to about 5 min.

The time delay is approximately 1 min.

The reading exceeding the predetermined threshold indicates that a supplemental gases source is connected at the second inlet.

The clearing of the alarm is further dependent on the reading exceeding the predetermined threshold within a second predetermined time period.

The second predetermined time period is about 1 s to about 30 s.

The second predetermined time period is about 5 s.

If the reading does not exceed the predetermined threshold, the second alarm is maintained, the second alarm being an alarm associated with the receipt of no supplemental gases from the supplemental gases source by the device.

If the second alarm is maintained, detection steps for detection the connection of a supplemental gases source are continued.

The predetermined threshold is a threshold of gas composition.

The predetermined threshold is a threshold of a concentration of one or more target gas species in the gases flow.

The target gas species is a gas species of the supplemental gases.

The target gas species is a gas species of both the supplemental gases and the gases of the first gases source.

The target gas species is oxygen.

The predetermined threshold is a threshold of oxygen composition of the gases flow.

The predetermined threshold is a threshold of oxygen composition of the gases flow in excess of an ambient air oxygen concentration.

The threshold of oxygen composition is a composition of about 21%.

The threshold of oxygen composition is a composition of about 25%.

The gases composition sensor measures an oxygen concentration in a gases flow past the gases composition sensor.

The device comprises a third inlet for receiving a supplemental gases flow.

The second inlet is for receiving a supplemental gases flow of higher pressure than the supplemental gases flow of the third inlet.

The device receives a supplemental gases flow from the second inlet when the device is operated in a high pressure mode.

The device receives a supplemental gases flow from the third inlet when the device is operated in a low pressure mode.

The second inlet and third inlet are of different configurations, such as to allow the connection of a gases source connector to only one of the second inlet and the third inlet.

The second inlet and third inlet are sized differently to each other.

The method is operated by a controller.

The gases composition sensor is located downstream of the second inlet.

The gases composition sensor is located downstream of both the first inlet and the second inlet.

The detection of the connection of a supplemental gases source to the second inlet is operated substantially continuously, independent of the operation of the device into either a therapy mode or a non-therapy mode.

The target threshold of gas composition is settable by a user.

The first inlet is an ambient air inlet.

The first gases source is an ambient air source.

The second inlet is an oxygen inlet.

The second inlet is a high pressure oxygen inlet.

The supplemental gases source is a pressurised gases source.

The supplemental gases source is an oxygen source.

The triggering of the first alarm is further dependent on the connection of the supplemental gases source at the second inlet.

The operation of the valve to attain the target threshold of gas composition based on the gases composition reading from the gases composition sensor.

The gases composition sensor comprises an ultrasonic transducer sensor.

The gases composition sensor is located downstream of the second inlet.

The gases composition sensor is located downstream of both the first inlet and the second inlet.

The device comprises a gases mixer for mixing a gases flow from the first inlet and at least one other inlet, and gases mixer is disposed upstream of the gases composition sensor.

The device is or comprises a flow generator for generating a flow of gases from the inlets to an outlet.

The flow generator comprises a blower.

A or the gases mixer is comprised by the blower.

The present invention provides a **respiratory therapy apparatus for delivery of** a **gases flow to** a **user,** the apparatus comprising:
a first inlet for receiving a supplementary gases flow,
a second inlet configured to receive a supplementary gases flow,
a blower to receive a gases flow from one or both of the first inlet and second inlet and generate a gases flow for delivery to the user,
a first flow path and second flow path for guiding a gases flow from the first inlet and second inlet respectively to the blower,
a valve and a sensor each located within the second flow path, and
a controller configured to
detect the disconnection of a gases source from the second inlet or the cessation of provision of a supplementary gases flow through the second inlet, and
respond by operating the valve to close the second flow path and/or triggering a first alarm.

The controller detects the disconnection of a gases source from the second inlet or the cessation of provision of a supplementary gases flow through the second inlet based on a signal received from the sensor located within the second flow path.

Subsequent to detecting the disconnection of a gases source from the second inlet or the cessation of provision of a supplementary gases flow through the second inlet the controller triggers the first alarm.

Subsequent to detecting the disconnection of a gases source from the second inlet or the cessation of provision of a supplementary gases flow through the second inlet the controller triggers the first alarm and operates the valve to a closed position to shut the second flow path.

The first alarm indicates that a gases source has been disconnected from the second inlet and/or that a supplementary gases flow has ceased to be provided through the second inlet.

The apparatus further comprises a gases composition sensor to measure a concentration of one or more supplementary gases within the gases flow for delivery to the user.

The gases composition sensor is located downstream of the blower.

Subsequent to responding by operating the valve to a closed condition and/or triggering the first alarm the controller performs the step of comparing a supplementary gas concentration sensed by the gases composition sensor to a threshold of supplementary gas concentration.

There is a time delay between the a) step of detecting the disconnection of a gases source from the second inlet or the cessation of provision of a supplementary gases flow through the second inlet and b) the step of comparing the sensed concentration against the threshold.

The controller operates the valve to close the second flow path and wherein there is a time delay between the step of operating the valve and the step of comparing the sensed concentration against the threshold.

The time delay comprises about 5 seconds to about 45 seconds.

The time delay comprises about 10 seconds to about 30 seconds.

The threshold comprises a decaying threshold.

The threshold decays from an initial threshold concentration which is above a second inlet supplementary gases concentration when supplementary gases are being supplied through the second inlet, and to a final threshold concentration.

The final threshold concentration is below a minimum supplementary gases concentration when supplementary gases are supplied through the first inlet.

The final threshold concentration is above an ambient air concentration of the supplementary gas.

The decay comprises an exponential decay.

There is a time delay between the step of a) detecting the disconnection of a gases source from the second inlet or the cessation of provision of a supplementary gases flow through the second inlet and b) the step of comparing the sensed concentration against the threshold, and wherein the time delay is about 0.5 seconds to about 5 seconds.

The time delay is about 1 second to about 2 seconds.

The threshold decays at a rate slower than an expected rate of flushing of second inlet supplementary gases from the apparatus.

If the sensed supplementary gas concentration exceeds the threshold the controller determines that a supplementary gases flow is being provided through the first inlet.

If the controller determines that a supplementary gases flow is being provided through the first inlet the controller triggers a second alarm.

The second alarm indicates that a supplementary gases flow is being provided through the first inlet.

The second alarm indicates that the apparatus may be operated in a **first mode,** wherein the first mode is dependent on the supply of supplementary gases from the first inlet.

The second alarm includes a prompt by which an operator may instruct the apparatus to be operated in a or the first mode, wherein the first mode is dependent on the supply of supplementary gases from the first inlet.

The second alarm indicates that the apparatus will be operated in a or the first mode.

In the first mode a closed loop gas control function may not be activated.

In the first mode the apparatus delivers a supplementary gases flow from either the first inlet, or both the first inlet and the second inlet.

The apparatus comprises a third mode wherein the apparatus delivers a supplementary gases flow from both of the first inlet and the second inlet.

Subsequent to determining that a supplementary gases flow is being provided through the first inlet the controller operates the apparatus in a **first mode,** the first mode dependent on the supply of supplementary gases from the first inlet.

The apparatus further comprises a **second mode** wherein the controller operates the valve to control a supplementary gases flow delivered by the apparatus.

In the second mode the controller operates the valve to control a supplementary gases flow through the second inlet.

The step of operating in the first mode comprises an operation from the second mode to an operation in the first mode.

In the second mode a closed loop gas control function may be activated.

In the first mode a closed loop gas control function may not be activated.

In the first mode the apparatus delivers a supplementary gases flow from either the first inlet, or both the first inlet and the second inlet.

The sensor in the second flow path comprises a sensor to sense the connection of a supplementary gases source at the second inlet.

The sensor comprises one or more of:
a contact sensor to sense the physical connection of a supplementary gases source at the second inlet,
a magnetic sensor to sense a magnetic proximity associated with the connection of a supplementary gases source at the second inlet,
an electrical conductivity sensor to sense a change in electrical conductivity associated with the connection of a supplementary gases source at the second inlet,
a pressure sensor to sense a pressure of a second inlet connected supplementary gases source, and
a flow sensor to sense a flow of supplementary gases.

The sensor in the second flow path is located upstream of the valve.

The controller determines if a supplementary gases flow is being provided to the second inlet if a sensed value from the sensor is above a threshold.

The sensor is a pressure sensor, and the threshold is a threshold of pressure greater than an ambient air pressure but less than an expected minimum pressure of a supplementary gases source for connection to the second inlet.

The first inlet is for receiving a supplementary gases flow from a relatively low pressure supplementary gases source and the second inlet is for receiving a supplementary gases flow from a relatively high pressure supplementary gases source.

The supplementary gases supplied to the first inlet and the second inlet are the same supplementary gas or gases.

The supplementary gases supplied to the first inlet and the second inlet are different supplementary gases.

The blower mixes gases flow from either or both of the first inlet and second inlet.

The apparatus further comprises an ambient inlet for receiving an ambient gas or gases or ambient air flow.

The apparatus further comprises an ambient flow path from the ambient inlet to the blower.

The blower is configured to mix gases variously from one or more of the first inlet, second inlet and ambient inlet.

The first flow path, second flow path, and a or the ambient flow path terminate at the blower, such that the blower can receive and mix supplementary gases from the first and second flow path and ambient gases from the ambient flow path.

One or more of the first flow path, second flow path, and a or the ambient flow path are flow paths at least partially in common in at least a region towards the blower.

The apparatus comprises a mixed gases flow path located downstream of the blower for receiving mixed gases from the blower.

The gases composition sensor is provided within the mixed gases flow path.

The gases composition sensor is an ultrasonic transducer sensor.

According to another aspect, the present disclosure provides a **method of operating a respiratory therapy apparatus** in response to the disconnection of or cessation of flow from a supplementary gases source associated with a second inlet of the apparatus, the apparatus comprising a first inlet and the second inlet each for receiving respective supplementary gases flows, a blower to receive gases from one or both of the first inlet and second inlet and generate a gases flow for delivery to the user, a first flow path and second flow path for guiding a gases flow from the first inlet and second inlet respectively to the blower, and a valve and a sensor each located within the second flow path, the method comprising
detecting by a hardware processor the disconnection of a gases source from the second inlet or the cessation of provision of a supplementary gases flow through the second inlet, and
responding by operating the valve and/or triggering a first alarm.

The hardware processor detects the disconnection of a gases source from the second inlet or the cessation of provision of a supplementary gases flow through the second inlet based on a signal received from the sensor located within the second flow path.

The apparatus further comprises a gases composition sensor to measure a concentration of one or more supplementary gases within the gases flow for delivery to the user, the gases composition sensor is located downstream of the blower, and wherein subsequent to responding by operating the valve to a closed condition and/or triggering the first alarm the hardware processor performs the step of comparing a supplementary gas concentration sensed by the gases composition sensor to a threshold of supplementary gas concentration.

If the sensed supplementary gas concentration exceeds the threshold the hardware processor determines that a supplementary gases flow is being provided through the first inlet and one or more of:
a) triggers an alarm to notify the user that a supplementary gases flow is being provided through the first inlet,
b) triggers an alarm to notify a user that an operational mode dependent on a supply of supplementary gases flow through the first inlet may be activated,
c) triggers an alarm to notify a user that an operational mode dependent on a supply of supplementary gases flow through the first inlet will or has been activated, and
d) activates an operational mode dependent on a supplementary gases flow being provided through the first inlet.

According to another aspect, the present disclosure provides a **respiratory therapy apparatus for delivery of a gases flow to a user,** the apparatus comprising:
a first inlet for receiving a supplementary gases flow,
a second inlet for receiving a supplementary gases flow,
a blower for receiving a gases flow from one or both of the first inlet and second inlet and generate a gases flow for delivery to the user,
a first flow path and second flow path for guiding a gases flow from the first inlet and second inlet respectively to the blower,
a valve and a sensor each located within the second flow path,
a gases composition sensor to measure a concentration of one or more supplementary gases within the gases flow for delivery to the user, and
a controller to operate the apparatus in a first mode wherein the apparatus delivers a supplementary gases flow from the first inlet by
determining that a supplementary gases flow is not being provided to the second inlet based on a signal received from the sensor located within the second flow path,
comparing a supplementary gas concentration sensed by the gases composition sensor to a threshold of supplementary gas concentration, and
operating in the first mode when the sensed supplementary gas concentration exceeds the threshold.

The respiratory therapy apparatus further comprises a second mode wherein the controller operates the valve to control a supplementary gases flow delivered by the apparatus.

The step of operating in the first mode comprises an operation from the second mode to the first mode.

In the first mode the apparatus delivers a supplementary gases flow from either the first inlet or both the first inlet and the second inlet.

The respiratory therapy apparatus further comprises a third mode wherein the apparatus delivers a supplementary gases flow from both of the first inlet and the second inlet.

Subsequent to the step of determining that a supplementary gases flow is not being provided to the second inlet, the controller triggers a first alarm.

The first alarm indicates that a supplementary gases flow is not being provided to the second inlet.

Subsequent to the step of determining that a supplementary gas is not being provided to the second inlet, the controller operates the valve to close the second flow path.

Before the step of operating in the first mode, the controller triggers a second alarm.

The second alarm indicates that a supplementary gases flow is being provided through the first inlet.

The second alarm indicates that the apparatus may be operated in the first mode.

The second alarm indicates that the apparatus will be operated in the first mode.

The second alarm includes a prompt by which an operator may instruct or approve the apparatus to be operated in the first mode.

The second alarm includes a prompt which must be approved by an operator in order for the controller to operate the apparatus in the first mode.

The threshold comprises a decaying threshold.

There is a time delay between the a) step of detecting the disconnection of a gases source from the second inlet or the cessation of provision of a supplementary gases flow through the second inlet and b) the step of comparing the sensed concentration against the threshold.

The first mode is dependent on a supplementary gases flow through the first inlet.

The respiratory therapy apparatus further comprises a second mode wherein the controller operates the valve to control a supplementary gases flow delivered by the apparatus.

In the second mode the controller operates the valve to control a supplementary gases flow through the second inlet.

The step of operating in the first mode comprises an operation from the second mode to the first mode.

In the second mode a closed loop gas control function may be activated.

In the first mode a closed loop gas control function may not be activated.

The sensor in the second flow path comprises a sensor to sense the connection of a supplementary gases source at the second inlet.

The controller determines if a supplementary gases flow is being provided to the second inlet if a sensed value from the sensor is above a threshold.

The sensor is a pressure sensor, and the threshold is a threshold of pressure greater than an ambient air pressure but less than an expected minimum pressure of a supplementary gases source for connection to the second inlet.

According to another aspect, the present disclosure provides a **controller** comprising a memory configured to store a program and one or more processors configured to execute the program and control a respiratory therapy apparatus to:
detect the disconnection of a supplementary gases source from a second inlet of the respiratory therapy apparatus or the cessation of provision of supplementary gases flow through the second inlet, and
respond to a detected disconnection by operating a valve of the second inlet to close a flow path from the second inlet into the apparatus and/or by triggering a first alarm.

To detect the disconnection or cessation of provision of flow the controller receives a signal from a sensor provided in the flow path from the second inlet into the apparatus, compares the signal against a predetermined threshold value, and returns a positive determination of a disconnection or cessation of provision of flow if the signal or a processed value from the signal is less than the predetermined threshold value.

The controller is further configured to determine that a supplementary gases flow is being provided through a first inlet of the respiratory therapy apparatus based on a sensed supplementary gases concentration by a gases composition sensor exceeding a predetermined threshold of supplementary gas concentration.

According to another aspect, the present disclosure provides a **non-transitory computer-readable medium** that stores therein a program causing a computer to execute a process comprising:
detecting the disconnection of a supplementary gases source from a second inlet of a respiratory therapy apparatus or the cessation of provision of supplementary gases flow through the second inlet, and
responding to a detected disconnection by operating a valve of the second inlet to close a flow path from the second inlet into the apparatus and/or by triggering a first alarm.

The step of detecting the disconnection or cessation of provision of flow comprises receiving a signal from a sensor provided in the flow path from the second inlet into the apparatus, comparing the signal against a predetermined threshold value stored in memory, and returning a positive determination of a disconnection or cessation of provision of flow if the signal or a processed value from the signal is less than the predetermined threshold value.

The process further comprises determining that a supplementary gases flow is being provided through a first inlet based on a sensed supplementary gases concentration by a gases composition sensor exceeding a threshold of supplementary gas concentration stored in memory.

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7).

Embodiments described herein can also be said broadly to relate to the parts, elements and features referred to or indicated in the specification of the application, individually or collectively, and any or all combinations of any two or more of said parts, elements or features, and where specific integers are mentioned herein which have known equivalents in the art to which this invention relates, such known equivalents are deemed to be incorporated herein as if individually set forth.

In this specification, where reference has been made to external sources of information, including patent specifications and other documents, this is generally for the purpose of providing a context for discussing the features of the present invention. Unless stated otherwise, reference to such sources of information is not to be construed, in any jurisdiction, as an admission that such sources of information are prior art or form part of the common general knowledge in the art.

The term "comprising" as used in this specification means "consisting at least in part of". When interpreting statements in this specification which include that term, the features, prefaced by that term in each statement, all need to be present but other features can also be present. Related terms such as "comprise" and "comprised" are to be interpreted in the same manner.

### BRIEF DESCRIPTION OF THE DRAWINGS

The invention will now be described by way of example only and with reference to the drawings in which:
Figure 1A shows in diagrammatic form a respiratory therapy apparatus.
Figure 1B illustrates a sensing circuit board including a flow rate sensor that may be used in a respiratory therapy apparatus.
Figures 1C-1D illustrate schematic diagrams of various ultrasonic transducer configurations for the sensor system using cross-flow beams.
Figures 1E-1F illustrate schematic diagrams of various ultrasonic transducer configurations for the sensor system using along-flow beams.
Figure 2 is a schematic diagram of a closed loop control system.
Figures 3A-D are schematic diagrams of various configurations of a portion of a respiratory support device for providing a flow of gases to a patient.
Figure 4 is a flowchart for the control of a respiratory therapy device.
Figure 5 is a flowchart for the control of a respiratory therapy device.
Figure 6 is a flowchart for the control of a respiratory therapy device.
Figure 7 is a flowchart for the control of a respiratory therapy device.
Figure 8 is a flowchart for the control of a respiratory therapy device.
Figure 9 is a chart illustrating an example of a decaying threshold of a supplementary gases concentration.
Figure 10 is a flowchart for the control of a respiratory therapy device.
Figure 11 is a flowchart for the control of a respiratory therapy device.

### DETAILED DESCRIPTION

Reference is made in detail to an embodiment of the present invention, examples of which is illustrated in the accompanying drawings.

Patients suffering from various health conditions and diseases can benefit from oxygen therapy. For example, patients suffering from chronic obstructive pulmonary disease (COPD), pneumonia, asthma, bronchopulmonary dysplasia, heart failure, cystic fibrosis, sleep apnea, lung disease, trauma to the respiratory system, acute respiratory distress, receiving pre- and postoperative oxygen delivery, and other conditions or diseases can benefit from oxygen therapy. A common way of treating such problems is by supplying the patients with supplemental oxygen to prevent their blood oxygen saturation (SpO2) from dropping too low (e.g., below about 90%). However, supplying the patient with too much oxygen can over oxygenate their blood, and is also considered dangerous. Generally, the patient's SpO2 is kept in a range from about 80% to about 99%, and preferably about 92% to about 96%, although these ranges may differ due to patient conditions. Due to various factors such as respiratory rate, lung tidal volume, heart rate, activity levels, height, weight, age, gender, and other factors, there is no one prescribed level of supplemental oxygen that can consistently achieve a SpO2 response in the targeted range for each patient. Individual patients will regularly need their fraction of oxygen delivered to the patient (FdO2) monitored and adjusted to ensure they are receiving the correct FdO2 to achieve the targeted SpO2. Achieving a correct and consistent SpO2 is an important factor in treating patients with various health conditions or diseases. Additionally, patients suffering from these health problems may find benefit from a system that automatically controls oxygen saturation. The present disclosure is applicable to a wide range of patients that require fast and accurate oxygen saturation control.

The fraction of oxygen delivered to a patient (FdO2) may be controlled manually. A clinician can manually adjust an oxygen supply valve to change the flow rate or fraction of oxygen being delivered to the patient. The clinician can determine SpO2 levels of the patient using a patient monitor, such as a pulse oximeter. The clinician can continue to manually adjust the amount of oxygen being delivered to the patient until the SpO2 level of the patient reaches a determined level.

One problem with current methods is that when the clinician is trying to achieve a specific SpO2 level they would need to alter the FdO2, wait for the SpO2 reading to settle, and then apply further changes to the FdO2 until the SpO2 is at the required level. The repetitive process of altering the FdO2 and waiting for the SpO2 to settle can be a very time consuming process, particularly if multiple patients are requiring the same treatment.

Another problem is the accuracy of the SpO2 that can be achieved. Accuracy of the SpO2 control can be dependent on how fine the increments are for displayed SpO2 and selectable FdO2. The accuracy may be hampered by the increased amount of time required to get increasingly accurate values, as a clinician may get close to the ideal SpO2 and decide not to alter the FdO2 any further.

Another problem is that other factors may cause the patient's SpO2 levels to change over time without any change in FdO2. Patients would need to be regularly checked on and have their FdO2 adjusted in order to maintain their SpO2 at the correct value. This process can be quite time consuming for the clinician. Additionally, if the time between adjustments is too long, the patient can be at risk of their SpO2 drifting too far from the targeted level.

While some systems exist that attempt something similar, many of them are plagued by further problems stemming from difficulties in measuring patient oxygen saturation. Pulse oximeters and similar devices generate a signal that lags far behind the corresponding change in oxygen fraction delivered. Additionally, oxygen saturation readings can become inaccurate due to various factors.

The present disclosure provides for closed loop control of a respiratory therapy apparatus that allows a patient or clinician to set a target SpO2 instead of a target FdO2. The respiratory therapy apparatus can automatically alter the FdO2 of the respiratory therapy apparatus to achieve the targeted SpO2 based on values of target SpO2, current SpO2, and current FdO2. Automatically controlling the FdO2 can help to quickly and accurately adjust the FdO2 until a target SpO2 is achieved. In some configurations, the system can generate a patient specific model for each patient at the initiation of a therapy session. The respiratory therapy apparatus can have greater precision in achieving the targeted SpO2 by adjusting the FdO2, as needed, to stay within the targeted SpO2 range, without being constantly monitored by a clinician.

Reference to a concentration of a gas species or in particular the concentration of a target gas species will be understood to refer at least primarily to a fraction of inspired gas of that gas species. For example, where the target gas is oxygen, the concentration of the target species may be understood to refer to a FiO2 value.

It will be understood that the terms respiratory device, respiratory therapy device, respiratory support device, and similar may be used interchangeably with the term respiratory therapy apparatus.

While generally described as a respiratory support device or respiratory therapy apparatus, it will be appreciated that the described device or apparatus for use herein may be for use with a fluid, or a one or more fluids in combination with one or more gases.

While generally described as oxygen, a supplemental gas may be any one or number of other gases. For example, the supplemental gas may be one or any combination of nitrogen, carbon dioxide, argon, nitrous oxide, or any other gas known to have any therapeutic application.

The present disclosure provides for a respiratory therapy apparatus that can implement one or more closed loop control systems. Features of the closed loop control system may be combined with features of one or more configurations disclosed herein.

The respiratory support device may comprise one or more modes of operation. These modes may include both one or more therapy modes, in which a therapy is delivered for a patient, and one or more non-therapy modes, in which a therapy is not delivered for a patient. The non-therapy modes may comprise one or more standby modes. In a standby mode the device components, except for a user interface 14 may be inactive or off, and the device is awaiting an input. Such an input instruction may for example be a user input to transition to a therapy mode. Other input instructions may include program instructions or other automated internal or external controls systems.

The one or more closed loop control systems are systems which can be operational in one or more therapy modes.

The one or more therapy modes may comprise one or more high pressure modes and one or more low pressure modes, or a combination thereof. A high pressure mode may generally be a mode in which a high pressure supplemental gases flow is to be used in order to provide the desired therapy. Conversely, a low pressure mode may generally be a mode in which a low pressure supplemental gases flow is to be used. For example, high pressure gases may be used to provide the desired therapy. In various configurations, one or more low pressure modes or one or more high pressure modes may additionally utilise the other of a high pressure source and low pressure source. For example, low pressure gases may be used to provide the desired therapy.

Reference to terms such as high or low pressure gas or high or low pressure flow will generally be understood to refer variously to a gas or flow of a high pressure source or low pressure source as described above.

The respiratory therapy apparatus may operate in an automatic therapy mode or a manual therapy mode. In an automatic therapy mode, the controller can automatically control the FdO2 based on a target FdO2 determined based on the target SpO2 and/or measured SpO2. A valve at the oxygen inlet may be connected to the controller that can control the oxygen concentration in gases flow based on a target FdO2. The controller can execute a control algorithm that can measure FdO2 output by the respiratory therapy apparatus. The FdO2 measurement may be taken periodically at a defined frequency, such as a maximum sample rate of the gases concentration sensors or at a lower frequency, or the measurement may be taken aperiodically. The controller can continue to adjust the valve at the oxygen inlet until the measured FdO2 arrives at the target FdO2. The measured FdO2 may be determined by a gases composition sensor.

In a manual therapy mode, the controller can receive a target FdO2 from a clinician or patient, such as via a user interface. The controller can automatically control the FdO2 based on the received target FdO2. The controller can control the oxygen concentration in gases flow by controlling the oxygen inlet valve based on a target FdO2. The controller can execute a control algorithm that can use a measured FdO2 output by the respiratory therapy apparatus (for example, by a gases composition sensor of the respiratory therapy apparatus) as an input to the controller. The FdO2 measurement may be taken periodically at a defined frequency, such as a maximum sample rate of the gases concentration sensors or at a lower frequency, or the measurement may be taken aperiodically. The controller can continue to adjust the valve at the oxygen inlet to drive the measured FdO2 towards the target FdO2. The measured FdO2 may be determined by a gases composition sensor.

The respiratory therapy apparatus may be configured to change from an automatic therapy mode to a manual therapy mode when the SpO2 of the patient is not within an acceptable patient range. In some instances, the respiratory therapy apparatus reverts to a manual therapy mode when the SpO2 of the patient is outside of the patient limits (above or below) or if the patient's SpO2 did not move within the limits within a defined period of time after the start of the therapy session. The respiratory therapy apparatus may revert to a manual therapy mode when the signal quality of the patient sensor is below a threshold level for a defined period of time. In some configurations, the respiratory therapy apparatus may trigger an alarm when it switches from an automatic therapy mode to a manual therapy mode. In some configurations, the respiratory therapy apparatus may trigger an alarm when the signal quality of the patient sensor is below a threshold level for a defined period of time. The respiratory therapy apparatus may continue to function in an automatic therapy mode after the alarm is triggered. The respiratory therapy apparatus may provide the user, through a graphical user interface, with an option to disable the alarm or to exit the automatic therapy mode.

In an automatic therapy mode, the controller may utilize two control loops. The first control loop can determine a target FdO2 based on the target SpO2. The second control loop can use the target FdO2 output by the first control loop and measured FdO2 to output an oxygen inlet valve control signal. In a manual therapy mode the controller may only use the second the control loop, the second control loop can receive a target FdO2 output from user input or a default value.

During a high flow respiratory therapy session, the oxygen concentration measured in the device, fraction of delivered oxygen (FdO2), can be substantially the same as the oxygen concentration the user is breathing, fraction of inspired oxygen (FiO2), when the flow rate of gas delivered meets or exceeds the peak inspiratory demand of the patient. This means that the volume of gas delivered by the device to the patient during inspiration meets, or is in excess of, the volume of gas inspired by the patient during inspiration. High flow respiratory therapy helps to prevent entrainment of ambient air when the patient breathes in, as well as flushing the patient's airways of expired gas. So long as the flow rate of delivered gas meets or exceeds peak inspiratory demand of the patient, entrainment of ambient air is prevented and the gas delivered by the device, FdO2, is substantially the same as the gas the patient breathes in, FiO2.

In such a therapy apparatus capable of one or more closed loop control systems, the patient or clinician may automatically set a target SpO2, which the control system operates to attempt to achieve. While such a configuration may provide for improved therapy such as by reducing or eliminating the need to monitor and adjust FdO2 by the patient or clinician, there are multiplicities of other factors which may interfere with the desired operation of such a closed loop control system. For example, these may relate to user set up of the therapy device including supplemental gases source connections, the type, quantity, and quality of such supplemental gases being delivered, and the correct operation of components of the therapy apparatus itself. These factors may also interfere with the operation of a device which automatically adjusts a valve of the gases source in order to achieve a desired closed loop control characteristic.

An example of such a user error may be the user setting up the device without attaching a high pressure gases source to the high pressure inlet. If this happened, the device may provide an alarm during therapy if the device is not able to control the valve of the second inlet to achieve a target FiO2 value, regardless of how much the valve is opened. However, such an alarm will necessarily only be triggered once the device attempts to deliver the therapy, and more likely after some time of attempting to deliver the therapy. If this occurs, the clinician may have already left and be required to return and fix the fault in order for the patient to receive the therapy. According to various aspects described herein, a device may be provided where the device to be able to determine whether the supplementary gases source is connected during the setup of the device, such that device can notify the user of the lack of a high pressure gases source either before or at the beginning of the therapy session.

The foregoing issues may also apply to a user error in the connection of an incorrect gases source, for example the connection of nitrous oxide to the device instead of a desired source such as high pressure oxygen. This may present a health risk. According to various aspects described herein, a device may be provided where the device to be able to determine whether the desired supplementary gases source is connected during the setup of the device, such that device can notify the user of the lack of the desired high pressure gases source either before or at the beginning of the therapy session.

In addition, the delivery of supplemental gases such supplemental oxygen may carry with them safety considerations. The nature of oxygen gas as an oxidiser and the strong reactivity of oxygen with numerous other substances are examples. Therefore in some configurations it may be desirable for a therapy apparatus providing a supplemental gas, particularly supplemental oxygen, to be able to implement safety controls or sensing around the delivery of the supplemental oxygen.

For example, safety concerns may arise when the device is operated from a therapy mode, being a mode in which therapy by supplemental oxygen delivery provided, to a non-therapy mode, being a mode in which supplemental oxygen is not delivered. Cessation of the therapy mode may result in entrained supplemental oxygen within the therapy apparatus. Such entrained gases may present a fire or other safety risk.

When a therapy apparatus capable of providing supplemental oxygen is in a non-therapy mode and particularly a standby mode in which the device is not in use by a user, supplemental oxygen is not to be supplied. However, undesired or inadvertent delivery of supplemental oxygen could occur in various circumstances. For example, a supplemental gases source may be incorrectly connected or undesirably left supplying a gases flow to the apparatus although it is in a non-therapy mode. Also, a fault within the apparatus could result in the leakage of the supplemental oxygen through the apparatus. Any such faults could present the possibility of supplemental oxygen being entrained within the apparatus, or being delivered by the apparatus outside of a therapy setting. Dependent on the nature of the supplemental gas and particularly in the case of supplemental oxygen as previously described, these situations may present fire or safety risks.

In another example, additionally or alternatively, safety concerns may arise when high concentrations of oxygen are provided to patient for example oxygen toxicity (i.e. retinopathy of prematurity). Therefore it may be important to recognise the oxygen concentration of the gases, and/or the source of supplemental gas.

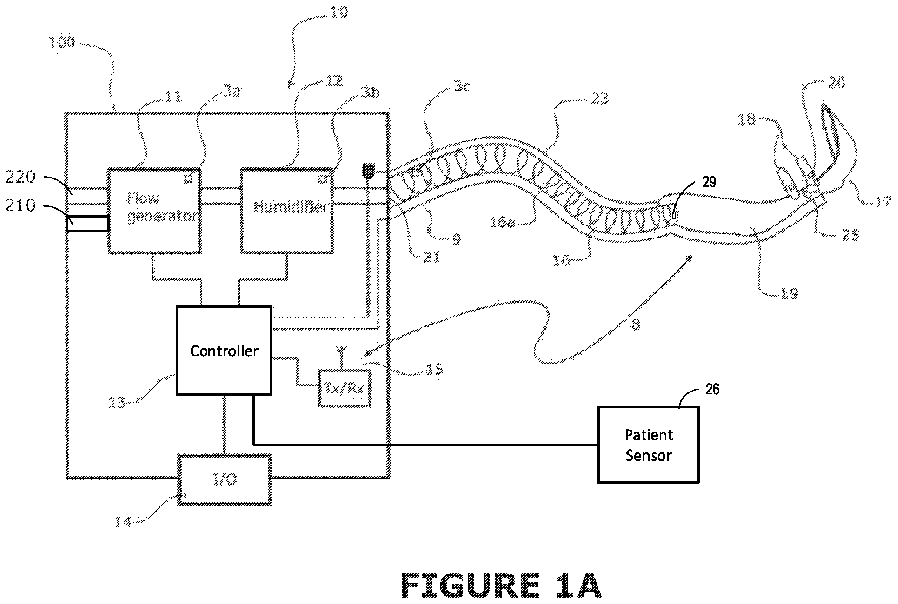

A respiratory therapy apparatus 10 is shown in Figure 1A. The apparatus 10 can comprise a main housing 100 that contains a flow generator 11 in the form of a motor/impeller arrangement (for example, a blower), an optional humidifier 12, a controller 13, and a user interface 14 (comprising, for example, a display and input device(s) such as button(s), a touch screen, or the like). The controller 13 can be configured or programmed to control the operation of the apparatus. For example, the controller can control components of the apparatus, including but not limited to: operating the flow generator 11 to create a flow of gas (gases flow) for delivery to a patient, operating the humidifier 12 (if present) to humidify and/or heat the generated gases flow, control a flow of oxygen into the flow generator blower, receiving user input from the user interface 14 for reconfiguration and/or user-defined operation of the apparatus 10, and outputting information (for example on the display) to the user. The user can be a patient, healthcare professional, or anyone else interested in using the apparatus. As used herein, a "gases flow" can refer to any flow of gases that may be used in the breathing assistance or respiratory device, such as a flow of ambient air, a flow comprising substantially 100% oxygen, fa flow comprising some combination of ambient air and oxygen, and/or the like.

The controller 13 may be implemented as a purely hardware controller, as a software regime running on controller hardware, or as software operational on other non-dedicated controller hardware of the device. Alternatively, it may be implemented as any number of combinations of the foregoing implementation examples.

In various forms the controller 13 may include a processor and a memory.

It will be understood that the various detection, operation, sensing, comparison, enabling or disabling, triggering, pulsing, monitoring, receiving, determining and such like steps by the controller 13 or as part of a method of operation of the device may be carried out autonomously, automatically, or dynamically. For example, the steps or various of the steps may be conducted by the controller, whether as a hardware, hardware and software, or internal or external system implementation, independent of any other input or control signal. As a further example, they may be conducted automatically in response to one or more precursor steps or preconditions.

Where a method or control step or structural element is described to be associated with another method or control step or structural element, this will generally be understood to indicate a relationship between the two features. In particular, relation to method or control steps, it may indicate a relationship of antecedence, dependence, subsequency, or general connection to the method or controls steps. In relation to structural elements, it may indicate a functional association between the structural elements, such as a working relationship to provide a particular result or a connection or synergy. Alternatively, in relation to structural elements it may indicate a direct physical relationship between the two structural elements. Where appropriate, further meaning of the term "associated" in relation to such elements or steps is to be understood from the surrounding context.

A patient breathing conduit 16 is coupled at one end to a gases flow outlet 21 in the housing 100 of the respiratory therapy apparatus 10. The patient breathing conduit 16 is coupled at another end to a patient interface 17 such as a non-sealed nasal cannula with a manifold 19 and nasal prongs 18. Additionally, or alternatively, the patient breathing conduit 16 can be coupled to a face mask, a nasal mask, a nasal pillows mask, an endotracheal tube, a tracheostomy interface, and/or the like. The gases flow that is generated by the respiratory therapy apparatus 10 may be humidified, and delivered to the patient via the patient conduit 16 through the cannula 17. The patient conduit 16 can have a heater wire 16a to heat gases flow passing through to the patient. The heater wire 16a can be under the control of the controller 13. The patient conduit 16 and/or patient interface 17 can be considered part of the respiratory therapy apparatus 10, or alternatively peripheral to it. The respiratory therapy apparatus 10, breathing conduit 16, and patient interface 17 together can form a respiratory therapy system.

The controller 13 can control the flow generator 11 to generate a gases flow of the desired flow rate. The controller 13 can also control a supplemental oxygen inlet to allow for delivery of supplemental oxygen, the humidifier 12 (if present) can humidify the gases flow and/or heat the gases flow to an appropriate level, and/or the like. The gases flow is directed out through the patient conduit 16 and cannula 17 to the patient. The controller 13 can also control a heating element in the humidifier 12 and/or the heating element 16a in the patient conduit 16 to heat the gas to a desired temperature for a desired level of therapy and/or level of comfort for the patient. The controller 13 can be programmed with or can determine a suitable target temperature of the gases flow.

The second inlet 220, in some embodiments an oxygen inlet, can include a valve through which a pressurized gas may enter the flow generator or blower. The valve can control a flow of oxygen into the flow generator blower. The valve can be any type of valve, including a proportional valve or a binary valve. The source of oxygen can be an oxygen tank or a hospital oxygen supply. Medical grade oxygen is typically between 95% and 100% purity. Oxygen sources of lower purity can also be used. Examples of valve modules and filters are disclosed in U.S. Provisional Application No. 62/409,543, titled "Valve Modules and Filter", filed on October 18, 2016, and U.S. Provisional Application No. 62/488,841, titled "Valve Modules and Filter", filed on April 23, 2017.

The respiratory therapy apparatus 10 can measure and control the oxygen content of the gas being delivered to the patient, and therefore the oxygen content of the gas inspired by the patient. During high flow therapy, the high flow rate of gas delivered meets or exceeds the peak inspiratory demand of the patient. This means that the volume of gas delivered by the device to the patient during inspiration meets, or is in excess of, the volume of gas inspired by the patient during inspiration. High flow therapy therefore helps to prevent entrainment of ambient air when the patient breathes in, as well as flushing the patient's airways of expired gas. So long as the flow rate of delivered gas meets or exceeds peak inspiratory demand of the patient, entrainment of ambient air is prevented, and the gas delivered by the device is substantially the same as the gas the patient breathes in. As such, the oxygen concentration measured in the device, fraction of delivered oxygen, (FdO2) would be substantially the same as the oxygen concentration the user is breathing, fraction of inspired oxygen (FiO2), and as such the terms may can be seen as equivalent.

Operation sensors 3a, 3b, 3c, such as flow, temperature, humidity, and/or pressure sensors can be placed in various locations in the respiratory therapy apparatus 10. Additional sensors (for example, sensors 20, 25) may be placed in various locations on the patient conduit 16 and/or cannula 17 (for example, there may be a temperature sensor 29 at or near the end of the inspiratory tube). Output from the sensors can be received by the controller 13, to assist the controller in operating the respiratory therapy apparatus 10 in a manner that provides suitable therapy. In some configurations, providing suitable therapy includes meeting a patient's peak inspiratory demand. The apparatus 10 may have a transmitter and/or receiver 15 to enable the controller 13 to receive signals 8 from the sensors and/or to control the various components of the respiratory therapy apparatus 10, including but not limited to the flow generator 11, humidifier 12, and heater wire 16a, or accessories or peripherals associated with the respiratory therapy apparatus 10. Additionally, or alternatively, the transmitter and/or receiver 15 may deliver data to a remote server or enable remote control of the apparatus 10.

Oxygen may be measured by placing one or more gas composition sensors (such as an ultrasonic transducer system, also referred to as an ultrasonic sensor system) after the oxygen and ambient air have finished mixing. The measurement can be taken within the device, the delivery conduit, the patient interface, or at any other suitable location.

Oxygen concentration may also be measured by using flow rate sensors on at least two of the ambient air inlet conduit, the oxygen inlet conduit, and the final delivery conduit to determine the flow rate of at least two gases. By determining the flow rate of both inlet gases or one inlet gas and one total flow rate, along with the assumed or measured oxygen concentrations of the inlet gases (about 20.9% for ambient air, about 100% for oxygen), the oxygen concentration of the final gas composition can be calculated. Alternatively, flow rate sensors can be placed at all three of the ambient air inlet conduit, the oxygen inlet conduit, and the final delivery conduit to allow for redundancy and testing that each sensor is working correctly by checking for consistency of readings. Other methods of measuring the oxygen concentration delivered by the respiratory therapy apparatus 10 can also be used.

The respiratory therapy apparatus 10 can include a patient sensor 26, such as a pulse oximeter or a patient monitoring system, to measure one or more physiological parameters of the patient, such as a patient's blood oxygen saturation (SpO2), heart rate, respiratory rate, perfusion index, and provide a measure of signal quality. The sensor 26 can communicate with the controller 13 through a wired connection or by communication through a wireless transmitter on the sensor 26. The sensor 26 may be a disposable adhesive sensor designed to be connected to a patient's finger. The sensor 26 may be a non-disposable sensor. Sensors are available that are designed for different age groups and to be connected to different locations on the patient, which can be used with the respiratory therapy apparatus. The pulse oximeter would be attached to the user, typically at their finger, although other places such as an earlobe are also an option. The pulse oximeter would be connected to a processor in the device and would constantly provide signals indicative of the patient's blood oxygen saturation. The patient sensor 26 can be a hot swappable device, which can be attached or interchanged during operation of the respiratory therapy apparatus 10. For example, the patient sensor 26 may connect to the respiratory therapy apparatus 10 using a USB interface or using wireless communication protocols (such as, for example, near field communication, WiFi or Bluetooth^{®}). When the patient sensor 26 is disconnected during operation, the respiratory therapy apparatus 10 may continue to operate in its previous state of operation for a defined time period. After the defined time period, the respiratory therapy apparatus 10 may trigger an alarm, transition from an automatic therapy mode to a manual therapy mode, and/or exit control mode (e.g., automatic therapy mode or manual therapy mode) entirely. The patient sensor 26 may be a bedside monitoring system or other patient monitoring system that communicates with the respiratory therapy apparatus 10 through a physical or wireless interface.

The respiratory therapy apparatus 10 may comprise a high flow respiratory therapy apparatus (for example a high respiratory therapy apparatus). High flow therapy as discussed herein is intended to be given its typical ordinary meaning as understood by a person of skill in the art which generally refers to a respiratory assistance system delivering a targeted flow of humidified respiratory gases via an intentionally unsealed patient interface with flow rates generally intended to meet or exceed inspiratory flow of a patient. Typical patient interfaces include, but are not limited to, a nasal or tracheal patient interface. Typical flow rates for adults often range from, but are not limited to, about fifteen liters per minute (LPM) to about seventy liters per minute or greater. Typical flow rates for pediatric patients (such as neonates, infants and children) often range from, but are not limited to, about one liter per minute per kilogram of patient weight to about three liters per minute per kilogram of patient weight or greater. High flow therapy can also optionally include gas mixture compositions including supplemental oxygen and/or administration of therapeutic medicaments. High flow therapy is often referred to as nasal high flow (NHF), humidified high flow nasal cannula (HHFNC), high flow nasal oxygen (HFNO), high flow therapy (HFT), or tracheal high flow (THF), among other common names.

The flow rates used to achieve "high flow" may be any of the flow rates listed below. For example, in some configurations, for an adult patient 'high flow respiratory therapy' may refer to the delivery of gases to a patient at a flow rate of greater than or equal to about 10 litres per minute (10 LPM), such as between about 10 LPM and about 100 LPM, or between about 15 LPM and about 95 LPM, or between about 20 LPM and about 90 LPM, or between 25 LPM and 75 LPM, or between about 25 LPM and about 85 LPM, or between about 30 LPM and about 80 LPM, or between about 35 LPM and about 75 LPM, or between about 40 LPM and about 70 LPM, or between about 45 LPM and about 65 LPM, or between about 50 LPM and about 60 LPM. In some configurations, for a neonatal, infant, or child patient 'high flow respiratory therapy' may refer to the delivery of gases to a patient at a flow rate of greater than 1 LPM, such as between about 1 LPM and about 25 LPM, or between about 2 LPM and about 25 LPM, or between about 2 LPM and about 5 LPM, or between about 5 LPM and about 25 LPM, or between about 5 LPM and about 10 LPM, or between about 10 LPM and about 25 LPM, or between about 10 LPM and about 20 LPM, or between about 10 LPM and 15 LPM, or between about 20 LPM and 25 LPM. A high flow respiratory therapy apparatus with an adult patient, a neonatal, infant, or child patient, may deliver gases to the patient at a flow rate of between about 1 LPM and about 100 LPM, or at a flow rate in any of the sub-ranges outlined above. The respiratory therapy apparatus 10 can deliver any concentration of oxygen (e.g., FdO2), up to 100%, at any flowrate between about 1 LPM and about 100 LPM. In some configurations, any of the flowrates can be in combination with oxygen concentrations (FdO2s) of about 20%-30%, 21%-30%, 21%-40%, 30%-40%, 40%-50%, 50%-60%, 60%-70%, 70%-80%, 80%-90%, and 90%-100%. In some combinations, the flow rate can be between about 25 LPM and 75 LPM in combination with an oxygen concentration (FdO2) of about 20%-30%, 21%-30%, 21%-40%, 30%-40%, 40%-50%, 50%-60%, 60%-70%, 70%-80%, 80%-90%, and 90%-100%. In some configurations, the respiratory therapy apparatus 10 may include safety thresholds when operating in a manual therapy mode that prevent a user from delivering too much oxygen to the patient.

High flow respiratory therapy may be administered to the nares of a user and/or orally, or via a tracheostomy interface. High flow respiratory therapy may deliver gases to a user at a flow rate at or exceeding the intended user's peak inspiratory flow requirements. The high flow respiratory therapy may generate a flushing effect in the nasopharynx such that the anatomical dead space of the upper airways is flushed by the high incoming gases flow. This can create a reservoir of fresh gas available for each and every breath, while minimizing re-breathing of nitrogen and carbon dioxide. Meeting inspiratory demand and flushing the airways is additionally important when trying to control the patient's FdO2. High flow respiratory therapy can be delivered with a non-sealing patient interface such as, for example, a nasal cannula. The nasal cannula may be configured to deliver breathing gases to the nares of a user at a flow rate exceeding the intended user's peak inspiratory flow requirements.

The term "non-sealing patient interface" as used herein can refer to an interface providing a pneumatic link between an airway of a patient and a gases flow source (such as from flow generator 11) that does not completely occlude the airway of the patient. Non-sealed pneumatic link can comprise an occlusion of less than about 95% of the airway of the patient. The non-sealed pneumatic link can comprise an occlusion of less than about 90% of the airway of the patient. The non-sealed pneumatic link can comprise an occlusion of between about 40% and about 80% of the airway of the patient. The airway can include one or more of a nare or mouth of the patient. For a nasal cannula the airway is through the nares.

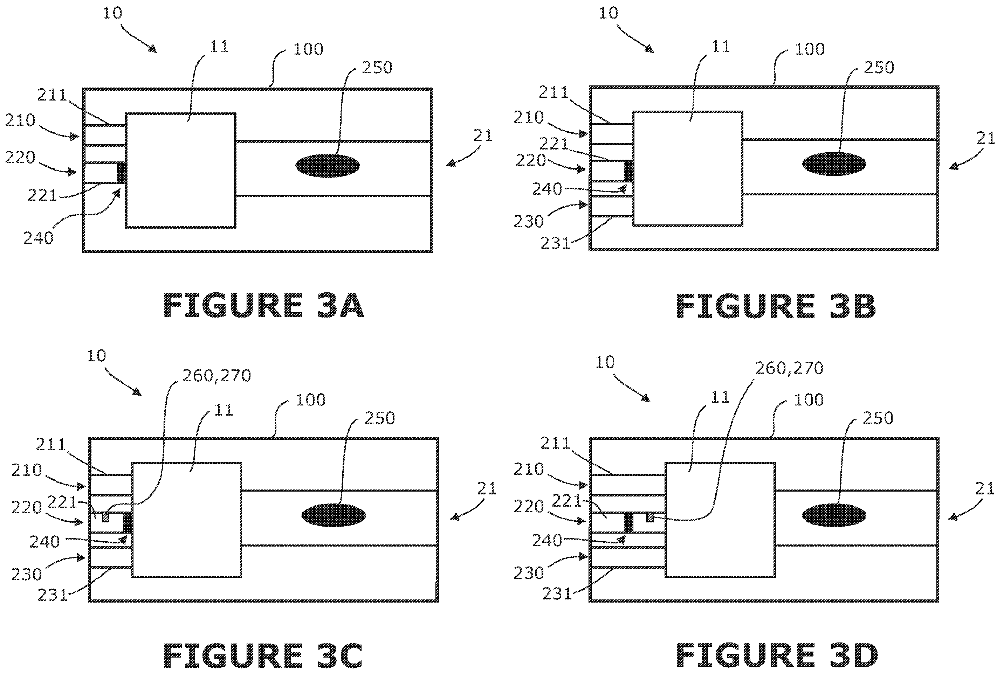

The device has a first inlet 210, such as is seen in Figure 3A-D, for receiving a gases flow from a first gases source. The first gases source may be able to be connected at the first inlet 210. The first inlet may in various forms be an ambient air inlet to entrain ambient room air into the blower. The device may comprise a first inlet port 211.

The device may also include a second inlet 220 for receiving a gases flow from a supplemental gases source. In various embodiments, the second inlet 220 is an oxygen inlet for receiving a supply of oxygen from a pressurised oxygen source. The second inlet 220 may include an oxygen inlet port 221 leading to a valve 240 through which a pressurized gas may enter the flow generator or blower 11. The valve 240 can control a flow of oxygen into the flow generator blower 11. The valve can be any type of valve, including a proportional valve or a binary valve.

An example schematic configuration of a portion of a respiratory support device having both an ambient air inlet 210 and a second inlet 220 for a supplementary gas such as oxygen is seen in Figure 3A.

The device may also include a third inlet 230 for receiving a supplemental gases flow from a supplemental gases source. Examples of a device 10 having such a configuration are seen in any one of Figures 3B-D. This supplemental gases source may be the same as the source for the second inlet 220, or may be a different source. For example, as seen in the configuration of Figure 3B, the supplemental gases source of the second inlet 220 may be a high pressure oxygen source while the third inlet 230 source is a relatively lower pressure source. In general, high pressure refers to a gases source that needs to be further regulated by a valve of the device, while a low pressure gases source refers to a gases source where the pressure or flow has already been titrated externally of the device to a target amount, such as by a valve on the gases source itself.

In such a configuration where the device has a second inlet 220 and third inlet 230, and only the second inlet 220 is for receiving a flow from a high pressure gases source, only the second inlet 220 may comprise a second inlet port 221 leading to a valve 240 for control of the introduction of the pressurised gas into the device. Such a configuration is shown in Figure 3B, for example. Alternatively, the gases sources may be sources of a different gas and either or both of the second inlet 220 and third inlet 230 may comprise a valve 240. Independently of comprising a valve 240, the third inlet 230 may also comprise a third inlet port 231 of similar configuration to the second inlet port 221.

A gases composition sensor 250 is preferably located downstream of the second inlet port 221 and valve 240, such that a flow of supplementary gases to the gases composition sensor is controllable by the valve 240.

The gases composition sensor 250 may further be downstream of both the first inlet 210 and second inlet port 221 and valve 240. Such a configuration may be desirable to allow for a mixing of first inlet and second inlet gases prior to the point at which they reach the gases composition sensor 250.

In one configuration the third inlet 230 is for receiving a relatively low pressure gases supply as compared to that of the second inlet 220. In such a configuration, the third inlet 230 gases source may be pressure regulated upstream of the device, such as by a manual valve operable by a user.

Where the second inlet 220 and third inlet 230 are for receiving between them a high pressure gases flow and a low pressure gases flow, at least some therapy modes may be defined by the gases source upon which they at least in part rely, or upon which their control regime is based. For example, the therapy modes may comprise a high pressure mode wherein the high pressure gases source is to be utilised, and a low pressure mode where in the low pressure gases source is to be utilised.

The inlet ports may be for the attachment of their respective supplementary gases sources. To this end, the inlet ports may comprise respective connector portions to interface with connector portions of their supplemental gases sources. In various configurations, the second inlet port 221 and third inlet port 231 are of different configurations, such as to discourage or prevent the connection of the third inlet supplementary gases source to the second inlet 220, or the second inlet supplementary gases source to the third inlet 230. To this end, the second inlet port 221 and third inlet port 231 may be sized or externally configured differently to each other. Where the supplementary gases sources are different, for example a high pressure source and a low pressure source, this configuration may help prevent the inadvertent connection of the correct gases source to an incorrect inlet, or an incorrect gases source to the correct inlet. This may as a result remove failure modes of the therapy delivery.

The device may in various configurations comprise a pressure sensor 260 associated with the second inlet 220 and more particularly with the second inlet port 221 which leads to the valve 240. The pressure sensor 260 may for example be partially or entirely contained within the second inlet port 221. The pressure sensor 260 may be located adjacent the second inlet port 221, or may penetrate through a body of the second inlet port 221 so as to position the pressure sensor within the port. The controller 13 is configured to receive sensor information from the pressure sensor 260. By being located upstream of the valve 240, such as seen in Figure 3C, the pressure sensor may sense a pressure associated with a connected supplemental gases source independent of an open or closed state of the valve 240. Where the pressure sensor 260 is located upstream of the valve 240 in the inlet port there may be requirements for sealing of the pressure sensor in the inlet port. This may be the case where the supplemental gas may present some safety risks, such as in the case of oxygen. Any need for sealing may additionally be heightened in cases where the device receives an input of a pressurised supplemental gas. For example, the sensor itself may penetrate a body of the inlet port, or may have one or more control or power wires which may pass through a body of the inlet.

In alternate configurations, the pressure sensor 260 may be located at or downstream of the valve 240. An example of such a configuration is seen in Figure 3D. In such a configuration, the location downstream of the inlet port may lessen or eliminate the requirements for sealing of the sensor within the inlet port.

The blower can operate at a motor speed of greater than about 1,000 RPM and less than about 30,000 RPM, greater than about 2,000 RPM and less than about 21,000 RPM, or between any of the foregoing values. For example, the blower can operate at a motor speed of greater than about 4,000 RPM and less than about 19,000 RPM, or greater than about 6,000 RPM and less than about 17,000 RPM. Operation of the blower can mix the gases entering the blower through the inlet ports. Using the blower as the mixer can decrease the pressure drop that would otherwise occur in a system with a separate mixer, such as a static mixer comprising baffles, because mixing requires energy.

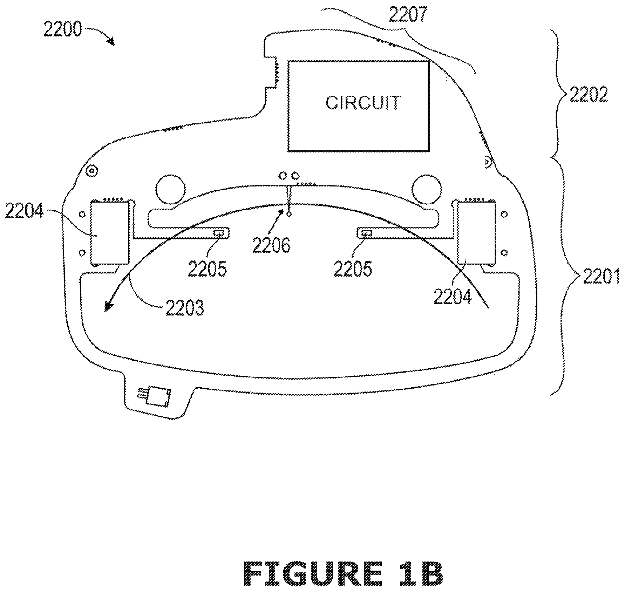

With additional reference to Figure 1B, a sensing circuit board 2200 is shown that can be implemented in the respiratory therapy apparatus 10. The sensing circuit board 2200 can be positioned in a sensor chamber such that the sensing circuit board 2200 is at least partially immersed in the flow of gases. The flow of gases may exit the blower 11 through a conduit and enter a flow path in the sensor chamber. At least some of the sensors on the sensing circuit board 2200 can be positioned within the flow of gases to measure gas properties within the flow. After passing through the flow path in the sensor chamber, the gases can exit to the humidifier 12 described above.

The sensing circuit board 2200 can be a printed sensing circuit board (PCB). Alternatively, the circuit on the board 2200 can be built with electrical wires connecting the electronic components instead of being printed on a circuit board. At least a portion of the sensing circuit board 2200 can be mounted outside of a flow of gases. The flow of gases can be generated by the flow generator 11 described above. The sensing circuit board 2200 can comprise ultrasonic transducers 2204. The sensing circuit board 2200 can comprise one or more of thermistors 2205. The thermistors 2205 can be configured to measure a temperature of the gases flow. The sensing circuit board 2200 can comprise a thermistor flow rate sensor 2206. The sensing circuit board 2200 can comprise other types of sensors, such as humidity sensors including humidity only sensors to be used with a separate temperature sensor and combined humidity and temperature sensors, sensors for measuring barometric pressure, sensors for measuring differential pressure, and/or sensors for measuring gauge pressure. The thermistor flow rate sensor 2206 can comprise hot wire anemometer, such as a platinum wire, and/or a thermistor, such as a negative temperature coefficient (NTC) or positive temperature coefficient (PTC) thermistor. Other non-limiting examples of the heated temperature sensing element include glass or epoxy-encapsulated or non-encapsulated thermistors. The thermistor flow rate sensor 2206 can be configured to measure flow rate of the gases by being supplied with a constant power, or be maintained at a constant sensor temperature or a constant temperature difference between the sensor and the flow of gases.

The sensing circuit board 2200 can comprise a first portion 2201 and a second portion 2202. The first portion 2201 can be positioned to be within the flow path of the gases, whereas the second portion 2202 can be positioned to be outside the flow path of the gases. The direction of the flow of gases is indicated in Figure 1B by the arrow 2203. The direction of the flow of gases can be a straight line, or curved in shown in Figure1B.

Positioning the one or more of thermistors 2205 and/or the thermistor flow rate sensor 2206 downstream of the combined blower and mixer can take into account heat supplied to the gases flow from the blower. Also, immersing the temperature-based flow rate sensors in the flow path can increase the accuracy of measurements because the sensors being immersed in the flow can more likely to be subject to the same conditions, such as temperature, as the gases flow and therefore provide a better representation of the gases characteristics.

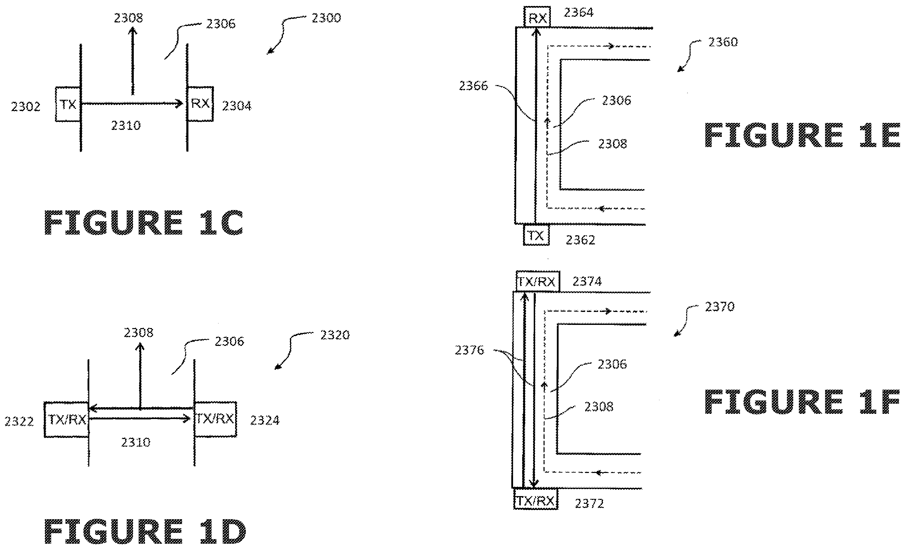

The sensing circuit board 2200 can comprise ultrasonic transducers, transceivers, or sensors of the sensing circuit board to measure gases properties of the gases flow, such as gas composition or concentration of one or more gases within the gases stream. Any suitable transducer, transceiver, or sensor may be mounted to the sensing circuit board 2200 as will be appreciated. In this configuration, the sensing circuit board includes an ultrasonic transducer system (also referred to as an ultrasonic sensor system) that employs ultrasonic or acoustic waves for determining gas concentrations. Various sensor configurations are described below with respect to Figures 1C-1F.

The ultrasonic transducer system may determine the relative gas concentrations of two or more gases in the gases flow. The ultrasonic transducer system may be configured to measure the oxygen fraction in the bulk gases stream flow, which consists of atmospheric air augmented with supplemental oxygen, which is essentially a binary gas mixture of nitrogen (N2) and oxygen (O2). It will also be appreciated that the ultrasonic transducer system may be configured to measure the gas concentrations of other augmentation gases that have blended with atmospheric air in the gases stream, including nitrogen (N2) and carbon dioxide (CO2). The ultrasonic transducers can determine the gas concentration of gases in the gases flow at a relatively high frequency. For example, the ultrasonic transducers can output a measured FdO2 value at a maximum sample rate of the sensors or at a lower frequency than the maximum sample rate, such as between about 1 Hz and 200 Hz, about 1 Hz and 100 Hz, about 1 Hz and 50 Hz, and about 1 Hz and 25 Hz.

In some configurations, sensing circuit board 2200 includes a pair of ultrasonic transducers that are provided on opposite sides of the sensing circuit board. Various alternative configurations of the ultrasonic transducers can be used for sensing the characteristics of the gases stream by the transmission and reception of ultrasonic beams or pulses.

The distance between the ultrasonic transducers 2204 on opposite ends of the sensing circuit board 2200 can affect measurement resolution. An increased distance between each of the ultrasonic transducers 2204 can reduce the proportional or fractional error, since in general a measured length will have a certain amount of error, and if the length is increased, the proportion of error generated during measurement is less than for a shorter length. Thus, the overall uncertainty of the measurement decreases. An increased distance can also increase measurement resolution and accuracy, since it allows for a longer time period for acoustic signals between the ultrasonic transducers 2204. However, an increased distance can lead to a weaker signal.

The ultrasonic transducers 2204 can be positioned such that the space between the ultrasonic transducers 2204 at least partially coincides with the flow path. In some configurations, the ultrasonic transducers are positioned on opposing ends of the sensing circuit board. Because the whole face of the flow path is exposed to the acoustic path, the sound waves propagate through all of the gases in the flow path. Averaging of the waves can occur across the entire flow path rather than a section of the flow path. Averaging over a longer distance reduces error and reduces the dependence of air-oxygen mixing. The ultrasonic transducers can be configured to measure the gases characteristics from any angle relative to the flow path.

Positioning sensors in the flow path or module, instead of outside the flow path or module, allows the transducers 2204 to both operate within a smaller temperature range relative to one another, or both substantially at one temperature (namely, the temperature of the gas flow). Having them at a substantially homogenous temperature increases accuracy as the transducers are sensitive to temperature. Further, positioning sensors along the flow path allows for measurements and calculations that account for the influence of the gas velocity so that the effect of gas velocity can be removed from the sensor measurement.

The ultrasonic transducer system is configured as an ultrasound binary gas sensing system. Binary gas analysis using ultrasound is based on sensing the speed of an acoustic pulse through the gas sample, which in this case is the bulk or primary flow of the gases stream flowing through sensing passage of the sensor housing. The speed of sound is a function of gas mean molecular weight and temperature. The system can receive a sensor signal indicative of the temperature of the gases flowing between the beam path between ultrasonic transducers. With knowledge of sensed speed of sound and sensed temperature, the gas composition in the gases stream may be determined or calculated. In particular, measurements of the speed of sound across the sensing passage may be used to infer the ratios of two known gases by reference to empirical relationships, standard algorithms, or data stored in the form of look-up tables, as is known in the art of binary gas analysis with ultrasound. It will be appreciated that alternatively an estimate of the temperature of the gases stream in the beam path of the ultrasound transducers may be used in the binary gas analysis calculations if a temperature sensor is not employed. In such alternative embodiments, the temperature of the gases stream may be conditioned or controlled to within a narrow temperature band to enable an estimate of temperature of the gases stream in the beam path to be used.

In some configurations, the respiratory therapy apparatus may also be provided with a humidity sensor that is located in the flow path and which is configured to generate a humidity signal indicative of the humidity of the gases stream flowing through the sensor assembly. In such embodiments, the gas composition may be determined by the sensed speed of sound, and the sensed temperature and/or sensed humidity. The humidity sensor may be a relative humidity sensor or an absolute humidity sensor. In some embodiments, the gas composition may be determined based on the sensed speed of sound and the sensed humidity, without the need for a temperature sensor.

The ultrasonic transducer system may be used to measure respective ratios of any two known gases in a gas composition. The ultrasonic transducer system can determine the relative gas concentration in a mixture of air blended with supplementary oxygen, which is substantially equivalent to a nitrogen/oxygen mixture. In such a binary gas mixture, by monitoring the speed of sound and taking the temperature into account, the mean molecular weight of the gas can be determined, and thus, the relative concentrations of the two gases may be determined. From this ratio, the oxygen fraction or nitrogen fraction of the gases stream may be extracted.

Referring to Figures 1C-1F, various configurations of the ultrasonic transducers will be described for the gas composition sensing system for sensing the speed of sound through the gases stream by the transmission and reception of ultrasonic beams or pulses. Like reference numerals, represent like components.

Referring to Figure 1C, the transducer configuration 2300 provides an arrangement in which there is a pair of transducers 2302, 2304 opposing each other and positioned on opposite sides of the sensing passage 2306, with the gases flow path direction indicated generally by 2308. In this configuration, each of the transducers 2302, 2304 is driven as either a dedicated transmitter or receiver, such that ultrasonic pulses 2310 are transmitted uni-directionally across the gases flow path from the transmitter to the receiver transducer. As shown, the transducer pair is aligned (i.e. not-displaced upstream or downstream from each other) relative to the air flow path direction 2308 and is configured to transmit cross-flow pulses that are substantially perpendicular to the gases flow path direction.

Referring to Figure 1D, an alternative transducer configuration 2320 is illustrated in which a pair of transducers 2322, 2324 is provided opposing each other on opposite sides of the sensing passage, but wherein each transducer may operate as both a transmitter and receiver (i.e., the transducer is an ultrasonic transmitter-receiver or transceiver). In this configuration, bi-directional ultrasonic pulses 2326 may be sent between the transducer pair 2322, 2324. For example, pulses may be sent back and forth alternately between the transducers or in any other sequence or pattern. Again, the transducer pair is aligned relative to the gases flow path direction and are configured to transmit cross-flow pulses that are substantially perpendicular to the gases flow path direction.

Referring to Figure 1E, an alternative transducer configuration 2360 is illustrated in which there is a pair of transducers 2362, 2364 opposing each other from opposite ends of the sensing passage 2306, with the gases flow path direction or axis indicated generally by 2308. In this configuration 2360, each of the transducers 2362, 2364 is driven as either a dedicated transmitter or receiver, such that along-flow ultrasonic pulses 2366 are transmitted uni-directionally in a beam path between the transmitter and receiver that is substantially aligned or parallel with the gases flow path axis 2308 in the sensing passage 2306. In the embodiment shown, the transmitter is upstream of the receiver, but it will be appreciated that the opposite arrangement could be employed. With this configuration, a flow rate sensor is provided in the sensing passage to provide a flow rate signal indicative of the flow rate of the gases stream in the sensing passage. It will be appreciated that the speed of sound in the sensing passage can be derived or determined in a similar manner to that previously described, and that the flow rate signal is utilized in the signal processing to remove or compensate for the gases flow rate in the calculated speed of sound signal.

Referring to Figure 1F, an alternative transducer configuration 2370 is illustrated in which a pair of transducers 2372, 2374 is provided opposing each other from opposite ends of the sensing passage like in Figure 1E, but wherein each transducer may operate as both a transmitter and receiver, i.e. is an ultrasonic transmitter-receiver or transceiver. In this configuration, bi-directional along-flow ultrasonic pulses 2376 may be sent between the transducer pair 2372, 2374. For example, pulses may be sent back and forth alternately between the transducers or in any other sequence or pattern. Again, the transducer pair are aligned with the gases flow path axis 2308 and are configured to transmit along-flow pulses in a beam path or paths that are substantially aligned or parallel to the gases flow path axis 2308 in the sensing passage 2306. With this configuration, a separate flow rate sensor need not necessarily be provided, as the flow rate component of the speed of sound signal can be directly derived or determined from processing of the transmitted and received acoustic pulses.

Some examples of respiratory therapy apparatuses are disclosed in International Application No. PCT/NZ2016/050193, titled "Flow Path Sensing for Flow Therapy Apparatus", filed on December 2, 2016, and International Application No. PCT/IB2016/053761, titled "Breathing Assistance Apparatus", filed on June 24, 2016.

With reference again to Figure 1A, the controller 13 can be programmed with or configured to execute a closed loop control system for controlling the operation of the respiratory therapy apparatus. The closed loop control system can be configured to ensure the patient's SpO2 reaches a target level and consistently remains at or near this level.

The controller 13 can receive input(s) from a user that can be used by the controller 13 to execute the closed loop control system. Other input instructions may include program instructions or other automated internal or external controls systems. The target SpO2 value can be a single value or a range of values. The value(s) could be pre-set, chosen by a clinician, or determined based on the type of patient, where type of patient could refer to current affliction, and/or information about the patient such as age, weight, height, gender, and other patient characteristics. Similarly, the target SpO2 could be two values, each selected in any way described above. The two values would represent a range of acceptable values for the patient's SpO2. The controller can target a value within said range. The targeted value could be the middle value of the range, or any other value within the range, which could be pre-set or selected by a user. Alternatively, the range could be automatically set based on the targeted value of SpO2. The controller can be configured to have one or more set responses when the patient's SpO2 value moves outside of the range. The responses may include alarming, changing to manual control of FdO2, changing the FdO2 to a specific value, and/or other responses. The controller can have one or more ranges, where one or more different responses occur as it moves outside of each range.

The graphical user interface of the respiratory therapy apparatus may be configured to prompt the user to input a patient type, and the SpO2 limits would be determined based on what the user selects. Additionally, the user interface may include a custom option, where the user can define the limits.

Generally, SpO2 would be controlled between about 80% and about 100%, or about 80% and about 90%, or about 88% and about 92%, or about 90% and about 99%, or about 92% and about 96%. The SpO2 could be controlled between any two suitable values from any two of the aforementioned ranges. The target SpO2 could be between about 80% and about 100%, or between about 80% and about 90%, or between about 88% and about 92%, or between about 90% and about 99%, or between about 92% and about 96%, or about 94%, or 94% or about 90%, or 90%, or about 85%, or 85%. The SpO2 target could be any value between any two suitable values from any two of the aforementioned ranges. The SpO2 target can correspond to the middle of the SpO2 for a defined range.

The FdO2 can be configured to be controlled within a range. As discussed previously, the oxygen concentration measured in the apparatus (FdO2) would be substantially the same as the oxygen concentration the patient is breathing (FiO2) so long as the flow rate meets or exceeds the peak inspiratory demand of the patient, and as such the terms may can be seen as equivalent. Each of the limits of the range could be pre-set, selected by a user, or determined based on the type of patient, where the type of patient could refer to current affliction, and/or information about the patient such as age, weight, height, gender, and/or other patient characteristic. Alternatively, a single value for FdO2 could be selected, and the range could be determined at least partially based on this value. For example, the range could be a set amount above and below the selected FdO2. The selected FdO2 could be used as the starting point for the controller. The system could have one or more responses if the controller tries to move the FdO2 outside of the range. These responses could include alarming, preventing the FdO2 moving outside of the range, switching to manual control of FdO2, and/or switching to a specific FdO2. The device could have one or more ranges where one or more different responses occur as it reaches the limit of each range.

FdO2 can be controlled between about 21% and about 100%, or about 21% and about 90%, or about 21% and about 80%, or about 21% and about 70%, or about 21% and about 60%, or about 21% and about 50%, or about 25% and about 45%. The FdO2 could be controlled between any two suitable values from any two ranges described. The FdO2 target could be between any two suitable values from any two ranges described. If the range is based on the single value, the upper and lower limits could be decided by adding/subtracting a fixed amount from the selected value. The amount added or subtracted could be about 1%, or about 5%, or 10 %, or about 15%, or about 20%, or about 30%, or about 50%, or about 100%. The amount added/subtracted could change relative to the selected value. For example, the upper limit could be 20% higher than the selected value, so a selected value of 50% FdO2 would have an upper limit of 60% for the range of control. The percentage used for the range could be about 1%, or about 5%, or 10 %, or about 15%, or about 20%, or about 30%, or about 50%, or about 100%. The method for calculating the lower limit and the upper would not necessarily need to be the same. If a single value is used, the value could be between about 21% and about 100%, or about 25% and about 90%, or about 25% and about 80%, or about 25% and about 70%, or about 25% and about 60%, or about 25% and about 50%, or about 25% and about 45%.

The graphical user interface 14 (GUI) can be configured to display the range of values between which FdO2 and/or SpO2 are being controlled. The range could be displayed by having the two limits set apart from each other on the GUI, with an indicator appearing within the range to graphically represent the position of the current value with respect to the limits of the range.

The GUI can display graphs of recent FdO2 and/or SpO2 data. The GUI can display the level of each parameter on the same or different graphs over a defined period of time, such as one or more hours. The length of time over which data is displayed could match the length of the time for which data is currently available.

FdO2 data displayed can be at least one of the target FdO2 or the measured FdO2. The SpO2 data can include a line indicating target SpO2. Additionally, or alternatively, SpO2 and/or FdO2 data can include one or more lines or shaded areas indicating their respective control limits.

The graphs can be displayed on the default display. Alternatively, the graphs can be hidden with only current data values being shown. The graph can become available through interaction with the GUI, such as by selecting to view a graph for a defined parameter.

Where the device is operable in different therapy modes, or is operable between a standby mode and a therapy mode, the instruction to toggle between any such modes may be provided at the user interface.

The configuration of the controller 13 may be such that it receives manual input from a user by a user interface 14 in or supplemental to the execution of a closed loop control system. In particular, the controller 13 may receive input from a user by an input device such as one or more buttons, a touch screen, or the like. The controller may also be pre-programmed to provide the input, or may receive an input from another internal or external system.

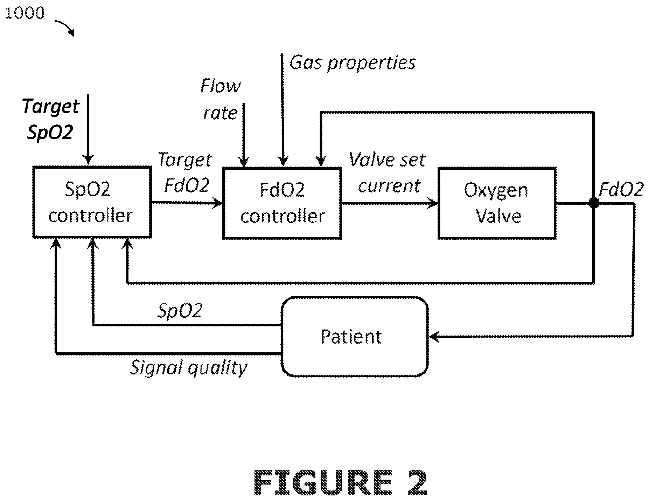

With reference to Figure 2 a schematic diagram of the closed loop control system 1000 is illustrated. The closed loop control system may utilize two control loops. The first control loop may be implemented by the SpO2 controller. The SpO2 controller can determine a target FdO2 based in part on the target SpO2 and/or the measured SpO2. As discussed above, the target SpO2 value can be a single value or a range of acceptable values. The value(s) could be pre-set, chosen by a clinician, or determined automatically based on client characteristics. Generally, target SpO2 values are received or determined before or at the beginning of a therapy session, though target SpO2 values may be received at any time during the therapy session. During a therapy session, the SpO2 controller can also receive as inputs: measured FdO2 reading(s) from a gases composition sensor, and measured SpO2 reading(s) and a signal quality reading(s) from the patient sensor. In some configurations, the SpO2 controller can receive target FdO2 as an input, in such a case, the output of the SpO2 controller may be provided directly back to the SpO2 controller as the input. Based at least in part on the inputs, the SpO2 controller can output a target FdO2 to the second control loop.

The second control loop may be implemented by the FAO2 controller. The FAO2 controller can receive inputs of measured FdO2 and target FdO2. The FdO2 controller can then output an oxygen inlet valve control signal to control the operation of the oxygen valve based on a difference between these measured FdO2 and target FdO2 values. The FdO2 controller may receive the target FdO2 value that is output from the first control loop when the respiratory therapy apparatus is operating in an automatic therapy mode. The FdO2 controller may also receive additional parameters such as flow rate values, gas properties, and/or measured FdO2. The gas properties may include the temperature of the gas at the O2 inlet and/or the oxygen content of the supply source. The gases supply source connected to the oxygen inlet valve may be an enriched oxygen gas flow where the oxygen content of the supply source may be less than pure oxygen (i.e., 100%). For example, the oxygen supply source may be an oxygen enriched gas flow having an oxygen content of less than 100% and greater than 21%.

From at least some of the inputs, the FdO2 controller can determine an oxygen flow rate that would be required to achieve the target FdO2. The FdO2 controller can use the flow rate input in order to alter the valve control signal. If the flow rate changes, the FdO2 controller can automatically calculate a new required oxygen flow rate required to maintain the target FdO2 at the new flow rate without having to wait for feedback from the gas concentration sensor, such as the measured FdO2 value. The FdO2 controller can then output the altered valve control signal to control the valve based on the new flow rate. In some configurations, the control signal of the FdO2 controller may set the current of the oxygen valve in order to control operation of the oxygen valve. Additionally, or alternatively, the FdO2 controller could detect changes to the measured FdO2 and alter the position of the valve accordingly. During a manual therapy mode, the second control loop can operate independently without receiving the target FdO2 from the first control loop. Rather, the target FdO2 can be received from user input or a default value.

During the therapy session, the SpO2 and FdO2 controllers can continue to automatically control the operation of the respiratory therapy apparatus until the therapy session ends or an event triggers a change from an automatic therapy mode to a manual therapy mode.

As previously described, in some closed loop control systems the device may measure and adjust the fraction of delivered oxygen FdO2 in order to meet the requirements of the closed loop control, such as meeting a target FiO2 or FdO2 level, or a target blood oxygen saturation SpO2.

It will be appreciated that the FiO2 value may be equal to the FdO2 value in circumstances where the flow rate provided by the device meets or exceeds the inspiratory demand from the patient.

Some examples of respiratory therapy apparatuses and closed loop control systems are disclosed in International Application No. PCT/NZ2018/050137, titled "Closed loop oxygen control", filed on 5 October 2018**.**

In various operational conditions it may be desirable to detect the connection of a supplemental gases source at an inlet, such as the second inlet 220. For example, it may be desirable to determine the connection status at the second inlet 220 upon the powering on of the device, or the operation of the device in to a therapy mode such as a therapy mode where the use of the supplemental gases from the second inlet 220 is necessary or desired. An example of such a therapy mode may be the operation of the device in a closed loop control system, being an automatic therapy mode, or the operation of the device with a target oxygen concentration in excess of ambient levels, being a manual therapy mode. In either mode pressurised oxygen may be needed to be supplied and controlled by the device in order to provide desired therapy outcomes.

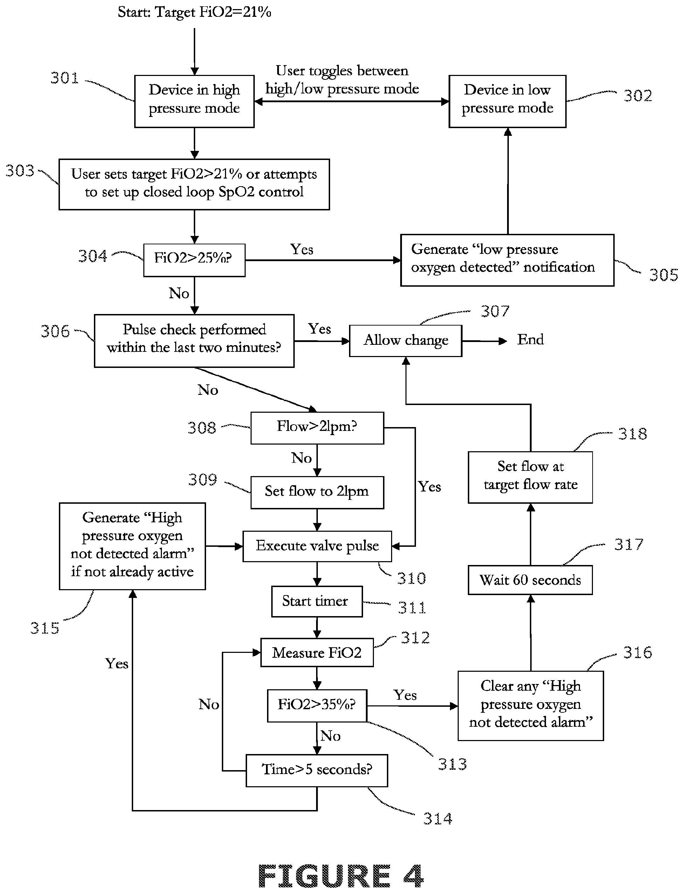

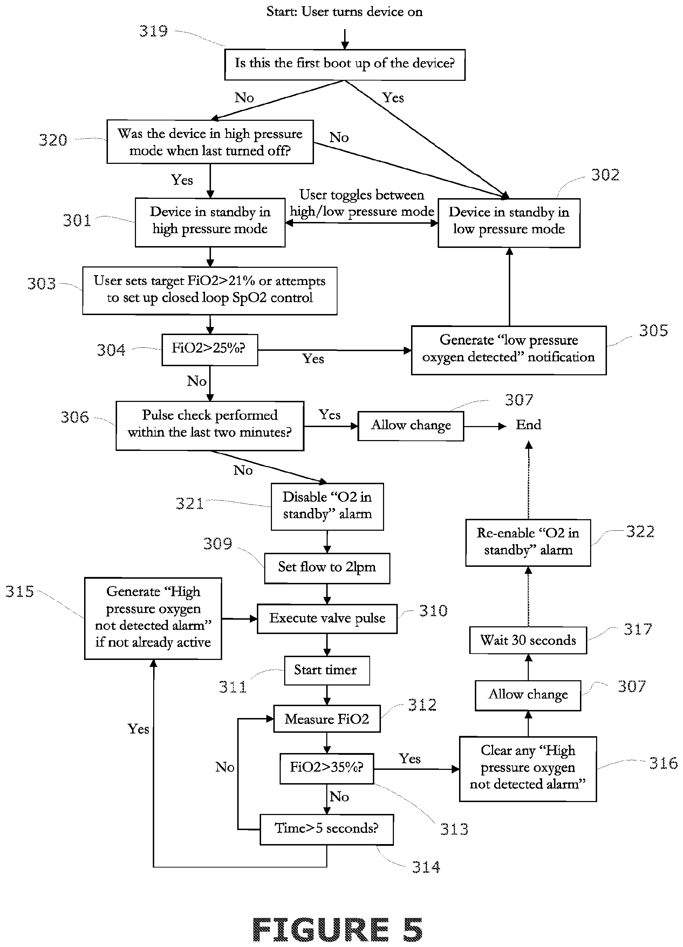

Figures 4 and 5 illustrate flowcharts for the control of a respiratory therapy device, and in particular the control of such a device in the detection of the connection of a supplemental gases source at the second inlet 220 at the operation of the device into a high pressure therapy mode.

As seen in Figure 4, the device can be operated in either a high pressure mode in block 301 or a low pressure mode, and a user may toggle between these two modes, such as by an input at a user interface 14. If the device is in or is toggled into the high pressure mode of block 301, a user may optionally instruct the device to either enter into a closed loop oxygen control system or instruct the setting of a target oxygen concentration of an amount greater than an ambient level, for example an oxygen concentration of greater than 21%. This is shown in block 303. Alternatively, the controller may receive such an instruction by programmed instructions of the device or other automated internal or external controls systems.

Alternatively, in some configurations the selection of a target oxygen concentration in greater than ambient or the selection of closed loop oxygen control may cause the device to be operated into the high pressure mode.

Directly subsequent to block 303, in block 304, the controller may check if a sensed oxygen concentration of gases flow is in excess of a threshold. The threshold is preferably a value in excess of 21%, such as a threshold between about 22% and about 35%. This sensed oxygen concentration may be indicative of the presence of supplemental oxygen, such as from a third inlet 230. Such a supplemental oxygen source may be a low pressure oxygen source, and the flow from the source may not be able to be controlled by the device. If such a threshold is exceeded, the device may generate a notification that a low pressure oxygen source has been detected at block 305, and operate the device into a low pressure mode as seen at block 302. The generation of the notification and operation of the device into the low pressure mode may be further dependent on the valve 240 of the second inlet 220 being in a closed condition, or having been in a closed condition for a certain period of time preceding the determination of block 304.

This functionality may be desirable where the provision of the therapy is to utilise only a supplemental gases flow from a high pressure source, such as is in some configurations to be connected at the second inlet 220. In such a situation, the delivery of supplemental gases from a low pressure source may hinder the provision of the desired therapy, for example because the device is not operable to control the pressure and or flow of the gases from a low pressure gases source. Alternatively, however, in various high pressure therapy modes it may be allowable or even desirable for a low pressure gases flow to be provided, and the functionality of block 304 and 305 may not be utilised.

Provided that the threshold is not exceeded, the device as shown at block 306 may check the time since a valve pulse test was last conducted, and if a pulse check was conducted sufficiently recently, allow the operation of the device into the closed loop oxygen control system or the setting of the target oxygen concentration in excess of ambient. This is shown at block 307. If a valve pulse test was not conducted within the predetermined threshold time, then the controller instructs a further valve pulse test. The steps associated with such a valve pulse test are shown in blocks 310-314. At block 310 the controller 13 operates the valve 240 through a pulse from a closed condition, to an open condition, and back to a closed condition. The open condition may be a fully open condition, or an only partially open condition. The open condition may reflect the setting of a valve operation current to a maximum allowable or maximum achievable value. The open condition may not be a fully open condition, but rather a threshold determined based on a total maximum flow through the valve and the predetermined threshold of oxygen concentration required for the determination. The purpose of the valve pulse is to allow a pulse of supplemental gases from the second inlet port 221 - if they are present - to pass from the inlet port 221, through the valve 240, and into the device. Once part of the flow of gases through the device, the supplemental gases from the pulse may be sensed by a gases composition sensor 250. From a sensed increase in a level of the supplemental gases as at block 312, the controller may determine that a supplemental gases source is present at the second inlet 220. The sensed level in order to make a determination that a supplemental gases source is present and the valve pulse time may each be set dependent on the other respective value. For an example oxygen concentration threshold of 35% to make a positive determination, the valve pulse may involve a total time, or a total time of the valve at its open condition, of about 100 ms to about 1 s, or about 300 ms.

Making such a determination based on the sensed oxygen concentration may be delayed for a predetermined time period, as at block 311 and 314. Such a time period may be a time so as to allow for passage of the gases flow containing any supplemental gases inlet at the valve 240 to reach the gases composition sensor 250 and/or to allow time for an adequate mixing of the first inlet and second inlet gases. This predetermined time may be set based at least in part on a flow condition of the device.

As at block 313, if the sensed oxygen concentration is not in excess of a predetermined threshold then the process proceeds to block 315 and the controller can trigger an alarm indicating that a high pressure oxygen source has not been detected. This alarm may further specify that the high pressure oxygen source has not been detected at the second inlet 220. The controller 13 may continue to operate the valve pulse test following a negative determination. This continued operation of the pulse test may continue until the controller determines that a supplemental gases source is connected, or a user intervenes. Such a user intervention may for example be the manual connection of a supplemental gases source at the second inlet 220, the operation of the device to a low pressure mode responsive to the notification of block 315, the cancellation of the instruction to enter a closed loop oxygen control regime, the setting of a target oxygen concentration at ambient level, or an instruction for the device to enter a standby mode. In other configurations, the intervention may be an automated intervention, such as by a preprogramed system of the device or an external control system.

In some configurations, the controller may allow a user to override the notification of block 315 by inputting an acknowledgement the notification or operating a function to mute or ignore the notification. Overriding the notification would have the result of bypassing the control steps of blocks 310-313, and instead allowing the entry into closed loop oxygen control or the setting of the target oxygen concentration in excess of 21% (not shown in Figure 4).

If the predetermined threshold of oxygen concentration at block 313 is met or exceeded, the process may continue to block 316 wherein the controller clears any alarm indicating that a high pressure oxygen source was not detected. Subsequent to this clearing, the controller may as at block 317 delay for a predetermined time such as to allow supplemental gases introduced into the device by the valve pulse to be flushed from the device. This predetermined time may be set based at least in part on a flow condition of the device. Following any such delay, the controller will operate the blower to a target flow rate at block 318, then finally allow the change to the selected closed loop oxygen control system or target oxygen concentration in excess of ambient at block 307.

As a precursor to the valve pulse test, as seen at blocks 308 and 309 the controller may operate the blower to a flow condition above a minimum level. For example, it may operate the blower to a flow condition in excess of 2 litres per minute.

As a further precursor to the valve pulse test, it is desirable that the operation of the device into a closed loop oxygen control system or setting of a target oxygen concentration in excess of 21% causes the controller to operate the valve to a closed condition. It may further be preferable that the controller operates the valve to a closed condition by default, unless the opening of the valve is instructed as part of a high pressure therapy mode control regime.

The alarm at block 315 may be triggered in a number of circumstances. For example it may occur where a supplemental gases source is not connected at the second inlet 220. It may occur when a supplemental gases source is connected but no gases are being delivered by the supplemental gases source. It may also occur when there is a fault with the valve, such that the valve did not open to allow supplemental gases past it and to the gases composition sensor.

Where the device comprises a pressure sensor 260, the controller may be configured to receive sensor information from the pressure sensor 260, and make a determination of the connection of a supplemental gases source at the second inlet based on the sensor information and independent of a pulsing of the valve 240. In such a configuration, the controller 13 may determine that a supplemental gases source is or is not connected at the second inlet based solely on the pressure sensor information. Alternatively, a combination of pressure sensor and pulsing of the valve may be utilised, for example the controller may utilise the pressure sensor information as a confirmation of a determination made by a valve pulse test. Further alternatively, pressure sensor information may be used as a prompt for the device to execute a valve pulse test.

The controller 13 may operate such a pressure test by reading the pressure information of the pressure sensor continuously or substantially continuously. This pressure test may further be operated continuously or substantially continuously independent of any operational mode of the device.

The device may also comprise a flow sensor 270, to sense a flow of gases through the device. The controller may be configured to detect the connection of a supplemental gases source at the second inlet by the reading of an increased gases flow by the flow sensor 270 within a predetermined time of the pulsing of the valve. This determination may be independent of the reading of a signal from the gases composition sensor and/or the reading of any pressure sensor information.

Subsequent to a detected connection of a high pressure supplemental gases source at the second inlet 220, the controller will enter the associated high therapy mode.

As seen in Figure 5, a similar control process to what has been described in relation to the entry into a high pressure mode or switching from a low pressure mode to a high pressure mode may also be conducted upon start-up of the device.

Prior to block 319, the user provides power to the device and/or an input to the device to cause it to be operational. At block 319 the controller 13 checks if this is the first time that the device has been started up. If it is the first time the device has been started up, the device may be set to a low therapy mode and put into standby, waiting for a user instruction to activate a therapy. This is shown at block 302. Alternatively, if it is not the first start-up of the device, the controller 13 may check a previous therapy mode state of the device at block 320, and consequently operate the device in standby but configured for a low therapy mode at block 302, or a high therapy mode at block 301. As described in relation to the process of Figure 4, the device may be able to be toggled by a user between the selection of a high pressure therapy mode or a low pressure therapy mode.

Subsequent operation of the device by the controller may be substantially as described in relation to Figure 4 for blocks 303 and 304. If an excess of the threshold of oxygen is not detected, the device may proceed to the pulse test status check of 306, wherein the controller checks if a pulse check was performed sufficiently recently. For example, the controller 13 may check if a pulse check was performed within the last two minutes. If the test has been performed sufficiently recently, the controller 13 may directly allow the selection of the high pressure therapy mode at block 307. At this state, the device may begin operation of the selected high pressure therapy mode, or may remain in a standby mode awaiting a user instruction for the therapy to commence. As previously, this instruction may also be automated, and may be provided by the device itself or by some external control system.

If the pulse test was not performed sufficiently recently, the controller proceeds to execute a valve pulse test at blocks 310-316 as previously described in relation to Figure 4. As a precursor to the beginning of the valve pulse test the controller 13 may additionally operate the blower to a desired flow condition, such as to provide a desired flow of gases from the inlets of the device to the gases flow sensor. For example, this may be a flow rate of about 2 litres per minute. Such an operation may be necessary in the control situation of Figure 5 as the device has begun from an off condition, and the blower may not be activated.

As the device at the beginning of the valve pulse test may still be in a standby mode, where a therapy is not being delivered, it may additionally be desirable to disable any alarm for the detection of supplemental oxygen while in a standby mode. Accordingly, as a precursor to the valve pulse test of blocks 310-316 which may result in the introduction of supplemental oxygen to the system while in standby mode, the controller 13 may disable the triggering of an alarm for the detection of oxygen in a standby mode.

As the device in the process of Figure 5 has been operated from an off state, there may be a reduced amount of supplemental oxygen entrained within the device subsequent to a successful detection of a supplemental gases source at block 315 and 316. Accordingly, the controller 13 may require a relatively reduced delay period to allow for the flushing of the supplemental gases from the device. For example as shown in Figure 5, the controller 13 may delay for about 30 seconds.

If an alarm for the detection of oxygen in a standby mode was disabled at block 321, the controller 13 may re-enable it at block 322.

A detected lack of connection of a supplemental gases source at the second inlet may be due to a variety of possible reasons. For example, these may include:
- The high pressure gases source is not connected to the device.
- A low pressure gases source has been connected to the low pressure gases inlet instead of a high pressure gases source being connected to the high pressure inlet.
- The high pressure gases source was incorrectly connected to the low pressure inlet.
- A low pressure gases source was incorrectly connected to the high pressure inlet.
- The high pressure gases source was incorrectly connected to the device.
- A valve on the high pressure gases source was not opened.
- A blockage has occurred either in the high pressure gases source, or in the device itself.
- The valve at the high pressure inlet is damaged and/or malfunctioning.
- The high pressure gases source has run out (most applicable when an oxygen bottle is being used).
- The wrong high pressure gases source is being used (for example, a high pressure nitrogen source is being used instead of a high pressure oxygen source).

The operation of the respiratory therapy device to detect such fault conditions or issues may provide improved functionality to a respiratory therapy device, such as by improving the likelihood of correct identification of therapy-interfering faults so that they can be remedied and the desired therapy continued.

The source detection test may also be utilised in the operation of the device to assist a user in selecting the correct therapy mode. For example, the use of the source detection test may not allow a user to select a high pressure therapy mode where the supplemental gases source is not available to provide the associated therapy in the high pressure mode.

As previously discussed, the use of various supplemental gases such as supplemental oxygen by the device may present safety risks. For example, if one or more supplemental gases source are connected to the device but the device is not in a therapy mode, for example it is in a standby mode, it may be possible that supplemental oxygen is still passed from the supplemental gas source. This could occur for example by the incorrect or not full closing of a valve 240 in association with a high pressure oxygen source, or the operation external of the device of a low pressure oxygen source to begin to flow. This could occur by a user accidentally or inadvertently opening a valve associated with the low pressure source, the failure of that valve, or the fact that the valve was not shut off when a therapy mode was terminated.

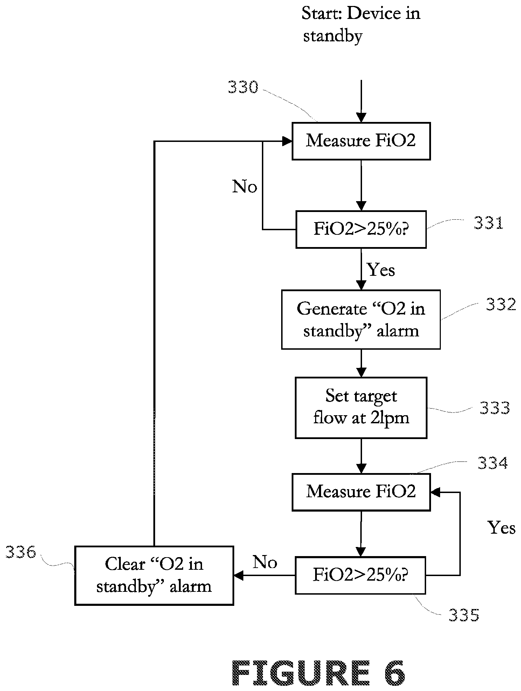

To this end it may be desirable when the device is operated in a standby mode for the controller to operate a test to detect the presence of supplemental oxygen above a predetermined allowable threshold. Figure 6 illustrates a flowchart for the control of a respiratory device for the detection of supplemental oxygen in a standby mode.

At block 330 the device is in a standby mode. The controller 13 is then operated to measure an oxygen concentration sensed at the gases composition sensor 250. If the measured oxygen concentration exceeds a predetermined allowable threshold of oxygen concentration at block 331, the controller proceeds to block 332 and triggers an alarm indicating the detection of supplemental oxygen. Responsive to this alarm, the device may also operate the blower to a predetermined flow rate to assist in purging the supplemental oxygen from the device, as an attempt to reduce or manage any safety risk posed by the supplemental oxygen. The controller 13 may then proceed at block 334 and 335 to continue to measure an oxygen concentration reading from the gases composition sensor 250 until a reading below the predetermined allowable threshold is sensed. Only once the sensed value is below the predetermined threshold may the controller 13 clear the alarm for the detection of oxygen in the standby mode.

Similarly, if a supplemental oxygen level in excess of the predetermined allowable threshold of oxygen is not detected at block 331, the controller 13 may continue to operate the step of blocks 330 and 331 continuously or substantially continuously while the device remains in a standby mode.

The predetermined allowable threshold of oxygen may be any threshold in excess of ambient levels, in normal conditions a level in excess of 21%. In some situations, the threshold may be the sum of the ambient level and a calculated measurement error of the gases composition sensor. For example, if the error was known to be an absolute amount of 3% concentration, the threshold may be set at 24%.

The alarm triggered at block 332 may additionally indicate that supplemental oxygen has been detected from the first supplemental gases source. Such an indication may be dependent on the operation of a valve pulse test as previously described, where the valve pulse test does not result in an increased level of sensed oxygen concentration by the gases composition sensor. Alternatively or in addition, such an indication may be dependent on the controller determining that the valve 240 is in a closed condition.

Another situation in which entrained supplemental gases within the device may be undesirable is following a user direction to cease a therapy mode and enter a standby mode. In the therapy mode, supplemental gases may have been being delivered by the device. As such, when the therapy mode is terminated there may be residual supplemental gases entrained within the device.

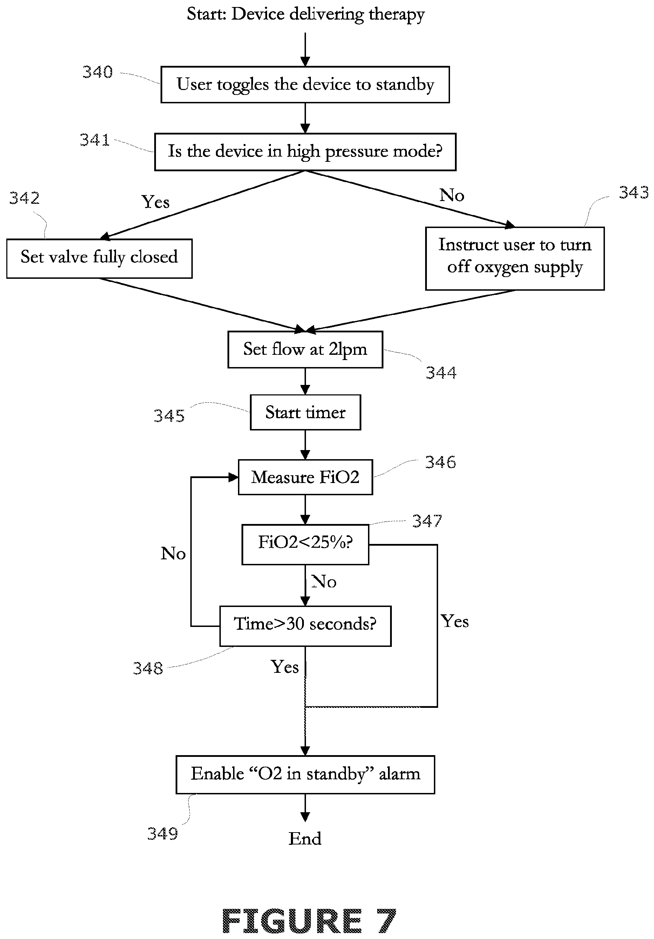

Figure 7 illustrates an example flowchart of a process for the operation of the device from a therapy mode into a standby mode in order to mitigate any potential risks posed by entrained supplemental gases.

Preceding block 340, the device was delivering a therapy in a therapy mode. At block 340 the user toggles the device to a standby mode. This toggling may represent a request from the user to enter the standby mode, but this request may not be acted on and the device may not enter the standby mode until the transition process is completed.

At block 344 the controller 13 sets the blower to a predetermined flow condition such as to assist in the flushing of the supplemental gases from the device. The controller sets a timer at block 345, and may continue to operate the blower at the predetermined flow condition until the expiry of the timer at block 348. The controller 13 may then allow the operation of the device into the standby mode.

In addition to this process, the controller 13 may additionally at blocks 346 and 347 sense a supplemental gases concentration from the gases composition sensor to check if it is less than a predetermined allowable threshold. The predetermined allowable threshold of oxygen may be any threshold in excess of ambient levels, in normal conditions a level in excess of 21%. In some situations, the threshold may be the sum of the ambient level and a calculated measurement error of the gases composition sensor. For example, if the error was known to be an absolute amount of 3% concentration, the threshold may be set at 24%. If the sensed oxygen concentration is below the predetermined allowable level, the controller may bypass the time limit and allow the operation of the device into the standby mode.

As a precondition to the running of the timer and/or the checking of a gases composition reading below the predetermined allowable threshold, the controller 13 may as demonstrated at block 344 set the blower to a flow rate such as to assist in the flushing of the supplemental gases from the device or such as to provide a desired gases flow past the gases composition sensor.

Where the device is to be operated to flush gases from the device, this will be understood to involve a purging from the device of a desired amount of the gases contained within the device or a particular target gases contained within the device. For example, this may comprise a reduction of the level of a target gases, such as oxygen, within the device to below a safe threshold. Such entrained gases may present a fire or other safety risk.

The flushing of gases may also act to remove residual humidity from the device, and in particular from about the gases composition sensor 250.

The controller 13 may further optionally at block 341 check the therapy mode state of the device at the time of the user toggling into the standby mode. If the device is operated in a high pressure mode, the controller may then proceed to block 342 and operates the valve 240 of the second inlet 220 to a fully closed condition. If instead the device is not operated in a high pressure therapy mode, for example it was operated in a low pressure therapy mode, the controller at block 343 may generate an instruction to the user to turn off the low pressure supplemental gas supply. This may be the gases source which is associated with the third inlet 230.

If the triggering of an alarm for the detection of oxygen is not by default enabled, or could be disabled and not re-enabled in the prior therapy mode, the controller at block 349 may additionally enable this alarm.

As described, various high pressure and/or low pressure therapy modes may require the use of supplemental gases as delivered from a supplemental gases source. If the supplemental gases are not delivered, or are not delivered in the necessary quantity or quality, or are not delivered from the desired source or to the desired inlet, the therapy may be compromised or may not be possible to be provided.

Examples of reasons that the device is unable to achieve the target oxygen concentration even through the oxygen concentration is in excess of ambient levels may include:
- The high pressure oxygen source isn't producing a sufficient amount of oxygen (e.g. the oxygen bottle is almost empty).
- The high pressure oxygen source is not connected correctly.
- The high pressure oxygen source has just been disconnected (i.e. supplemental oxygen is no longer being supplied, but the FiO2 is still greater than 21% due to the final oxygen still being flushed from the device).
- The device is actually being supplied with oxygen from a low pressure oxygen source via the low pressure gases inlet.

Examples of reasons why the sensed oxygen concentration may remain 21% when the target oxygen concentration is in excess of 21% may include:
- The high pressure oxygen source is not supplying any oxygen (e.g. the oxygen bottle has completely run out).
- The high pressure oxygen source has been disconnected.

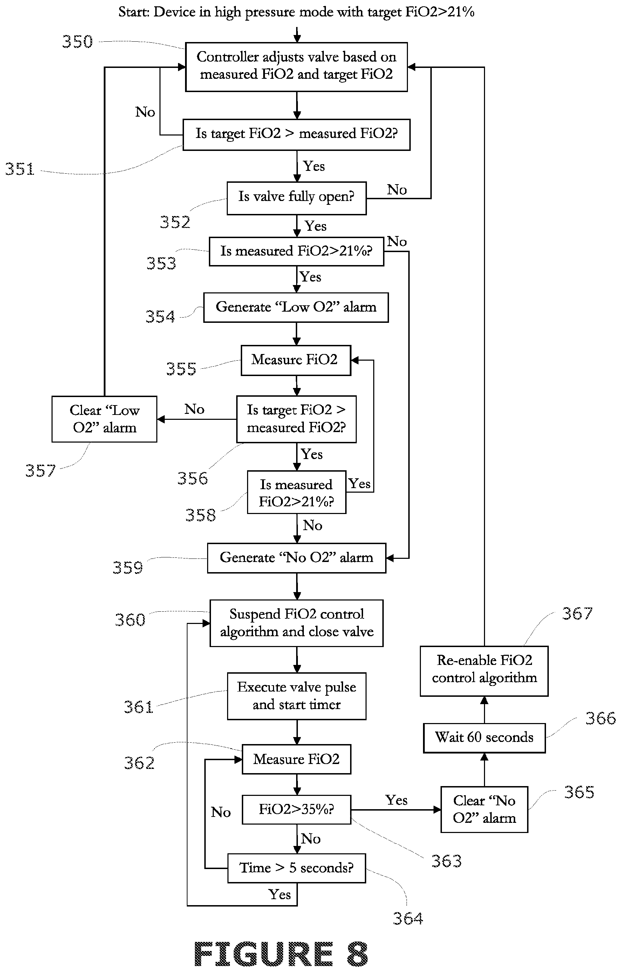

Figure 8 demonstrates an example flowchart for the process of detection of various oxygen source issues during the operation of the device in a manual high pressure therapy mode, being a mode in which the target oxygen concentration is in excess of 21%.

At block 350 the controller adjusts the valve according to the manual therapy mode control regime. Preferably continuously or periodically throughout this control regime, the controller 13 operates the check of block 351 as to whether the target oxygen concentration is in excess of the sensed oxygen concentration by the gases composition sensor 250. If the sensed oxygen concentration is not in excess of the target, and the valve is fully open as at block 352, the controller 13 may trigger an alarm shown at block 354. This alarm may be indicative that insufficient levels of oxygen are being delivered.

The controller 13 may be operable to additionally trigger an alarm indicative of the receipt of no supplemental gases by the device. This alarm may be generated as seen at block 359 provided that several conditions are met. The controller may first measure a sensed oxygen concentration at block 355, and at block 356 check if the target oxygen concentration exceeds the sensed value. If it does, the controller proceeds to block 358, the first condition for the triggering of the second alarm which is indicative of the receipt of no supplemental gases by the device. This first condition may be, as shown at block 358, that the sensed oxygen concentration is approximately at or below a threshold concentration. This threshold concentration may be the sum of the concentration of oxygen in the first gases source gases and a known measurement error value of the gases composition sensor. For example, where the first gases source is an ambient air source and the oxygen concentration is being sensed, the threshold concentration may be 21% plus the known error of the gases composition sensor. The second condition may be, as seen at block 356, that the sensed oxygen concentration is less than the target oxygen concentration set by the user in the manual therapy mode. By the combination of these two conditions the controller 13 may determine that supplemental oxygen is not being received by the device. Accordingly, the controller may trigger the alarm indicating that supplemental gases are not being received as shown at block 359.

Further to this process, if the target oxygen concentration at block 351 has not been met, the controller at block 352 may check that the valve 240 is fully open, and if not return to the manual control regime of block 350. In this situation this would include an opening of the vale further in order to attempt to reach the target oxygen concentration. While described as fully open, such a fully open condition of the valve may not represent a physical maximum open condition, but rather a maximum desired degree of opening. This desired degree of opening may be determined based at least in part on the flow rate of gases through the device and/or the target oxygen concentration.

If the valve is sensed to be fully open at block 352, the controller may continue to block 353. At block 353, the measured oxygen concentration may be compared against the ambient oxygen concentration. This may be a sensed ambient concentration, such as from the ambient air inlet 210, or it may be a preprogramed ambient oxygen concentration value, such as 21%. If the sensed oxygen concentration is in excess of the ambient oxygen concentration, the controller may proceed to block 354 as will be described. Otherwise, if the sensed oxygen concentration is in excess of the ambient oxygen concentration, the controller may proceed to block 359.

If the target gases composition is sensed to have been met or exceeded at block 356, then the controller 13 may clear any alarm indicating insufficient oxygen is being delivered at block 357 before returning the device to the manual therapy mode control regime of block 350.

The triggering of the second alarm at block 359 may additionally result in the controller 13 suspending the manual therapy mode control regime of block 350, as seen at block 360. Following block 360, the controller 13 may proceed to operating a valve pulse test as shown at blocks 361-364. As previously described, the valve pulse test may be desirable for determining the connection of a supplementary gases source at the second inlet 220. The controller 13 may continue to operate the valve pulse test substantially continuously or periodically until a positive test result is received. Following the receipt of a positive test result, the controller may proceed to block 365 and clear the second alarm.

The controller may then delay for a predetermined time period as at block 366 in order to flush the supplemental gases entered into the device by the valve pulse test from the device. Subsequent to the deactivation of the second alarm and any delay expiring, the controller 13 preferably re-enables the manual therapy mode control regime at block 367 and allows the therapy to continue.

The first alarm, as at block 354, may indicate to a user that the target oxygen composition cannot be achieved. It may additionally or alternatively direct a user to check the connection of the supplemental gases source at the second inlet 220.

The second alarm, as at block 359, may indicate to the user that no supplemental oxygen is being received. This alarm may direct a user to connect a supplementary gases source at the second inlet, and/or to check the connection of any connected supplementary gases source at the second inlet.

Where the device comprises a pressure sensor 260 located in the second inlet 220 and upstream of the valve 240 of the second inlet, the controller may additionally or alternatively detect the connection of a supplemental gases source at the second inlet by monitoring a pressure signal from the pressure sensor 260. Such a pressure signal may be monitored by the controller 13 and used to indicate the connection status of a gases source at the second inlet 220 independent of any therapy mode state of the device, including any operational state of the valve 240.

The use of the pressure sensor 260 to indicate the connection status of a supplementary gases source at the second inlet 220 may be used in addition to or alternatively of the valve pulse test as described herein.

In addition or alternatively to the pressure sensor 260, the device may comprise a gases flow sensor 270. The gases flow sensor may be utilised to detect the connection of a supplemental gases source at the second inlet due to a controller sensed increase in the sensed flow above a threshold within a predetermined time after the pulsing of the valve in a valve pulse test. This sensing may be operated independently of any gases composition reading from a gases composition sensor 250.

In various forms the device may comprise primarily a pressure sensor 250 associated with the second inlet 220 and upstream of the valve 240, where the controller is configured to detect the connection of a supplemental gases source at the second inlet 220 based at least in the first instance on a reading from the pressure sensor. In such a configuration, the controller 13 may monitor the pressure reading from the pressure sensor 240 and if the pressure exceeds a threshold pressure, determine that a supplemental gases source has been connected at the second inlet 220.

For example, if the inlet 220 is initially at ambient pressure, the connection of a pressurised supplemental gases source will result in an increase in pressure sensed at the pressure sensor 260. Provided that the predetermined threshold is less than the pressure of the supplemental gases source, the controller will be able to make a determination that the source is connected at the second inlet. Such a configuration may be able to be sensed independent of a closed or open state of the valve 240, provided that the valve 240 in a fully open condition or any other downstream component of the device provides some degree of impedance to the flow from the pressurised gases source.

The device comprising the pressure sensor 250 may additionally comprise a gases composition sensor and may monitor for an alarm upon the occurrence of an insufficient supplemental gas delivery or absence of supplemental gas as previously described in relation to Figure 7.

In various operational conditions it may be desirable to detect the disconnection of a supplementary gases source from an inlet, or otherwise the cessation of flow of supplementary gases from the inlet, of a respiratory therapy device or apparatus.

The disconnection of or cessation of flow from a supplementary gases source (hereinafter simply referred to as a gases source disconnection, but understood to refer to any situation in which a flow from a gases source to the device ceases) may affect the ability of the respiratory therapy device to provide a predetermined treatment. For at least this reason, it may be desirable to notify a user or operator of the disconnection of the source.

Such a notification may be in the form of an alarm or other alert, either on the respiratory therapy device itself or to some external device, for example a wireless mobile device with which the respiratory therapy device is directly or indirectly in communication. The alarm or alert may provide an audible, visual, or kinaesthetic notification to a user or operator, and may be presented on the respiratory therapy device itself or on any external connected device.

For example, an alarm or alert may be presented on a GUI of the respiratory therapy device itself, or a GUI of a connected device such as a user or operator's mobile device such as a smartphone, or a screen of a nurses monitoring station. Any such GUI or screen may be a black and white screen or a colour screen. In some implementations it may further be a touch screen.

As a result of or in addition to changing a type or nature of respiratory support (i.e. respiratory therapy) that the device may provide, it may be desirable to operate various components of the device in response to the disconnection of a gases source. For example, particularly where a previous therapy mode is no longer able to be provided without the supplementary gases flow from the source, it may be desirable that the device is operated in a different therapy mode. In such or other examples, it may be desirable to notify a user or operator that the current therapy mode may no longer be provided, or may be provided with differing characteristics.

Where the device may be operated in a different therapy mode subsequent to the disconnection it may be desirable or even necessary to notify or obtain the approval of a user or operator of this proposed, impending, or already operated change to the therapy mode.

Where a source is disconnected it may be desirable to operate various components of the device accordingly. For example, where a valve is associated with an inlet from which a supplementary gases source is determined to be disconnected, it may be desirable to operate the valve to a closed condition. This may for example be for safety, either to prevent leakage of gases from the inlet to the surroundings, or to prevent the provision of supplementary gases from a reconnected source at that inlet without first an opening of the valve.

Such a valve may be a proportional valve. In other configurations it may be a one-way mechanical valve.

Various additional features and aspects may be compounded with or flow from the detection of the disconnection of a gases source. For example, various operational situations may involve the disconnection of one gases source and the reconnection or initiation of flow from another gases source, or the same gases source at the same or a different inlet of the device.

In an example situation, a respiratory therapy device may first be connected to a high-pressure supplementary gases source. For a variety of reasons, it may be desired to instead operate the device with a low-pressure supplementary gases source. To effect this, a user or operator may disconnect or shut off the gases flow from the high-pressure source. Either prior to, concurrent with, or subsequent to disconnecting or turning off the gases flow from the high-pressure gases source, the user or operator may connect or begin flow from an already connected low-pressure gases source.

It may be desirable that the device can operate to detect such a source connection. As with the disconnection of a source, the connection of a source may one or both of affect the provision of an existing therapy where one is concurrently being provided, or change the types of therapy or characteristics of the therapy which may be provided by the respiratory therapy device.

Where the connection of a gases source is detected, it may be desirable to alert a user to this detection and/or operate the device in various ways. For example, if the connection of a supplementary gases source is detected the device may alert the user of this detection.

Where the connection of the gases source may alter any respiratory therapy that is being provided, or may enable the operation of the device in other respiratory therapy modes, it may additionally be desirable to alert a user or operator of this.

The device may be operated in a different therapy mode as a result of the connection of the gases source. It may be desirable or even necessary to notify a user or operator of a proposed, impending, or already operated change to the therapy mode. In addition, it some configurations it may also be desirable or necessary to or obtain the approval of the user or operator of a change to the therapy mode.

As previously described, a respiratory therapy apparatus may include one or more but preferably a plurality of inlets. These may include one or more inlets for connection of a supplementary gases source or supplementary gases sources. For example, they may include a first inlet 210 and a second inlet 220 such as are seen in Figure 1A, each for receiving either a same or different supplementary gases flow or flows.

Such inlets may also include one or more ambient air inlets, for receiving ambient air into the apparatus.

Gases received from the inlets, whether for supplementary gases or ambient air, passes to a flow generator, such as a flow generator 11 seen in Figure 1A. In various forms the flow generator may be in the form of a blower, which receives the inlet gases and generates a gases flow for delivery to a user of the apparatus.

In some configurations the blower may additionally mix the received inlet gases flow or flows.

Between each inlet and the blower is an associated flow path. For example, where there is a first inlet 210 and a second inlet 220, there may be a respective first flow path and second flow path for guiding the gases flows from the respective inlets 210 and 220 to the blower 11. Where there are further inlets there may be further associated flow paths.

The flow paths between the inlets and the blower may be substantially or entirely separate from each other. In other configurations they may be at least partially shared. For example the flow paths may define separate flow paths in at least a portion towards two or more of the inlets, and a shared flow path or paths in at least a portion towards the blower.

In at least one configuration the first flow path and second flow path may terminate at the blower, and be pneumatically isolated from each from their respective inlets to their termination at the blower. Such a configuration may reduce a potential for gases of the respective flow paths being mixed upstream of the blower. Where for example one flow path contains oxygen gas and the other an ambient air, this isolated configuration may prevent the ambient air becoming more highly oxygenated upstream of the blower.

However the flow path or paths between the one or more inlets and the blower are configured the flow path or paths may pass through a filter to filter out particulate materials and potentially other components from the gases flow or flows. For example, such a filter may be to filter microbes or aerosols. In one example a HEPA filter may be used.

Where the disconnection of a gases sources is to be detected from a particular inlet, for example the second inlet 220, the respiratory therapy apparatus may include a sensor such as the sensor 260 or 270 seen in Figure 3C and Figure 3D, provided within the flow path from the second inlet to the flow generator.

The purpose of such a sensor is to provide sensor information to a controller of the respiratory therapy apparatus. The sensor information that is provided when a gases source is supplying a gases flow to the second inlet will be different from the sensor information that is provided when no gases flow is being supplied to the second inlet.

For example, the sensor may be a pressure sensor which may sense a first pressure when a gases source is not connected at the second inlet and a second pressure when the gases source is connected at the second inlet.

In other forms the sensor may, by way of example only, comprise one or more of the following. The sensor may be a contact sensor to sense the physical connection of a supplementary gases source at the second inlet. The sensor may be a magnetic sensor to sense a magnetic proximity associated with the connection of a supplementary gases source at the second inlet. The sensor may be an electrical conductivity sensor to sense a change in electrical conductivity associated with the connection of a supplementary gases source at the second inlet. The sensor may be a flow sensor to sense a flow of supplementary gases. As a final example, the sensor may be a gases composition sensor such as been elsewhere described herein.

While described particularly in relation to the second inlet 220, which may in various forms be for the connection of a high-pressure supplementary gases source, one or more of such sensors may be implemented at any one or more of the inlets of the apparatus.

In at least some configurations where the apparatus includes two inlets for supplementary gases, being a first inlet and a second inlet, one may include one or more of such sensors while the other does not.

In addition to a sensor such as described above, the respiratory therapy apparatus may also include a valve operable on an inlet, for example the second inlet where it is also provided with the sensor. An example of such a valve is seen in the valve 240 of Figure 3C and 3D.

While in various forms the sensor may be provided downstream of the valve within the flow path of the second inlet 220, such as is seen in the example of Figure 3D, it may be preferable in at least some embodiments that the sensor be provided upstream of the valve, such as is illustrated in Figure 3C. This may be because the sensor may provide sensor information to indicate the connection or disconnection of a gases source even when the valve is in a closed condition. Furthermore, it may be desirable because if the sensor is upstream of the valve the sensor may provide sensor information to indicate the connection or reconnection of a gases source at the second inlet 220.

However, it will be appreciated that, if the valve is not closed, either an upstream or downstream location of the sensor relative to the valve may be utilised to detect the disconnection of a gases source.

The valve may be operable by a controller of the respiratory therapy apparatus, as has previously been described.

When a source disconnection is detected the valve may be operated to or at least toward a closed condition. This may act as a safety measure, to prevent either or both of backflow of gases from the inlet to the surroundings or entrainment of any gases from the surroundings. The closing of the valve may also prevent the uncontrolled or unintended provision of supplementary gases to the apparatus if a gases source is subsequently reconnected at the inlet.

An example of a source disconnection detection will now be given in relation to a configuration where the sensor is a pressure sensor. However, it will be appreciated that corresponding detection routines may be operated utilising the sensor information of one or more alternative types of sensors, such as have been previously described.

Where the supplementary gases source is a pressurised gases source the disconnection of the gases source from the second inlet 220 may be detected by a controller which receives information from the sensor determining that there has been a change in sensed pressure such as would be associated with a disconnection of the gases source. For example, without a connected gases source the inlet may be exposed to an ambient air pressure. The controller may therefore determine that a disconnection has occurred when a drop in pressure to, or to some predetermined threshold above, ambient pressure, is sensed.

In other configurations a disconnection may be determined when the pressure sensed drops by a particular threshold amount.

The values of either an amount of decrease or a decreased pressure threshold may be predetermined, or they may be variable. For example, in some embodiments these values may be modified by a user, or they may be modified by the controller based on sensed information regarding a type or characteristic of a connected gases source. For example, the controller may implement a relatively higher threshold and/or larger amount of decrease in pressure value where a relatively higher pressure gases source is connected. To this end information from a sensor, such as the sensor located in the flow path of the second inlet, may be utilised to indicate such characteristics of a connected gases source.

As previously described, in addition to or alternatively to the provision of one or more alarms or alerts, the respiratory therapy apparatus may be operated in a different therapy mode, or its operation may otherwise be modified, subsequent to the determination of a disconnection. For example, when a high-pressure gases source is disconnected and the apparatus was in an operational mode dependent on the supply of such high-pressure gases the apparatus may transition to a mode in which it provides a therapy not dependent on the high-pressure gases.

The apparatus may transition to a different operational mode following the determination of a gases source disconnection. This mode transition may be solely responsive to the disconnection. For example the apparatus may operate in a different mode because of the disconnection and in the absence of any other gases source connection state, either automatically or subsequent to an approval by a user or operator.

Alternatively, any such change to the operational mode may be further dependent on other conditions, such as a determined connection state of one or more other gases sources.

For example, where the apparatus was in a therapy mode dependent on a high-pressure gases source which was disconnected, the apparatus may only operate in a therapy mode dependent on low-pressure gases when a determination is made that a low-pressure gases source is connected. This connection of a source may be at the second inlet 220, the first inlet 210, or any other inlet of the apparatus.

While previously described in relation to the detection of a disconnection of a gases source a similar detection regime may be utilised to determine the presence or connection of a gases source. For example, where the sensor in the flow path of an inlet is a pressure sensor, a controller may determine that a gases source is connected at the inlet when the sensed pressure exceeds a threshold, or increases by a threshold amount, above an ambient pressure or a previously sensed pressure.

In other configurations, such as where an inlet may not have a sensor and a valve, the presence of a gases flow from a source may be determined by sensing the composition of the gases within the apparatus.

For example, the apparatus may comprise a gases composition sensor 250 such as has been elsewhere herein described. Such a gases composition sensor may be provided at any location within the apparatus, from an inlet to an outlet of the apparatus downstream of the flow generator. In some configurations it may be desirable to locate the gases composition sensor downstream of the flow generator, to sense a composition of gases from the various inlets after they have been combined and optionally mixed by the flow generator.

A controller of the respiratory apparatus may receive a signal from the gases composition sensor which is indicative of a composition of gases in proximity to the gases composition sensor. The controller may according to various circumstances determine that a gases source or a particular type of gases source, or a particular amount or nature of flow therefrom, is connected to the apparatus when a predetermined threshold of gases composition is sensed.

For example, where the apparatus is to operate in a low-pressure therapy mode which is dependent on a supplementary gases flow from a low-pressure supplementary gases source, the controller may determine that this source is connected or at least that the apparatus may be operated in the low-pressure mode when an associated gases composition threshold is sensed.

Where another gases source, such as a high-pressure gases source, is not connected or has been disconnected, the threshold may be some threshold in excess of a level of the supplementary gases in ambient air. For example, where the source necessary to operate a low-pressure mode is a low-pressure oxygen source, the threshold may be a threshold of some amount above an ambient oxygen concentration, conventionally approximately 21%.

The threshold may be a static threshold. In other configurations it may be a decaying threshold, for example a linearly or exponentially decaying threshold. Such a decaying threshold may compensate for an amount of entrained supplementary gases within the apparatus after a source is disconnected by beginning at a first level which is higher than a previous concentration of the supplementary gases when the disconnected source was connected, and decaying away to some other lower level. By this configuration false positives due to entrained supplementary gases may be reduced or avoided.

Where the threshold is a decaying threshold the lower level to which the threshold decays may be some level below a desired concentration for the particular therapy mode. By this configuration if supplementary gases are still being supplied the threshold will drop below the level sensed by the gases composition, and the controller may determine that a source is connected and/or that a particular therapy may be operated. Or, where a supplementary gases source is later connected once the threshold has decayed, the sensed gases composition will increase above the threshold and the controller may make the associated determination.

Where the threshold is a decaying threshold the decay profile may preferably be selected to decay at the same or a similar profile as with which the concentration of supplementary gases entrained within the apparatus decline following the disconnection of their source.

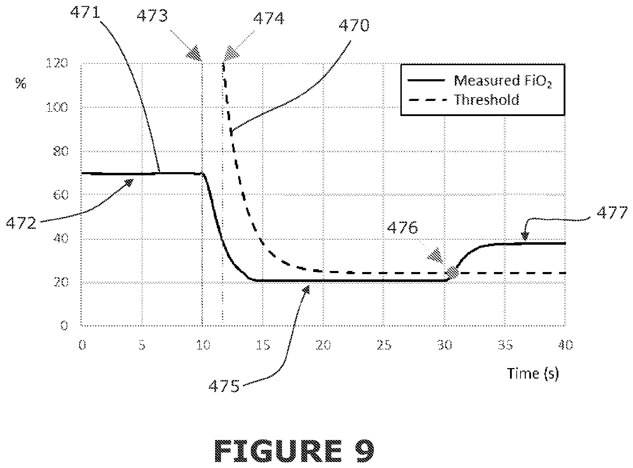

Figure 9 illustrates an example characteristic of a decaying threshold shown by the dashed line 470. A supplementary gases concentration, such as may be sensed by a gases composition sensor, is shown by the line 471. At the region indicated by the arrow 472 supplementary gases are being provided through for example the second inlet, and potentially in the form of a high-pressure oxygen supply.

At the point indicated by the arrow 473 the source is disconnected from the second inlet. The detection of the disconnection may occur at that time, or at some point subsequent thereto. For example, there may be a delay associated with determining a disconnection has occurred.

Provided that the detection has occurred, the controller may activate the check for a supplementary gases concentration which would indicate that supplementary gases are being supplied through the first inlet. This activation is seen at the point of arrow 474.

While the activation may occur immediately upon the detection of the source disconnection, in other preferred embodiments there may be at least some delay before the threshold decay is begun, for example the delay between the time of arrow 473 and arrow 474.

Once the source has been disconnected from the second inlet the supplementary gases concentration sensed will begin to fall, for example as between the point of arrow 472 and arrow 475. Once the supplementary gases have all been purged the sensed level may reach a new steady state, for example for oxygen at an ambient air concentration of approximately 21%.

Where a supplementary gases source is subsequently connected at the first inlet the sensed supplementary gases concentration may rise again, for example as seen at approximately the 30-second mark in Figure 9. As the supplementary gases concentration rises it passes the decayed level of the threshold 470 at the point 476, and may go on to reach some new constant state at the point 477. The point 476 is indicative of when a supplementary gases source, and in particular a low-pressure supplementary gases source, is connected.

As seen in Figure 9 a low-pressure gases source is connected at the first inlet some time period after the high-pressure source is disconnected from the second inlet. In other use situations the low-pressure source may be connected prior to the high-pressure source disconnection, or concurrently with it. In such configurations instead of falling beneath the threshold 470 the supplementary gases concentration 471 may fall but then cross the threshold 470 while it is still decaying.

Where the disconnection of a gases source has been detected, a delay may be exercised between the time the disconnection is determined and the when the controller may make a determination that another gases source is connected, or another gases flow is being provided. This may be, for example, to allow for a flushing of previous supplementary gases from the apparatus.

If a gases source is not connected, the supplementary gases concentration 471 will remain below the threshold 470. If the supplementary gases concentration 471 remains below the threshold 470 for a particular period of time the controller may trigger an alert to the user that no supplementary gases source has been connected.

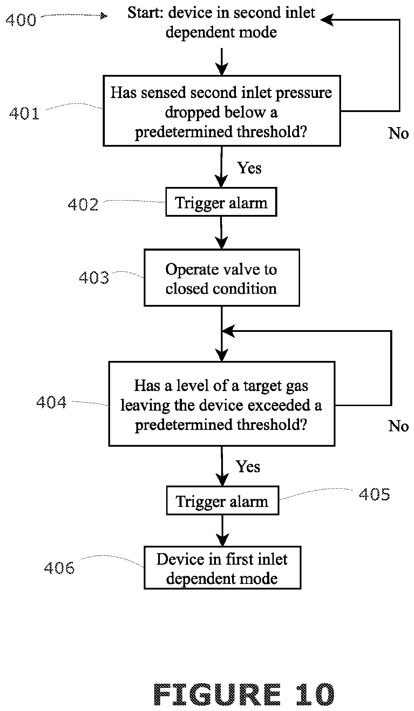

Figure 10 shows a flowchart of an example process for controlling a respiratory therapy apparatus in relation to the disconnection of a first supplementary gases source and operation of the apparatus in a mode dependent on the supply of supplementary gases from a second supplementary gases source. This process will now be described as an example of an implementation of source disconnection detection and/or source connection or supply determinations.

At the starting point 400 of Figure 10 the apparatus is being operated in a therapy mode which is dependent on supplementary gases received from the second inlet. Where the second inlet is for the connection of a high-pressure gases source this therapy mode may be characterised as a high-pressure mode.

At block 401 the controller 13 checks whether the sensor at the second inlet is providing a reading which would indicate that the source has been disconnected. In the particular example of Figure 10 the sensor is a pressure sensor, and the controller checks to see if the sensed pressure at the inlet has dropped below a predetermined threshold. As previously described, this predetermined threshold. If it has, the controller moves to block 402. If it has not, the controller loops back to the start of the control regime, whereupon it will repeat the step of block 401 albeit potentially with some interim delay period.

At block 402 the controller may optionally activate an alarm. Such an alarm may provide an audible, visual, or kinaesthetic notification to a user or operator. The alarm may include information related to the disconnection, such as the fact that a disconnection has been detected. The alarm triggered may also be an electronic alarm, which may be passed to some external connected device to provide the audible, visual or kinaesthetic alert. As previously described, such an alarm may simply notify the user, or in some forms it may require an input from the user before the remainder of the control process may continue, for example a confirmation by the user or operator that a source has been disconnected from the particular inlet.

After any alarm, the controller may at block 403 operate the valve of the second inlet to a closed condition. The controller provides a control signal to close the valve.

The controller may operate the valve within an overarching protocol, such example a limit on the frequency with which the valve may be closed. For example, it may be that the valve is not to be closed more frequently than every 45 seconds, and if 45 seconds has not elapsed since the last valve closure the controller may delay operating the valve to a closed condition, or before re-checking the connection status. Such a configuration may ensure that the patient is not repeatedly cut off from supplementary gases where the supplementary gases pressure is fluctuating so as to incorrectly indicate that the source has been disconnected.

In some configurations it may be desirable to operate various operational changes on the apparatus subsequent to the determination of a disconnection. The remaining portions of the control process of Figure 10 illustrate one such set of processes to operate to a different therapy mode dependent on first inlet supplementary gases.

In this configuration the respiratory therapy apparatus may include a gases composition sensor. The gases composition sensor may be in any form elsewhere herein described, such as but not limited to an ultrasonic transducer sensor.

At block 404 the controller operates to check if the level of target gases sensed by the gases composition sensor is in excess of a predetermined threshold. Such a threshold may be a set threshold or a decaying threshold, and may be implemented with or without a delay, as described previously.

Any such decaying threshold may decay according to a predetermined function. The function may be stored in the memory of the controller. Alternatively the decaying function may be defined in a look up table or other format.

The function may be pre-programmed static, or may alternatively be modified by the controller, for example to react to different flushing rates of gases from the apparatus because of different blower operational speeds.

If the threshold is met or exceeded, the controller may continue to block 405. If the threshold is not met or exceeded, the controller may revert back to before block 404, and operate the check of block 404 again. The controller may trigger an alarm based on a negative result, or a particular number of repeated negative results, or the expiry of a predetermined time delay. The controller may operate one or more delays between concurrent checks of the sensed gases composition against the threshold.

Any increase in concentration may be an absolute increase of the concentration of the one or more supplementary gases, or it may be a rate of increase of concentration. Where a rate of increase of concentration is utilised, the threshold may be considered to be met if the rate of increase of sensed concentration is greater than the concurrent decay rate of the decaying threshold.

If the threshold is met or exceeded the controller may optionally trigger an alarm at block 405. As described in relation to the alarm 402, the alarm of block 405 may be to notify a user, and/or it may require some authorisation to proceed. The alarm may notify the user or operator that a low-pressure supplementary gases source has been detected, and/or may require their authorisation to operate in a therapy mode which is dependent on first inlet supplementary gases.

An example of such a therapy mode may be a high-flow mode with either a set or manually controlled supplementary gases concentration. In another example such a therapy mode may be pressure supported, for example a CPAP or Bilevel therapy with supplementary gases, or even in the absence of any supplementary gases.

Subsequent to any notification and/or approval at block 405, at block 406 the controller may operate the apparatus in a therapy mode which is dependent on supplementary gases from the first inlet.

While illustrated in Figure 10 as proceeding after a determination is made at block 401 that the source has been disconnected to an optional alarm at block 402 and subsequent checks to determine if another source is connected, the controller may alternatively or additionally directly operate (optionally dependent on a user or operator authorisation) in a different therapy mode. For example, if the apparatus is in a therapy mode which requires a high-pressure oxygen flow, such as an oxygen control mode which would include operating to FiO2 threshold or in a closed loop SpO2 regime, the controller may switch to a high flow mode when the disconnection of the high-pressure oxygen source is detected.

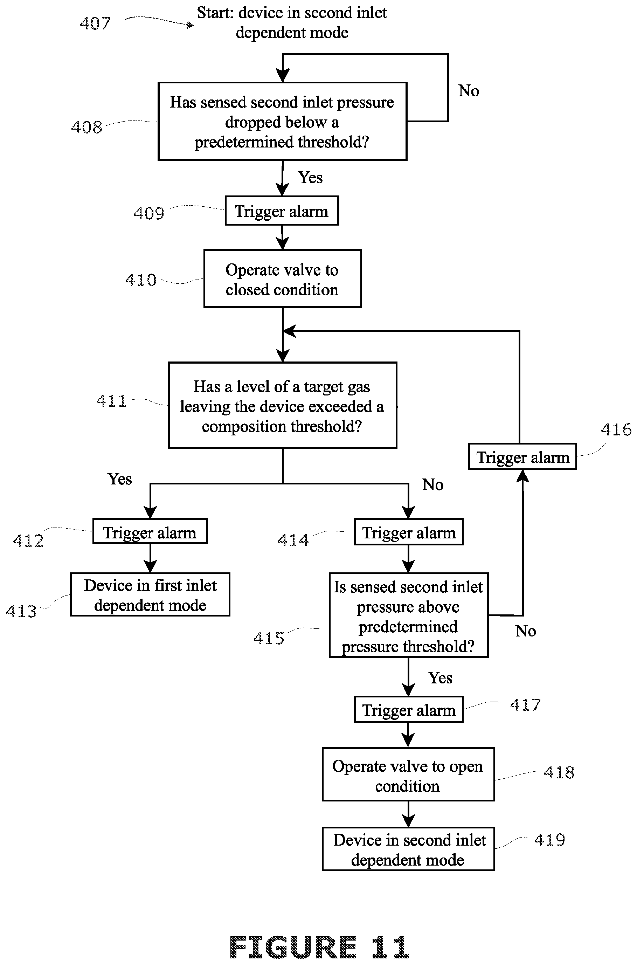

Figure 11 shows a flowchart of another example process of controlling a respiratory therapy apparatus in relation to the disconnection of a supplementary gases source from the second inlet.

As in Figure 10, in Figure 11 the control process start with the apparatus in a therapy mode which is dependent on supplementary gases received from the second inlet. These may for example be high-pressure supplementary gases.

At blocks 408, 409, and 410 the controller operates the same steps as described in relation to blocks 401, 402, and 403 of Figure 10. These steps at least include a check as to whether the sensor in the second inlet indicates that the associated supplementary gases source has been disconnected, for example by a decrease in the sensed pressure where the sensor is a pressure sensor.

If the controller determines that there has been a disconnection from the second inlet, subsequent to any alarm at block 409 and any valve operation at block 410, the controller moves to block 411.

As at block 404 of Figure 10, at block 411 the controller may operate to check if a level of the supplementary gas or particular target supplementary gas exceeds the predetermined composition threshold.

If the threshold is exceeded, the controller may move to block 412 to optionally trigger an alarm, as with block 405 of Figure 10, and subsequently to operate the apparatus in a therapy mode which is dependent on the first inlet supplementary gases at block 413.

If instead the threshold is not exceeded, the control process of Figure 11 provides for an alternative configuration to that of Figure 10. As seen at block 412, the controller may trigger an alarm relating to the fact that the threshold has not been met. The alarm may in various preferred forms be an audible alarm and/or a visual alert, which may communicate that the threshold has not been met. As previously described, the alarm may be communicated to the user or operator by the apparatus itself, such as by a GUI of the apparatus, may be communicated to a connected device such as a smartphone of the user or operator, or may be communicated to a device at a medical monitoring station such as a nurses station.

Subsequent to any alarm, the controller proceeds to block 415 at which it checks the sensor information received from the sensor at the second inlet.

If the sensor information has changed from its reading at block 408 which indicated a disconnection, this may indicate that a source has been reconnected at the second inlet. For example, where the sensor is a pressure sensor the controller may check for an increase of a certain amount above the value sensed at block 408, and/or an increase to or above a particular threshold. If the threshold is exceeded the controller determines that a source has been reconnected at the second inlet, while if it is not exceeded the controller determines that a source has not been reconnected at the second inlet.

If a negative determination is made the controller proceeds to block 416 at which it may optionally trigger an alarm, such as an alarm to notify the user that a reconnected source at the second inlet has not been detected. The controller will then operate back to before block 411, to continue checking if the gases composition sensor indicates that a source is connected at the first inlet.

If a positive determination is made the controller operates to block 417 at which it may optionally generate an alarm. This alarm may notify a user or operator that the apparatus has detected a supplementary gases source at the second inlet again. It may also optionally require user or operator approval to move to the next stage, at block 418.

At block 418 the controller operates the valve to an open condition, such that the gases from the reconnected source at the second inlet may be received into the apparatus to be provided in the therapy, in a second inlet dependent therapy mode at block 419.

In some embodiments, the advantages of detecting which source is connected, or whether a source is connected to one or more of the first inlet or the second inlet may include: the provision of information regarding the source detection to the clinician or user (for example a source disconnection) via for example an alarm or via a display as described in more detail above.

In some embodiments, the advantages of detecting the disconnection of a source may include: the provision of information regarding the source detection to the clinician or user (for example a source disconnection) via for example an alarm or via a display as described in more detail above.

The detection of the disconnection of a source, and the provision of this information to a clinician or user may reduce the risk of a patient not receiving adequate therapy. For example if a supplemental oxygen source is disconnected the patient may not receive supplemental oxygen, and therefore be provided with breathing gases which do not include the desired level of supplemental oxygen, and the patient may desaturate. If a clinician or user is provided with information as to the detection of the disconnection of a source, they can investigate the cause of the disconnection and attempt to remedy it. This may be of additional importance in a home care environment where there is less supervision as to the functioning of the apparatus, and the status of a supplemental gas source and its connection to the apparatus.

In some embodiments, the advantages of detecting which source is connected may include allowing the device to automatically operate in the correct mode depending on which source is connected (for example a high pressure mode or a low pressure mode) as described in more detail above.

Although embodiments have been described with reference to a number of illustrative embodiments thereof, it will be understood by those skilled in the art that various changes in form and details may be made therein without departing from the scope of the invention as defined by the appended claims. Therefore, the preferred embodiments should be considered in a descriptive sense only and not for purposes of limitation, and also the technical scope of the invention is not limited to the embodiments. Furthermore, the present invention is defined not by the detailed description of the invention but by the appended claims, and all differences within the scope will be construed as being comprised in the present disclosure.

Many modifications will be apparent to those skilled in the art without departing from the scope of the present invention as herein described with reference to the accompanying drawings.

## Claims

1. An apparatus (10) for delivery of a gases flow to a user, the apparatus (10) comprising:
a first inlet (210) for receiving a supplementary gases flow,
a second inlet (220) configured to receive a supplementary gases flow,
a blower (11) to receive a gases flow from one or both of the first inlet (210) and second inlet (220) and generate a gases flow for delivery to the user,
a first flow path and second flow path for guiding a gases flow from the first inlet (210) and second inlet (220) respectively to or towards the blower (11),
a valve and a sensor each located within the second flow path, and
a controller (13) configured to
detect the disconnection of a gases source from the second inlet (220) or the cessation of provision of a supplementary gases flow through the second inlet (220), and
respond by operating the valve to close the second flow path.

2. The apparatus (10) of claim 1, wherein the controller (13) detects the disconnection of a gases source from the second inlet (220) or the cessation of provision of a supplementary gases flow through the second inlet (220) based on a signal received from the sensor located within the second flow path.

3. The apparatus (10) of claim 1 or 2, wherein subsequent to detecting the disconnection of a gases source from the second inlet (220) or the cessation of provision of a supplementary gases flow through the second inlet (220) the controller (13) triggers a first alarm.

4. The apparatus (10) of claims 1 or 2, wherein subsequent to detecting the disconnection of a gases source from the second inlet (220) or the cessation of provision of a supplementary gases flow through the second inlet (220) the controller (13) triggers a first alarm and operates the valve to a closed position to shut the second flow path.

5. The apparatus (10) of claim 3 or 4, wherein the first alarm indicates that a gases source has been disconnected from the second inlet (220) and/or that a supplementary gases flow has ceased to be provided through the second inlet (220).

6. The apparatus (10) of any one of claims 3 to 5, wherein the apparatus (10) further comprises a gases composition sensor (250) to measure a concentration of one or more supplementary gases within the gases flow for delivery to the user.

7. The apparatus (10) of claim 6, wherein the gases composition sensor (250) is located downstream of the blower (11).

8. The apparatus (10) of claim 6 or 7, wherein subsequent to responding by operating the valve to a closed condition and triggering the first alarm the controller (13) performs the step of comparing a supplementary gas concentration sensed by the gases composition sensor (250) to a threshold of supplementary gas concentration.

9. The apparatus (10) of claim 8, wherein there is a time delay between a) the step of detecting the disconnection of a gases source from the second inlet (220) or the cessation of provision of a supplementary gases flow through the second inlet (220) and b) the step of comparing the sensed concentration against the threshold of supplementary gas concentration.

10. The apparatus (10) of claim 8, wherein the controller (13) operates the valve to close the second flow path and wherein there is a time delay between the step of operating the valve and the step of comparing the sensed concentration against the threshold of supplementary gas concentration.

11. The apparatus (10) of any one of claims 8 to 10, wherein the threshold of supplementary gas concentration comprises a decaying threshold from an initial threshold of supplementary gas concentration which is above a second inlet (220) supplementary gases concentration when supplementary gases are being supplied through the second inlet (220), and to a final threshold of supplementary gas concentration which is below a minimum supplementary gases concentration when supplementary gases are supplied through the first inlet (210) and above an ambient air concentration of the supplementary gas, and the threshold of supplementary gas concentration decays at a rate slower than an expected rate of flushing of second inlet (220) supplementary gases from the apparatus.

12. The apparatus (10) of any one of claims 8 to 11, wherein if the sensed supplementary gas concentration exceeds the threshold of supplementary gas concentration the controller (13) determines that a supplementary gases flow is being provided through the first inlet (210), and, where the controller (13) has determined that a supplementary gases flow is being provided through the first inlet (210), the controller (13) operates the apparatus (10) in a first mode, the first mode being a mode in which a closed loop gas control function may not be activated.

13. The apparatus (10) of any one of claims 8 to 12, wherein if the sensed supplementary gas concentration does not exceed the threshold of supplementary gas concentration the controller (13) determines that a supplementary gases flow is not being provided through the first inlet (210) and the controller (13) operates the apparatus (10) in a second mode wherein the controller (13) operates the valve to control a supplementary gases flow delivered through the second inlet (220).

14. The apparatus (10) of claim 13, wherein the operation of the apparatus (10) in the second mode is dependent on the controller (13) determining that a supplementary gases flow is being provided to the second inlet (220), and the controller (13) determines that a supplementary gases flow is being provided to the second inlet (220) if a sensed value from the sensor within the second flow path is above a sensor threshold.

15. The apparatus (10) of claim 13 or 14, wherein in the second mode a closed loop gas control function may be activated.

## Patentansprüche

1. Vorrichtung (10) zur Abgabe eines Gasstroms an einen Benutzer, die Vorrichtung (10) umfassend:
einen ersten Einlass (210) zum Aufnehmen eines zusätzlichen Gasstroms,
einen zweiten Einlass (220), der so konfiguriert ist, dass er einen zusätzlichen Gasstrom aufnimmt,
ein Gebläse (11) zum Aufnehmen eines Gasstroms aus einem oder beiden des ersten Einlasses (210) und des zweiten Einlasses (220) und zum Erzeugen eines Gasstroms zur Abgabe zum Benutzer,
einen ersten Strömungsweg und einen zweiten Strömungsweg zum Leiten eines Gasstroms von dem ersten Einlass (210) beziehungsweise von dem zweiten Einlass (220) zum Gebläse (11) oder in Richtung des Gebläses,
ein Ventil und einen Sensor, die sich jeweils innerhalb des zweiten Strömungswegs befinden, und
eine Steuereinheit (13), die so konfiguriert ist, dass sie die Trennung einer Gasquelle vom zweiten Einlass (220) oder die Unterbrechung der Abgabe eines zusätzlichen Gasstroms über den zweiten Einlass (220) erkennt, und
das Reagieren, indem das Ventil betätigt wird, um den zweiten Strömungsweg zu schließen.

2. Vorrichtung (10) nach Anspruch 1, wobei die Steuereinheit (13) die Trennung einer Gasquelle vom zweiten Einlass (220) oder die Unterbrechung der Abgabe eines zusätzlichen Gasstroms über den zweiten Einlass (220) auf der Grundlage eines Signals erkennt, das von dem im zweiten Strömungsweg angeordneten Sensor empfangen wird.

3. Vorrichtung (10) nach Anspruch 1 oder 2, wobei die Steuereinheit (13) nach der Erkennung der Trennung einer Gasquelle vom zweiten Einlass (220) oder der Beendigung der Abgabe eines zusätzlichen Gasstroms über den zweiten Einlass (220) einen ersten Alarm auslöst.

4. Vorrichtung (10) nach Anspruch 1 oder 2, wobei die Steuereinheit (13) nach der Erkennung der Trennung einer Gasquelle vom zweiten Einlass (220) oder der Beendigung der Abgabe eines zusätzlichen Gasstroms über den zweiten Einlass (220) einen ersten Alarm auslöst und das Ventil in eine geschlossene Position bringt, um den zweiten Strömungsweg zu schließen.

5. Vorrichtung (10) nach Anspruch 3 oder 4, wobei der erste Alarm anzeigt, dass eine Gasquelle vom zweiten Einlass (220) getrennt wurde und/oder dass kein zusätzlicher Gasstrom mehr über den zweiten Einlass (220) zugeführt wird.

6. Vorrichtung (10) nach einem der Ansprüche 3 bis 5, wobei die Vorrichtung (10) ferner einen Gaszusammensetzungssensor (250) umfasst, um die Konzentration eines oder mehrerer zusätzlicher Gase innerhalb des Gasstroms zu messen, der dem Benutzer zugeführt wird.

7. Vorrichtung (10) nach Anspruch 6, wobei sich der Gaszusammensetzungssensor (250) nachgelagert von dem Gebläse (11) befindet.

8. Vorrichtung (10) nach Anspruch 6 oder 7, wobei die Steuereinheit (13) nach dem Ansprechen durch Schließen des Ventils und Auslösen des ersten Alarms den Schritt des Vergleichens einer vom Gaszusammensetzungssensor (250) erfassten zusätzlichen Gaskonzentration mit einem Schwellenwert der zusätzlichen Gaskonzentration durchführt.

9. Vorrichtung (10) nach Anspruch 8, wobei eine Zeitverzögerung zwischen a) dem Schritt der Erkennung der Trennung einer Gasquelle vom zweiten Einlass (220) oder der Beendigung der Abgabe eines zusätzlichen Gasstroms über den zweiten Einlass (220) und b) dem Schritt des Vergleichens der erfassten Konzentration mit dem Schwellenwert der zusätzlichen Gaskonzentration besteht.

10. Vorrichtung (10) nach Anspruch 8, wobei die Steuereinheit (13) das Ventil betätigt, um den zweiten Strömungsweg zu schließen, und wobei zwischen dem Schritt des Betätigens des Ventils und dem Schritt des Vergleichens der gemessenen Konzentration mit dem Schwellenwert der zusätzlichen Gaskonzentration eine Zeitverzögerung besteht.

11. Vorrichtung (10) nach einem der Ansprüche 8 bis 10, wobei der Schwellenwert der zusätzlichen Gaskonzentration einen abklingenden Schwellenwert umfasst, der von einem anfänglichen Schwellenwert der zusätzlichen Gaskonzentration, der über der zusätzlichen Gaskonzentration am zweiten Einlass (220) liegt, wenn zusätzliche Gase über den zweiten Einlass (220) zugeführt werden, und bis zu einem Endschwellenwert der zusätzlichen Gaskonzentration abfällt, der unter einer minimalen zusätzlichen Gaskonzentration liegt, wenn zusätzliche Gase über den ersten Einlass (210) zugeführt werden, und über einer Umgebungsluftkonzentration des zusätzlichen Gases liegt, und der Schwellenwert der zusätzlichen Gaskonzentration mit einer Rate abnimmt, die langsamer ist als eine erwartete Ausspülrate der über den zweiten Einlass (220) zugeführten zusätzlichen Gase aus der Vorrichtung.

12. Vorrichtung (10) nach einem der Ansprüche 8 bis 11, wobei, wenn die gemessene zusätzliche Gaskonzentration den Schwellenwert der zusätzlichen Gaskonzentration überschreitet, die Steuereinheit (13) feststellt, dass ein zusätzlicher Gasstrom über den ersten Einlass (210) zugeführt wird, und wobei, wenn die Steuereinheit (13) festgestellt hat, dass ein zusätzlicher Gasstrom über den ersten Einlass (210) zugeführt wird, die Steuereinheit (13) die Vorrichtung (10) in einem ersten Modus betreibt, wobei der erste Modus ein Modus ist, in dem eine Gasregelungsfunktion mit geschlossenem Regelkreis gegebenenfalls nicht aktiviert ist.

13. Vorrichtung (10) nach einem der Ansprüche 8 bis 12, wobei, wenn die gemessene zusätzliche Gaskonzentration den Schwellenwert der zusätzlichen Gaskonzentration nicht überschreitet, die Steuereinheit (13) feststellt, dass kein zusätzlicher Gasstrom über den ersten Einlass (210) zugeführt wird, und die Steuereinheit (13) die Vorrichtung (10) in einem zweiten Modus betreibt, in dem die Steuereinheit (13) das Ventil betätigt, um einen über den zweiten Einlass (220) zugeführten zusätzlichen Gasstrom zu steuern.

14. Vorrichtung (10) nach Anspruch 13, wobei der Betrieb der Vorrichtung (10) im zweiten Modus davon abhängt, dass die Steuereinheit (13) feststellt, dass ein zusätzlicher Gasstrom zum zweiten Einlass (220) geleitet wird, und die Steuereinheit (13) feststellt, dass ein zusätzlicher Gasstrom zum zweiten Einlass (220) geleitet wird, wenn ein von dem Sensor im zweiten Strömungsweg gemessener Wert über einem Sensorschwellenwert liegt.

15. Vorrichtung (10) nach Anspruch 13 oder 14, wobei im zweiten Modus eine Gasregelungsfunktion mit geschlossenem Regelkreis aktiviert werden kann.

## Revendications

1. Appareil (10) pour la distribution d'un flux de gaz à un utilisateur, l'appareil (10) comprenant :
une première entrée (210) pour la réception d'un flux de gaz supplémentaires,
une seconde entrée (220) configurée pour recevoir un flux de gaz supplémentaires,
une soufflante (11) pour recevoir un flux de gaz provenant de l'une ou des deux parmi la première entrée (210) et la seconde entrée (220) et générer un flux de gaz pour une distribution à l'utilisateur,
un premier chemin d'écoulement et un second chemin d'écoulement destinés à guider un flux de gaz provenant de la première entrée (210) et de la seconde entrée (220) respectivement jusqu'à ou vers la soufflante (11),
une vanne et un capteur situés chacun au sein du second chemin d'écoulement, et
un dispositif de commande (13) configuré pour détecter la déconnexion d'une source de gaz de la seconde entrée (220) ou la cessation de la fourniture d'un flux de gaz supplémentaires par la seconde entrée (220), et
répondre par l'actionnement de la vanne pour fermer le second chemin d'écoulement.

2. Appareil (10) selon la revendication 1, dans lequel le dispositif de commande (13) détecte la déconnexion d'une source de gaz de la seconde entrée (220) ou la cessation de la fourniture d'un flux de gaz supplémentaires par la seconde entrée (220) sur la base d'un signal reçu du capteur situé au sein du second chemin d'écoulement.

3. Appareil (10) selon la revendication 1 ou la revendication 2, dans lequel, à la suite de la détection de la déconnexion d'une source de gaz de la seconde entrée (220) ou de la cessation de la fourniture d'un flux de gaz supplémentaires par la seconde entrée (220), le dispositif de commande (13) déclenche une première alarme.

4. Appareil (10) selon la revendication 1 ou la revendication 2, dans lequel, à la suite de la détection de la déconnexion d'une source de gaz de la seconde entrée (220) ou de la cessation de la fourniture d'un flux de gaz supplémentaires par la seconde entrée (220), le dispositif de commande (13) déclenche une première alarme et actionne la vanne vers une position fermée pour obturer le second chemin d'écoulement.

5. Appareil (10) selon la revendication 3 ou la revendication 4, dans lequel la première alarme indique qu'une source de gaz a été déconnectée de la seconde entrée (220) et/ou qu'un flux de gaz supplémentaires a cessé d'être fourni par la seconde entrée (220).

6. Appareil (10) selon l'une quelconque des revendications 3 à 5, dans lequel l'appareil (10) comprend en outre un capteur (250) de composition de gaz pour mesurer une concentration d'un ou de plusieurs gaz supplémentaires au sein du flux de gaz pour une distribution à l'utilisateur.

7. Appareil (10) selon la revendication 6, dans lequel le capteur (250) de composition de gaz est situé en aval de la soufflante (11).

8. Appareil (10) selon la revendication 6 ou la revendication 7, dans lequel, à la suite de la réponse par l'actionnement de la vanne vers un état fermé et le déclenchement de la première alarme, le dispositif de commande (13) réalise l'étape de comparaison d'une concentration en gaz supplémentaire détectée par le capteur (250) de composition de gaz avec un seuil de concentration en gaz supplémentaire.

9. Appareil (10) selon la revendication 8, dans lequel il y a un délai entre a) l'étape de détection de la déconnexion d'une source de gaz de la seconde entrée (220) ou de la cessation de la fourniture d'un flux de gaz supplémentaires par la seconde entrée (220) et b) l'étape de comparaison de la concentration détectée par rapport au seuil de concentration en gaz supplémentaire.

10. Appareil (10) selon la revendication 8, dans lequel le dispositif de commande (13) actionne la vanne pour fermer le second chemin d'écoulement et dans lequel il y a un délai entre l'étape d'actionnement de la vanne et l'étape de comparaison de la concentration détectée par rapport au seuil de concentration en gaz supplémentaire.

11. Appareil (10) selon l'une quelconque des revendications 8 à 10, dans lequel le seuil de concentration en gaz supplémentaire comprend un seuil décroissant allant d'un seuil initial de concentration en gaz supplémentaire qui est supérieur à une concentration en gaz supplémentaires de seconde entrée (220) lorsque des gaz supplémentaires sont alimentés par la seconde entrée (220), et jusqu'à un seuil final de concentration en gaz supplémentaire qui est inférieur à une concentration en gaz supplémentaires minimale lorsque des gaz supplémentaires sont alimentés par la première entrée (210) et au-dessus d'une concentration d'air ambiant du gaz supplémentaire, et le seuil de concentration en gaz supplémentaire décroît à une vitesse plus lente qu'une vitesse attendue de purge de gaz supplémentaires de seconde entrée (220) à partir de l'appareil.

12. Appareil (10) selon l'une quelconque des revendications 8 à 11, dans lequel si la concentration en gaz supplémentaire détectée dépasse le seuil de concentration en gaz supplémentaire, le dispositif de commande (13) détermine qu'un flux de gaz supplémentaires est fourni par la première entrée (210), et, lorsque le dispositif de commande (13) a déterminé qu'un flux de gaz supplémentaires est fourni par la première entrée (210), le dispositif de commande (13) actionne l'appareil (10) dans un premier mode, le premier mode étant un mode dans lequel une fonction de commande de gaz en boucle fermée ne peut pas être activée.

13. Appareil (10) selon l'une quelconque des revendications 8 à 12, dans lequel si la concentration en gaz supplémentaire détectée ne dépasse pas le seuil de concentration en gaz supplémentaire, le dispositif de commande (13) détermine qu'un flux de gaz supplémentaires n'est pas fourni par la première entrée (210) et le dispositif de commande (13) actionne l'appareil (10) dans un second mode dans lequel le dispositif de commande (13) actionne la vanne pour commander un flux de gaz supplémentaires distribué par la seconde entrée (220).

14. Appareil (10) selon la revendication 13, dans lequel le fonctionnement de l'appareil (10) dans le second mode est dépendant du fait que le dispositif de commande (13) détermine qu'un flux de gaz supplémentaires est fourni à la seconde entrée (220), et le dispositif de commande (13) détermine qu'un flux de gaz supplémentaires est fourni à la seconde entrée (220) si une valeur détectée par le capteur au sein du second chemin d'écoulement est supérieure à un seuil de capteur.

15. Appareil (10) selon la revendication 13 ou la revendication 14, dans lequel, dans le second mode, une fonction de commande de gaz en boucle fermée peut être activée.
